# EUROPEAN PATENT APPLICATION

(11) **EP 1 959 012 A2**
(43) Date of publication of application: **20.08.2008**
(21) Application number: 08155283.8
(22) Date of filing: 29.12.2005
(51) Int. Cl.: C12N 15/11, C07H 21/02

(54) **Novel oligonucleotide compositions and probe sequences useful for detection and analysis of microRNAs and their target mRNAs**

(30) Priority: 29.12.2004 DK 200402018; 29.04.2005 DK 200500638; 27.09.2005 DK 200501351; 29.04.2005 DK 200500637; 29.12.2004 US 640098 P
(62) Divisional of application: 05822995.6
(71) Applicant: Exiqon A/S, 2950 Vedbaek (DK)
(72) Inventor: Plasterk, Ronald, 2950 Vedbæk (DK); Wienholds, Erno, 1906 CK Limmen (NL); Kloosterman, Wigard, 2624 KZ Delft (NL); Kauppinen, Sakari, 2765 Smørum (DK)
(74) Representative: Inspicos A/S

(57) **Abstract**

The invention relates relates to ribonucleic acids and oligonucleotide probes useful for detection and analysis of microRNAs and their target mRNAs, as well as small interfering RNAs (siRNAs).

## Description

The present invention relates to ribonucleic acids and oligonucleotide probes useful for detection and analysis of microRNAs and their target mRNAs, as well as small interfering RNAs (siRNAs). The invention furthermore relates to oligonucleotide probes for detection and analysis of other non-coding RNAs, as well as mRNAs, mRNA splice variants, allelic variants of single transcripts, mutations, deletions, or duplications of particular exons in transcripts, e.g. alterations associated with human disease, such as cancer.

### Background of the Invention

The present invention relates to the detection and analysis of target nucleotide sequences in a wide variety of nucleic acid samples and more specifically to the methods employing the design and use of oligonucleotide probes that are useful for detecting and analysing target nucleotide sequences, especially RNA target sequences, such as microRNAs and their target mRNAs and siRNA sequences of interest and for detecting differences between nucleic acid samples (e.g., such as samples from a cancer patient and a healthy patient).

### MicroRNAs

The expanding inventory of international sequence databases and the concomitant sequencing of more than 200 genomes representing all three domains of life - bacteria, archea and eukaryota - have been the primary drivers in the process of deconstructing living organisms into comprehensive molecular catalogs of genes, transcripts and proteins. The importance of the genetic variation within a single species has become apparent, extending beyond the completion of genetic blueprints of several important genomes, culminating in the publication of the working draft of the human genome sequence in 2001 (Lander, Linton, Birren et al., 2001 Nature 409: 860-921; Venter, Adams, Myers et al., 2001 Science 291: 1304-1351; Sachidanandam, Weissman, Schmidt et al., 2001 Nature 409: 928-933). On the other hand, the increasing number of detailed, large-scale molecular analyses of transcription originating from the human and mouse genomes along with the recent identification of several types of non-protein-coding RNAs, such as small nucleolar RNAs, siRNAs, microRNAs and antisense RNAs, indicate that the transcriptomes of higher eukaryotes are much more complex than originally anticipated (Wong et al. 2001, Genome Research 11: 1975-1977; Kampa et al. 2004, Genome Research 14: 331-342).

As a result of the Central Dogma: 'DNA makes RNA, and RNA makes protein', RNAs have been considered as simple molecules that just translate the genetic information into protein.

Recently, it has been estimated that although most of the genome is transcribed, almost 97% of the genome does not encode proteins in higher eukaryotes, but putative, non-coding RNAs (Wong et al. 2001, Genome Research 11: 1975-1977). The non-coding RNAs (ncRNAs) appear to be particularly well suited for regulatory roles that require highly specific nucleic acid recognition. Therefore, the view of RNA is rapidly changing from the merely informational molecule to comprise a wide variety of structural, informational and catalytic molecules in the cell.

Recently, a large number of small non-coding RNA genes have been identified and designated as microRNAs (miRNAs) (for review, see Ke et al. 2003, Curr.Opin. Chem. Biol. 7:516-523). The first miRNAs to be discovered were the lin-4 and let-7 that are heterochronic switching genes essential for the normal temporal control of diverse developmental events (Lee et al. 1993, Cell 75:843-854; Reinhart et al. 2000, Nature 403: 901-906) in the roundworm *C. elegans.* miRNAs have been evolutionarily conserved over a wide range of species and exhibit diversity in expression profiles, suggesting that they occupy a wide variety of regulatory functions and exert significant effects on cell growth and development (Ke et al. 2003, Curr.Opin. Chem. Biol. 7:516-523). Recent work has shown that miRNAs can regulate gene expression at many levels, representing a novel gene regulatory mechanism and supporting the idea that RNA is capable of performing similar regulatory roles as proteins. Understanding this RNA-based regulation will help us to understand the complexity of the genome in higher eukaryotes as well as understand the complex gene regulatory networks.

miRNAs are 18-25 nucleotide (nt) RNAs that are processed from longer endogenous hairpin transcripts (Ambros et al. 2003, RNA 9: 277-279). To date more than 1420 microRNAs have been identified in humans, worms, fruit flies and plants according to the miRNA registry database release 5.1 in December 2004, hosted by Sanger Institute, UK, and many miRNAs that correspond to putative genes have also been identified. Some miRNAs have multiple loci in the genome (Reinhart et al. 2002, Genes Dev. 16: 1616-1626) and occasionally, several miRNA genes are arranged in tandem clusters (Lagos-Quintana et al. 2001, Science 294: 853-858). The fact that many of the miRNAs reported to date have been isolated just once suggests that many new miRNAs will be discovered in the future. A recent in-depth transcriptional analysis of the human chromosomes 21 and 22 found that 49% of the observed transcription was outside of any known annotation, and furthermore, that these novel transcripts were both coding and non-coding RNAs (Kampa et al. 2004, Genome Research 14: 331-342). Another recent paper decribes the use of phylogenetic shadowing profiles to predict 976 novel candidate miRNA genes in the human genome (Berezikov et al. 2005, CeII120: 21-24) from whole-genome human/mouse and human/rat aligments. Most of the candidate miRNA genes were found to be conserved in other vertebrates, including dog, cow, chicken, opossum and zebrafish. Thus, the identified miRNAs to date represent most likely the tip of the iceberg, and the number of miRNAs might turn out to be very large.

The combined characteristics of microRNAs characterized to date (Ke et al. 2003, Curr.Opin. Chem. Biol. 7:516-523; Lee et al. 1993, Cell 75:843-854; Reinhart et al. 2000, Nature 403: 901-906) can be summarized as:
1. miRNAs are single-stranded RNAs of about 18-25 nt that regulate the expression of complementary messenger RNAs
2. They are cleaved from a longer endogenous double-stranded hairpin precursor by the enzyme Dicer.
3. miRNAs match precisely the genomic regions that can potentially encode precursor miRNAs in the form of double-stranded hairpins.
4. miRNAs and their predicted precursor secondary structures may be phylogenetically conserved.

Several lines of evidence suggest that the enzymes Dicer and Argonaute are crucial participants in miRNA biosynthesis, maturation and function (Grishok et al. 2001, Cell 106: 23-24). Mutations in genes required for miRNA biosynthesis lead to genetic developmental defects, which are, at least in part, derived from the role of generating miRNAs. The current view is that miRNAs are cleaved by Dicer from the hairpin precursor in the form of duplex, initially with 2 or 3 nt overhangs in the 3' ends, and are termed pre-miRNAs. Cofactors join the pre-miRNP (microRNA RiboNucleoProtein- complexes) and unwind the pre-miRNAs into single-stranded miRNAs, and pre-miRNP is then transformed to miRNP. miRNAs can recognize regulatory targets while part of the miRNP complex. There are several similarities between miRNP and the RNA-induced silencing complex, RISC, including similar sizes and both containing RNA helicase and the PPD proteins. It has therefore been proposed that miRNP and RISC are the same RNP with multiple functions (Ke et al. 2003, Curr.Opin. Chem. Biol. 7:516-523). Different effectors direct miRNAs into diverse pathways. The structure of pre-miRNAs is consistent with the observation that 22 nt RNA duplexes with 2 or 3 nt overhangs at the 3' ends are beneficial for reconstitution of the protein complex and might be required for high affinity binding of the short RNA duplex to the protein components (for review, see Ke et al. 2003, Curr.Opin. Chem. Biol. 7:516-523).

Growing evidence suggests that miRNAs play crucial roles in eukaryotic gene regulation. The first miRNAs genes to be discovered, lin-4 and let-7, base-pair incompletely to repeated elements in the 3' untranslated regions (UTRs) of other heterochronic genes, and regulate the translation directly and negatively by antisense RNA-RNA interaction (Lee et al. 1993, Cell 75:843-854; Reinhart et al. 2000, Nature 403: 901-906). Other miRNAs are thought to interact with target mRNAs by limited complementary and suppressed translation as well (Lagos-Quintana et al. 2001, Science 294: 853-858; Lee and Ambros 2001, Science 294: 858-862). Many studies have shown, however, that given a perfect complementarity between miRNAs and their target RNA, could lead to target RNA degradation rather than inhibit translation (Hutvagner and Zamore 2002, Science 297: 2056-2060), suggesting that the degree of complementarity determines their functions. By identifying sequences with near complementarity, several targets have been predicted, most of which appear to be potential transcriptional factors that are crucial in cell growth and development. The high percentage of predicted miRNA targets acting as developmental regulators and the conservation of target sites suggest that miRNAs are involved in a wide range of organism development and behaviour and cell fate decisions (for review, see Ke et al. 2003, Curr.Opin. Chem. Biol. 7:516-523). For example, John et al. 2004 (PLoS Biology 2: e363) used known mammalian miRNAs to scan the 3' untranslated regions (UTRs) from human, mouse and rat genomes for potential miRNA target sites using a scanning algorithm based on sequence complementarity between the mature miRNA and the target site, binding energy of the miRNA:mRNA duplex and evolutionary conservation. They identified a total of 2307 target mRNAs conserved across the mammals with more than one target site at 90% conservation of target site sequence and 660 target genes at 100% conservation level. Scanning of the two fish genomes; *Danio rerio* (zebrafish) and *Fugu rubripes* (Fugu) identified 1000 target genes with two or more conserved miRNA sites between the two fish species (John et al. 2004 PLoS Biology 2: e363). Among the predicted targets, particularly interesting groups included mRNA encoding transcription factors, components of the miRNA machinery, other proteins involved in the translational regulation as well as components of the ubiquitin machinery. In a recent paper, Lewis *et al.* (Lewis et al. 2005, Cell 120: 15-20) predicted regulatory mRNA targets of vertebrate microRNAs by identifying conserved complementarity to the so-called seed (comprising nucleotides 2 to 7) sequence of the miRNAs. In a comparative four-genome analysis of all the 3' UTRs, ca. 5300 human genes were implicated as miRNA targets, which represented ca 30% of the gene set used in the analysis. In another recent publication, Lim *et al.* (Lim et al. 2005, Nature 433: 769-773) showed that transfection of HeLa cells with miR-124, a brain-specific microRNA, caused the expression profile of the HeLa cells to shift towards that of brain, as revealed by genome-wide expression profiling of the HeLa mRNA pool. By comparison, delivery of miR-1 to the HeLa cells shifted the mRNA profile toward muscle, the tissue where miR-1 is preferentially expressed. Lim et al. (Lim et al. 2005, Nature 433: 769-773) subsequently showed that the 3' un-translated regions of the downregulated mRNAs had a significant propensity to pair to the seed sequence of the 5' end of the two miRNAs, thus implying that metazoan miRNAs can reduce the levels of many of their target mRNAs. Wang et al. 2004 (Genome Biology 5:R65) have developed and applied a computational algorithm to predict 95 Arabidopsis thaliana miRNAs, which included 12 known ones and 83 new miRNAs. The 83 new miRNAs were found to be conserved with more than 90% sequence identity between the Arabidopsis and rice genomes. Using the Smith-Waterman nucleotide-alignment algorithm to predict mRNA targets for the 83 new miRNAs and by focusing on target sites that were conserved in both Arabidopsis and rice, Wang *et al.* 2004 (Genome Biology 5:R65) predicted 371 mRNA targets with an average of 4.8 targets per miRNA. A large proportion of these mRNA targets encoded proteins with transcription regulatory activity. Brennecke *et al.* 2005 (Brennecke et al. 2005 PLoS Biology 3: e85) have systematically evaluated the minimal requirements for functional miRNA:mRNA target duplexes *in vivo* and have grouped the target sites into two categories. The so-called 5' dominant sites have sufficient complementarity to the 5'-end on the miRNA, so that little or no pairing with the 3'-end of the miRNA is needed. The second class comprises the so-called 3' compensatory sites, which have insufficient 5'-end pairing and require strong 3'-end duplex formation in order to be functional. In addition to presenting experimental examples from both types of miRNA:target pairing *in vivo,* Brennecke *et al.* 2005 (Brennecke et al. 2005 PLoS Biology 3: e85) provide evidence that a given miRNA has in average ca. 100 mRNA target sites, further supporting the notion that miRNAs can regulate the expression of a large fraction of the protein-coding genes in multicellular eukaryotes.

### MicroRNAs and human disease

Analysis of the genomic location of miRNAs indicates that they play important roles in human development and disease. Several human diseases have already been pinpointed in which miRNAs or their processing machinery might be implicated. One of them is spinal muscular atrophy (SMA), a paediatric neurodegenerative disease caused by reduced protein levels or loss-of-function mutations of the survival of motor neurons (SMN) gene (Paushkin et al. 2002, Curr.Opin.Cell Biol. 14: 305-312). Two proteins (Gemin3 and Gemin4) that are part of the SMN complex are also components of miRNPs, whereas it remains to be seen whether miRNA biogenesis or function is dysregulated in SMA and what effect this has on pathogenesis. Another neurological disease linked to mi/siRNAs is fragile X mental retardation (FXMR) caused by absence or mutations of the fragile X mental retardation protein (FMRP)(Nelson et al. 2003, TIBS 28: 534-540), and there are additional clues that miRNAs might play a role in other neurological diseases. Yet another interesting finding is that the miR-224 gene locus lies within the minimal candidate region of two different neurological diseases: early-onset Parkinsonism and X-linked mental retardation (Dostie et al. 2003, RNA: 9: 180-186). Links between cancer and miRNAs have also been recently described. The most frequent single genetic abnormality in chronic lymphocytic leukaemia (CLL) is a deletion localized to chromosome 13q14 (50% of the cases). A recent study determined that two different miRNA (miR15 and miR16) genes are clustered and located within the intron of LEU2, which lies within the deleted minimal region of the B-cell chronic lymphocytic leukaemia (B-CLL) tumour suppressor locus, and both genes are deleted or down-regulated in the majority of CLL cases (Calin et al. 2002, Proc. Natl. Acad. Sci.U.S.A. 99: 15524-15529). Calin *et al.* 2004 (Calin et al. 2004, Proc. Natl. Acad. Sci.U.S.A. 101: 2999-3004) have further investigated the possible involvement of microRNAs in human cancers on a genome-wide basis, by mapping 186 miRNA genes and compared their location to the location of previous reported non-random genetic alterations. Interestingly, they showed that microRNA genes are frequently located at fragile sites, as well as in minimal regions of loss of heterozygosity, minimal regions of amplification (minimal amplicons), or common breakpoint regions. Overall, 98 of 186 (52.5%) of the microRNA genes in their study were in cancer-associated genomic regions or in fragile sites. Moreover, by Northern blotting, Calin *et al.* 2004 (Calin et al. 2004, Proc. Natl. Acad. Sci.U.S.A. 101: 2999-3004) showed that several miRNAs located in deleted regions had low levels of expression in cancer samples. These data provide the first catalog of miRNA genes that may have roles in cancer and indicate that the full complement of human miRNAs may be extensively involved in different cancers.

In a recent study, Eis *et al.* (Eis et al. 2005, Proc. Natl. Acad. Sci.U.S.A. 102: 3627-3632) showed that the human miR-155 is processed from sequences present in BIC RNA, which is a spliced and polyadenylated non-protein-coding RNA that accumulates in lymphoma cells. The precursor of miR-155 is most likely a transient spliced or unspliced nuclear BIC transcript rather than accumulated BIC RNA, which is primarily cytoplasmic. Eis *et al.* (Eis et al. 2005, Proc. Natl. Acad. Sci.U.S.A. 102: 3627-3632) also observed that clinical isolates of several types of B cell lymphomas, including diffuse large B cell lymphoma (DLBCL), have 10- to 30-fold higher copy numbers of miR-155 than do normal circulating B cells. Significantly higher levels of miR-155 were present in DLBCLs with an activated B cell phenotype than with the germinal center phenotype. Because patients with activated B cell-type DLBCL have a poorer clinical prognosis, Eis et al. (Eis et al. 2005, Proc. Natl. Acad. Sci.U.S.A. 102: 3627-3632) propose that quantification of this microRNA would be diagnostically useful.

In another recent paper, Poy et al. (Poy et al. 2004, Nature 432: 226-230) identified a novel, evolutionarily conserved and pancreatic islet-specific miRNA (miR-375), and showed that overexpression of miR-375 suppressed glucose-induced insulin secretion, and conversely, inhibition of endogenous miR-375 function enhanced insulin secretion. The mechanism by which secretion is modified by miR-375 is independent of changes in glucose metabolism or intracellular Ca²⁺-signalling but correlated with a direct effect on insulin exocytosis. In the study, Myotrophin was validated as a target of miR-375. Inhibition of Myotrophin by small interfering (si)RNA mimicked the effects of miR-375 on glucose-stimulated insulin secretion and exocytosis. Poy et al. (Poy et al. 2004, Nature 432: 226-230) thus conclude that miR-375 is a regulator of insulin secretion and could constitute a novel pharmacological target for the treatment of diabetes.

Yet another recent publication by Johnson et al. (Johnson et al. 2005, Cell 120: 635-647) showed that the let-7 miRNA family negatively regulates RAS in two different *C. elegans* tissues and two different human cell lines. Another interesting finding was that let-7 is expressed in normal adult lung tissue but is poorly expressed in lung cancer cell lines and lung cancer tissue. Furthermore, the expression of let-7 inversely correlates with expression of RAS protein in lung cancer tissues, suggesting a possible causal relationship. Overexpression of let-7 inhibited growth of a lung cancer cell line in vitro, suggesting a causal relationship between let-7 and cell growth in these cells. The combined results of Johnson *et al.* (Johnson et al. 2005, Cell 120: 635-647) that let-7 expression is reduced in lung tumors, that several let-7 genes map to genomic regions that are often deleted in lung cancer patients, that overexpression of let-7 can inhibit lung tumor cell line growth, that the expression of the RAS oncogene is regulated by let-7,and that RAS is significantly overexpressed in lung tumor samples strongly implicate let-7 as a tumor suppressor in lung tissue and also suggests a possible mechanism.

In conclusion, it has been anticipated that connections between miRNAs and human diseases will only strengthen in parallel with the knowledge of miRNAs and the gene networks that they control. Moreover, the understanding of the regulation of RNA-mediated gene expression is leading to the development of novel therapeutic approaches that will be likely to revolutionize the practice of medicine (Nelson et al. 2003, TIBS 28: 534-540).

### Small interfering RNAs and RNAi

Some of the recent attention paid to small RNAs in the size range of 18 to 25 nt is due to the phenomenon RNA interference (RNAi), in which double-stranded RNA leads to the degradation of any RNA that is homologous (Fire et al. 1998, Nature 391: 806-811). RNAi relies on a complex and ancient cellular mechanism that has probably evolved for protection against viral attack and mobile genetic elements. A crucial step in the RNAi mechanism is the generation of short interfering RNAs (siRNAs), double-stranded RNAs that are about 22 nt long each. The siRNAs lead to the degradation of homologous target RNA and the production of more siRNAs against the same target RNA (Lipardi et al. 2001, Cell 107: 297-307). The present view for the mRNA degradation pathway of RNAi is that antiparallel Dicer dimers cleave long double-stranded dsRNAs to form siRNAs in an ATP-dependent manner. The siRNAs are then incorporated in the RNA-induced silencing complex (RISC) and ATP-dependent unwinding of the siRNAs activates RISC (Zhang et al. 2002, EMBO J. 21: 5875-5885; Nykänen et al. 2001, Cell 107: 309-321). The active RISC complex is thus guided to degrade the specific target mRNAs.

### Detection and analysis of microRNAs and siRNAs

The current view that miRNAs may represent a newly discovered, hidden layer of gene regulation has resulted in high interest among researchers around the world in the discovery of miRNAs, their targets and mechanism of action. Detection and analysis of these small RNAs is, however not trivial. Thus, the discovery of more than 1400 miRNAs to date has required taking advantage of their special features. First, the research groups have used the small size of the miRNAs as a primary criterion for isolation and detection. Consequently, standard cDNA libraries would lack miRNAs, primarily because RNAs that small are normally excluded by sixe selection in the cDNA library construction procedure. Total RNA from fly embryos, worms or HeLa cells have been size fractionated so that only molecules 25 nucleotides or smaller would be captured (Moss 2002, Curr.Biology 12: R138-R140). Synthetic oligomers have then been ligated directly to the RNA pools using T4 RNA ligase. Then the sequences have been reverse-transcribed, amplified by PCR, cloned and sequenced (Moss 2002, Curr.Biology 12: R138-R140). The genome databases have subsequently been queried with the sequences, confirming the origin of the miRNAs from these organisms as well as placing the miRNA genes physically in the context of other genes in the genome. The vast majority of the cloned sequences have been located in intronic regions or between genes, occasionally in clusters, suggesting that the tandemly arranged miRNAs are processed from a single transcript to allow coordinate regulation. Furthermore, the genomic sequences have revealed the fold-back structures of the miRNA precursors (Moss 2002, Curr.Biology 12: R138-R140).

The size and often low level of expression of different miRNAs require the use of sensitive and quantitative analysis tools. Due to their small size of 18-25 nt, the use of conventional quantitative real-time PCR for monitoring expression of mature miRNAs is excluded. Therefore, most miRNA researchers currently use Northern blot analysis combined with polyacrylamide gels to examine expression of both the mature and pre-miRNAs (Reinhart et al. 2000, Nature 403: 901-906; Lagos-Quintana et al. 2001, Science 294: 853-858; Lee and Ambros 2001, Science 294: 862-864). Primer extension has also been used to detect the mature miRNA (Zeng and Cullen 2003, RNA 9: 112-123). The disadvantage of all the gel-based assays (Northern blotting, primer extension, RNase protection assays etc.) as tools for monitoring miRNA expression includes low throughput and poor sensitivity. Consequently, a large amount of total RNA per sample is required for Northern analysis of miRNAs, which is not feasible when the cell or tissue source is limited.

DNA microarrays would appear to be a good alternative to Northern blot analysis to quantify miRNAs in a genome-wide scale, since microarrays have excellent throughput. Krichevsky et al. 2003 used cDNA microarrays to monitor the expression of miRNAs during neuronal development with 5 to 10 µg aliquot of input total RNA as target, but the mature miRNAs had to be separated from the miRNA precursors using micro concentrators prior to microarray hybridizations (Krichevsky et al. 2003, RNA 9: 1274-1281). Liu et al 2004 (Liu et al. 2004, Proc.Natl. Acad. Sci, U.S.A 101:9740-9744) have developed a microarray for expression profiling of 245 human and mouse miRNAs using 40-mer DNA oligonucleotide capture probes. Thomson *et al.* 2004 (Thomson et al. 2004, Nature Methods 1: 1-6) describe the development of a custom oligonucleotide microarray platform for expression profiling of 124 mammalian miRNAs conserved in human and mouse using oligonucleotide capture probes complementary to the mature microRNAs. The microarray was used in expression profiling of the 124 miRNAs in question in different adult mouse tissues and embryonic stages. A similar approach was used by Miska et al. 2004 (Genome Biology 2004; 5:R68) for the development of an oligoarray for expression profiling of 138 mammalian miRNAs, including 68 miRNAs from rat and monkey brains. Yet another approach was taken by Barad et al. 2004 (Genome Research 2004; 14: 2486-2494), who developed a 60-mer oligonucleotide microarray platform for known human mature miRNAs and their precursors. The drawback of all DNA-based oligonucleotide arrays regardless of the capture probe length is the requirement of high concentrations of labelled input target RNA for efficient hybridization and signal generation, low sensitivity for rare and low-abundant miRNAs, and the necessity for post-array validation using more sensitive assays such as real-time quantitative PCR, which is not currently feasible. In addition, at least in some array platforms discrimination of highly homologous miRNA differing by just one or two nucleotides could not be achieved, thus presenting problems in data interpretation, although the 60-mer microarray by Barad et al. 2004 (Genome Research 2004; 14: 2486-2494) appears to have adequate specificity.

A PCR approach has also been used to determine the expression levels of mature miRNAs (Grad et al. 2003, Mol. Cell 11: 1253-1263). This method is useful to clone miRNAs, but highly impractical for routine miRNA expression profiling, since it involves gel isolation of small RNAs and ligation to linker oligonucleotides. Allawi et al. (2004, RNA 10: 1153-1161) have developed a method for quantitation of mature miRNAs using a modified Invader assay. Although apparently sensitive and specific for the mature miRNA, the drawback of the Invader quantitation assay is the number of oligonucleotide probes and individual reaction steps needed for the complete assay, which increases the risk of cross-contamination between different assays and samples, especially when high-throughput analyses are desired. Schmittgen et al. (2004, Nucleic Acids Res. 32: e43) describe an alternative method to Northern blot analysis, in which they use real-time PCR assays to quantify the expression of miRNA precursors. The disadvantage of this method is that it only allows quantification of the precursor miRNAs, which does not necessarily reflect the expression levels of mature miRNAs. In order to fully characterize the expression of large numbers of miRNAs, it is necessary to quantify the mature miRNAs, such as those expressed in human disease, where alterations in miRNA biogenesis produce levels of mature miRNAs that are very different from those of the precursor miRNA. For example, the precursors of 26 miRNAs were equally expressed in non-cancerous and cancerous colorectal tissues from patients, whereas the expression of mature human miR143 and miR145 was greatly reduced in cancer tissues compared with non-cancer tissues, suggesting altered processing for specific miRNAs in human disease (Michael et al. 2003, Mol. Cancer Res. 1: 882-891). On the other hand, recent findings in maize with miR166 and miR165 in *Arabidopsis thaliana,* indicate that microRNAs act as signals to specify leaf polarity in plants and may even form movable signals that emanate from a signalling centre below the incipient leaf (Juarez et al. 2004, Nature 428: 84-88; Kidner and Martienssen 2004, Nature 428: 81-84).

Most of the miRNA expression studies in animals and plants have utilized Northern blot analysis, tissue-specific small RNA cloning and expression profiling by microarrays or real-time PCR of the miRNA hairpin precursors, as described above. However, these techniques lack the resolution for addressing the spatial and temporal expression patterns of mature miRNAs. Due to the small size of mature miRNAs, detection of them by standard RNA *in situ* hybridization has proven difficult to adapt in both plants and vertebrates, even though *in situ* hybridization has recently been reported in *A. thaliana* and maize using RNA probes corresponding to the stem-loop precursor miRNAs (Chen et al. 2004, Science 203: 2022-2025; Juarez et al. 2004, Nature 428: 84-88). Brennecke et al. 2003 (Cell 113: 25-36) and Mansfield et al. 2004 (Nature Genetics 36: 1079-83) report on an alternative method in which reporter transgenes, so-called sensors, are designed and generated to detect the presence of a given miRNA in an embryo. Each sensor contains a constitutively expressed reporter gene (e.g. lacZ or green fluorescent protein) harbouring miRNA target sites in its 3'-UTR. Thus, in cells that lack the miRNA in question, the transgene RNA is stable allowing detection of the reporter, whereas cells expressing the miRNA, the sensor mRNA is targeted for degradation by the RNAi pathway. Although sensitive, this approach is time-consuming since it requires generation of the expression constructs and transgenes. Furthermore, the sensor-based technique detects the spatiotemporal miRNA expression patterns via an indirect method as opposed to direct *in situ* hybridization of the mature miRNAs.

The large number of miRNAs along with their small size makes it difficult to create loss-of-function mutants for functional genomics analyses. Another potential problem is that many miRNA genes are present in several copies per genome occurring in different loci, which makes it even more difficult to obtain mutant phenotypes. Boutla et al. 2003 (Nucleic Acids Research 31: 4973-4980) describe the use of DNA antisense oligonucleotides complementary to 11 different miRNAs in *Drosophila* as well as their use to inactivate the miRNAs by injecting the DNA oligonucleotides into fly emryos. Of the 11 DNA antisense oligonucleotides, only 4 constructs showed severe interference with normal development, while the remaining 7 oligonucleotides didn't show any phenotypes presumably due to their inability to inhibit the miRNA in question. Thus, the succes rate for using DNA antisense oligonucleotides to inhibit miRNA function would most likely be too low to allow functional analyses of miRNAs on a larger, genomic scale. An alternative approach to this has been reported by Hutvagner et al. 2004 (PLoS Biology 2: 1-11), in which 2'-O-methyl antisense oligonucleotides could be used as potent and irreversible inhibitors of siRNA and miRNA function *in vitro* and *in vivo* in *Drosophila* and *C. elegans,* thereby inducing a loss-of-function phenotype. A drawback of this method is the need of high 2'-O-methyl oligonucleotide concentrations (100 micromolar) in transfection and injection experiments, which may be toxic to the animal.

In conclusion, the biggest challenge in detection, quantitation and functional analysis of the mature miRNAs as well as siRNAs using currently available methods is their small size of the of 18-25 nt and often low level of expression. The present invention provides the design and development of novel oligonucleotide compositions and probe sequences for accurate, highly sensitive and specific detection and functional analysis of miRNAs, their target mRNAs and siRNA transcripts.

### RNA editing and alternative splicing

RNA editing is used to describe any specific change in the primary sequence of an RNA molecule, excluding other mechanistically defined processes such as alternative splicing or polyadenylation. RNA alterations due to editing fall into two broad categories, depending on whether the change happens at the base or nucleotide level (Gott 2003, C. R. Biologies 326 901-908). RNA editing is quite widespread, occurring in mammals, viruses, marsupials, plants, flies, frogs, worms, squid, fungi, slime molds, dinoflagellates, kinetoplastid protozoa, and other unicellular eukaryotes. The current list most likely represents only the tip of the iceberg; based on the distribution of homologues of known editing enzymes, as RNA editing almost certainly occurs in many other species, including all metazoa. Since RNA editing can be regulated in a developmental or tissue-specific manner, it is likely to play a significant role in the etiology of human disease (Gott 2003, C. R. Biologies 326 901-908).

A common feature for eukaryotic genes is that they are composed of protein-encoding exons and introns. Introns are characterized by being excised from the pre-mRNA molecule in RNA splicing, as the sequences on each side of the intron are spliced together. RNA splicing not only provides functional mRNA, but is also responsible for generating additional diversity. This phenomenon is called alternative splicing, which results in the production of different mRNAs from the same gene. The mRNAs that represent isoforms arising from a single gene can differ by the use of alternative exons or retention of an intron that disrupts two exons. This process often leads to different protein products that may have related or drastically different, even antagonistic, cellular functions. There is increasing evidence indicating that alternative splicing is very widespread (Croft et al. Nature Genetics, 2000). Recent studies have revealed that at least 80% of the roughly 35,000 genes in the human genome are alternatively spliced (Kampa et al. 2004, Genome Research 14: 331-342). Clearly, by combining different types of modifications and thus generating different possible combinations of transcripts of different genes, alternative splicing together with RNA editing are potent mechanisms for generating protein diversity. Analysis of the alternative splice variants and RNA editing, in turn, represents a novel approach to functional genomics, disease diagnostics and pharmacogenomics.

### Misplaced control of alternative splicing as a causative agent for human disease

The detection of the detailed structure of the transcriptional output is an important goal for molecular characterization of a cell or tissue. Without the ability to detect and quantify the splice variants present in one tissue, the transcript content or the protein content cannot be described accurately. Molecular medical research shows that many cancers result in altered levels of splice variants, so an accurate method to detect and quantify these transcripts is required. Mutations that produce an aberrant splice form can also be the primary cause of such severe diseases such as spinal muscular dystrophy and cystic fibrosis.

Much of the study of human disease, indeed much of genetics is based upon the study of a few model organisms. The evolutionary stability of alternative splicing patterns and the degree to which splicing changes according to mutations and environmental and cellular conditions influence the relevance of these model systems. At present, there is little understanding of the rates at which alternative splicing patterns or RNA editing change, and the factors influencing these rates.

Previously, other analysis methods have been performed with the aim of detecting either splicing of RNA transcripts *per se* in yeast, or of detecting putative exon skipping splicing events in rat tissues, but neither of these approaches had sufficient resolution to estimate quantities of splice variants, a factor that could be essential to an understanding of the changes in cell life cycle and disease. Thus, improved methods are needed for nucleic acid hybridization and quantitation. The present method of invention enables discrimination between mRNA splice variants as well as RNA-edited transcripts and detects each variant in a nucleic acid sample, such as a sample derived from a patient in e.g. addressing the spatiotemporal expression patterns by RNA *in situ* hybridization.

### Antisense transcription in eukaryotes

RNA-mediated gene regulation is widespread in higher eukaryotes and complex genetic phenomena like RNA interference, co-suppression, transgene silencing, imprinting, methylation, and possibly position-effect variegation and transvection, all involve intersecting pathways based on or connected to RNA signalling (Mattick 2001; EMBO reports 2, 11: 986-991). Recent studies indicate that antisense transcription is a very common phenomenon in the mouse and human genomes (Okazaki et al. 2002; Nature 420: 563-573; Yelin et al. 2003, Nature Biotechnol.). Thus, antisense modulation of gene expression in eukaryotic cells, e.g. human cells appear to be a common regulatory mechanism. In light of this, the present invention provides a method for detection and functional analysis of non-coding antisense RNAs, as well as a method for detecting the overlapping regions between sense-antisense transcriptional units.

### Cancer diagnosis and identification of tumor origin

Cancer classification relies on the subjective interpretation of both clinical and histopathological information by eye with the aim of classifying tumors in generally accepted categories based on the tissue of origin of the tumor. However, clinical information can be incomplete or misleading. In addition, there is a wide spectrum in cancer morphology and many tumors are atypical or lack morphologic features that are useful for differential diagnosis. These diffculties may result in diagnostic confusion, with the need for mandatory second opinions in all surgical pathology cases (Tomaszewski and LiVolsi 1999, Cancer 86: 2198-2200).

Molecular diagnostics offer the promise of precise, objective, and systematic human cancer classification, but these tests are not widely applied because characteristic molecular markers for most solid tumors have yet to be identified. In the recent years microarray-based tumor gene expression profiling has been used for cancer diagnosis. However, studies are still limited and have utilized different array platforms making it difficult to compare the different datasets (Golub et al. 1999, Science 286: 531-537; Alizadeh et al. 2000, Nature 403: 503-511; Bittner et al. 2000, Nature 406: 536-540). In addition, comprehensive gene expression databases have to be developed, and there are no established analytical methods yet capable of solving complex, multiclass, gene expression-based classification problems.

Another problem for cancer diagnostics is the identification of tumor origin for metastatic carcinomas. For example, in the United States, 51,000 patients (4% of all new cancer cases) present annually with metastases arising from occult primary carcinomas of unknown origin (ACS Cancer Facts & Figures 2001: American Cancer Society). Adenocarcinomas represent the most common metastatic tumors of unknown primary site. Although these patients often present at a late stage, the outcome can be positively affected by accurate diagnoses followed by appropriate therapeutic regimens specific to different types of adenocarcinoma (Hillen 2000, Postgrad. Med. J. 76: 690-693). The lack of unique microscopic appearance of the different types of adenocarcinomas challenges morphological diagnosis of adenocarcinomas of unknown origin. The application of tumor-specific serum markers in identifying cancer type could be feasible, but such markers are not available at present (Milovic et al. 2002, Med. Sci. Monit. 8: MT25-MT30). Microarray expression profiling has recently been used to successfully classify tumors according to their site of origin (Ramaswamy et al. 2001, Proc. Natl. Acad. Sci.U.S.A. 98: 15149-15154), but the lack of a standard for array data collection and analysis make them difficult to use in a clinical setting. SAGE (serial analysis of gene expression), on the other hand, measures absolute expression levels through a tag counting approach, allowing data to be obtained and compared from different samples. The drawback of this method is, however, its low throughput, making it inappropriate for routine clinical applications. Quantitative real-time PCR is a reliable method for assessing gene expression levels from relatively small amounts of tissue (Bustin 2002, J. Mol. Endocrinol. 29: 23-39). Although this approach has recently been successfully applied to the molecular classification of breast tumors into prognostic subgroups based on the analysis of 2,400 genes (Iwao et al. 2002, Hum. Mol. Genet. 11: 199-206), the measurement of such a large number of randomly selected genes by PCR is clinically impractical.

Since the discovery of the first miRNA gene lin-4, in 1993, microRNAs have emerged as important non-coding RNAs, involved in a wide variety of regulatory functions during cell growth, development and differentiation. Furthermore, an expanding inventory of microRNA studies has shown that many miRNAs are mutated or down-regulated in human cancers, implying that miRNAs can act as tumor supressors or even oncogenes. Thus, detection and quantitation of all the microRNAs with a role in human disease, including cancers, would be highly useful as biomarkers for diagnostic purposes or as novel pharmacological targets for treatment. The biggest challenge, on the other hand, in detection and quantitation of the mature miRNAs using currently available methods is the small size of 18-25 nt and sometimes low level of expression.

The present invention solves the abovementioned problems by providing the design and development of novel oligonucleotide compositions and probe sequences for accurate, highly sensitive and specific detection and quantitation of microRNAs and other non-coding RNAs, useful as biomarkers for diagnostic purposes of human disease as well as for antisense-based intervention, which is targeted against tumorigenic miRNAs and other non-coding RNAs. The invention furthermore provides novel oligonucleotide compositions and probe sequences for sensitive and specific detection and quantitation of microRNAs, useful as biomarkers for the identification of the primary site of metastatic tumors of unknown origin.

### SUMMARY OF THE INVENTION

The challenges of establishing genome function and understanding the layers of information hidden in the complex transcriptomes of higher eukaryotes call for novel, improved technologies for detection and analysis of non-coding RNA and protein-coding RNA molecules in complex nucleic acid samples. Thus, it would be highly desirable to be able to detect and analyse the expression of mature microRNAs, siRNAs, RNA-edited transcripts as well as highly homologous splice variants in the transcriptomes of eukaryotes using methods based on specific and sensitive oligonucleotide detection probes.

The present invention solves the current problems faced by conventional approaches used in detection and analysis of mature miRNAs, their target mRNAs as well as siRNAs as outlined above by providing a method for the design, synthesis and use of novel oligonucleotide compositions and probe sequences with improved sensitivity and high sequence specificity for RNA target sequences, such as mature miRNAs and siRNAs- so that they are unlikely to detect a random RNA target molecule. Such oligonucleotide probes comprise a recognition sequence complementary to the RNA target sequence, which said recognition sequence is substituted with high-affinity nucleotide analogues, e.g. LNA, to increase the sensitivity and specificity of conventional oligonucleotides, such as DNA oligonucleotides, for hybridization to short target sequences, e.g. mature miRNAs, stem-loop precursor miRNAs, siRNAs or other non-coding RNAS as well as miRNA binding sites in their cognate mRNA targets, mRNAs, mRNA splice variants, RNA-edited mRNAs and antisense RNAs. The invention features a method of designing the detection probe sequences by selecting optimal substitution patterns for the high-affinity analogues, e.g. LNAs for the detection probes. This method involves (a) substituting the detection probe sequence with the high affinity analogue LNA in chimeric LNA-DNA oligonucleotides using regular spacing between the LNA substitutions, e.g. at every second nucleotide position, every third nucleotide position, or every fourth nucleotide position, in order to promote the A-type duplex geometry between the substituted detection probe and its complementary RNA target; with the said LNA monomer substitutions spiked in all the possible phases in the probe sequence with an unsubstituted monomer at the 5'-end position and 3'-end position in all the substituted designs; (b) determining the ability of the designed detection probes with different regular substitution patterns to self-anneal; and (c) determining the melting temperature of the substituted probes sequences of the invention, and (d) selecting the probe sequences with the highest melting temperatures and lowest self-complementarity score, i.e. lowest ability to self-anneal are selected.

Another aspect the invention features a method of designing the detection probe sequences by selecting optimal substitution patterns for the LNAs, which said method involves substituting the detection probe sequence with the high affinity analogue LNA in chimeric LNA-DNA oligonucleotides using irregular spacing between the LNA monomers and selecting the probe sequences with the highest melting temperatures and lowest self-complementarity score. In yet another aspect the invention features a computer code for a preferred software program of the invention for the design and selection of the said substituted detection probe sequences.

The present invention hence also relates to a collection of detection probes, wherein each member of said collection comprises a recognition sequence consisting of nucleobases and affinity enhancing nucleobase analogues, and wherein the recognition sequences exhibit a combination of high melting temperatures and low self-complementarity scores, said melting temperatures being the melting temperature of the duplex between the recognition sequence and its complementary DNA or RNA sequence.

Also single probes taken from such a collection form part of the present invention.

The invention also relates to a method for A method for expanding or building a collection defined above, comprising
A) defining a reference nucleotide sequence consisting of nucleobases, said reference nucleotide sequence being complementary to a target sequence for which the collection does not contain a detection probe,
B) substituting the reference nucleotide sequence's nucleobases with affinity enhancing nucleobase analogues to provide a set of chimeric sequences wherein,
C) determining usefulness of each of the chimeric sequences based on assessment of their ability to self-anneal and their melting temperature, and
D) synthesizing and adding, to the collection, a probe comprising as its recognition sequence the chimeric sequence with the optimum combination of high melting temperature and low self-annealing.

Also part of the invention is a method for designing an optimized detection probe for a target nucleotide sequence, comprising
1) defining a reference nucleotide sequence consisting of nucleobases, said reference nucleotide sequence being complementary to said target nucleotide sequence,
2) substituting the reference nucleotide sequence's nucleobases with affinity enhancing nucleobase analogues to provide a set of chimeric sequences
3) determining usefulness of each of the chimeric sequences based on assessment of their ability to self-anneal and their melting temperatures, and
4) defining the optimized detection probe as the one in the set having as its recognition sequence the chimeric sequence with the optimum combination of high melting temperature and low self-annealing.

Furthermore, the present invention also relates to a computer system for designing an optimized detection probe for a target nucleic acid sequence, said system comprising
a) input means for inputting the target nucleotide,
b) storage means for storing the target nucleotide sequence,
c) optionally executable code which can calculate a reference nucleotide sequence being complementary to said target nucleotide sequence and/or input means for inputting the reference nucleotide sequence,
d) optionally storage means for storing the reference nucleotide sequence,
e) executable code which can generate chimeric sequences from the reference nucleotide sequence or the target nucleic acid sequence, wherein said chimeric sequences comprise the reference nucleotide sequence, wherein has been in-substituted affinity enhancing nucleobase analogues,
f) executable code which can determine the usefulness of such chimeric sequences based on assessment of their ability to self-anneal and their melting temperatures and either rank such chimeric sequences according to their usefulness,
g) storage means for storing at least one chimeric sequence, and
h) output means for presenting the sequence of at least one optimized detection probe.

Also a storage means embedding executable code (e.g. a computer program) which executes the design steps of the method referred to above is part of the present invention.

Further, the present invention also relates to a method for specific isolation, purification, amplification, detection, identification, quantification, inhibition or capture of a target nucleotide sequence in a sample, said method comprising contacting said sample with a member of a collection defined above under conditions that facilitate hybridization between said member/probe and said target nucleotide sequence.

In another aspect the invention features detection probe sequences containing a ligand, which said ligand means something, which binds. Such ligand-containing detection probes of the invention are useful for isolating target RNA molecules from complex nucleoc acid mixtures, such as miRNAs, their cognate target mRNAs and siRNAs. Ligands comprise biotin and functional groups such as: aromatic groups (such as benzene, pyridine, naphtalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicar-bazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, C1-C20 alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-β-alanine, polyglycine, polylysine, peptides, oligo/polysaccharides, oligo/polyphosphates, toxins, antibiotics, cell poisons, and steroids, and also affinity ligands, i.e. functional groups or biomolecules that have a specific affinity for sites on particular proteins, antibodies, poly- and oligosaccharides, and other biomolecules.

In another aspect the invention features detection probe sequences, which said sequences have been furthermore modified by Selectively Binding Complementary (SBC) nucleobases, i.e. modified nucleobases that can make stable hydrogen bonds to their complementary nucleobases, but are unable to make stable hydrogen bonds to other SBC nucleobases. Such SBC monomer substitutions are especially useful when highly self-complementary detection probe sequences are employed. As an example, the SBC nucleobase A', can make a stable hydrogen bonded pair with its complementary unmodified nucleobase, T. Likewise, the SBC nucleobase T' can make a stable hydrogen bonded pair with its complementary unmodified nucleobase, A. However, the SBC nucleobases A' and T' will form an unstable hydrogen bonded pair as compared to the base pairs A'-T and A-T'. Likewise, a SBC nucleobase of C is designated C' and can make a stable hydrogen bonded pair with its complementary unmodified nucleobase G, and a SBC nucleobase of G is designated G' and can make a stable hydrogen bonded pair with its complementary unmodified nucleobase C, yet C' and G' will form an unstable hydrogen bonded pair as compared to the base pairs C'-G and C-G'. A stable hydrogen bonded pair is obtained when 2 or more hydrogen bonds are formed e.g. the pair between A' and T, A and T', C and G', and C' and G. An unstable hydrogen bonded pair is obtained when 1 or no hydrogen bonds is formed e.g. the pair between A' and T', and C' and G'. Especially interesting SBC nucleobases are 2,6-diaminopurine (A', also called D) together with 2-thio-uracil (U', also called 2SU)(2-thio-4-oxo-pyrimidine) and 2-thio-thymine (T', also called 2ST)(2-thio-4-oxo-5-methyl-pyrimidine).

In another aspect the detection probe sequences of the invention are covalently bonded to a solid support by reaction of a nucleoside phosphoramidite with an activated solid support, and subsequent reaction of a nucleoside phosphoramide with an activated nucleotide or nucleic acid bound to the solid support. In some embodiments, the solid support or the detection probe sequences bound to the solid support are activated by illumination, a photogenerated acid, or electric current. In other embodiments the detection probe sequences contain a spacer, e.g. a randomized nucleotide sequence or a non-base sequence, such as hexaethylene glycol, between the reactive group and the recognition sequence. Such covalently bonded detection probe sequence populations are highly useful for large-scale detection and expression profiling of mature miRNAs, stem-loop precursor miRNAs, siRNAs and other non-coding RNAs.

The present oligonucleotide compositions and detection probe sequences of the invention are highly useful and applicable for detection of individual small RNA molecules in complex mixtures composed of hundreds of thousands of different nucleic acids, such as detecting mature miRNAs, their target mRNAs or siRNAs, by Northern blot analysis or for addressing the spatiotemporal expression patterns of miRNAs, siRNAs or other non-coding RNAs as well as mRNAs by in situ hybridization in whole-mount embryos, whole-mount animals or plants or tissue sections of plants or animals, such as human, mouse, rat, zebrafish, *Caenorhabditis elegans, Drosophila melanogaster, Arabidopsis thaliana,* rice and maize. The present oligonucleotide compositions and detection probe sequences of invention are furthermore highly useful and applicable for large-scale and genome-wide expression profiling of mature miRNAs, siRNAs or other non-coding RNAs in animals and plants by oligonucleotide microarrays. The present oligonucleotide compositions and detection probe sequences are furthermore highly useful in functional analysis of miRNAs, siRNAs or other non-coding RNAs *in vitro* and *in vivo* in plants or animals, such as human, mouse, rat, zebrafish, *Caenorhabditis elegans, Drosophila melanogaster, Arabidopsis thaliana,* rice and maize, by inhibiting their mode of action, e.g. the binding of mature miRNAs to their cognate target mRNAs. The oligonucleotide compositions and detection probe sequences of invention are also applicable to detecting, testing, diagnosing or quantifying miRNAs, siRNAs, other non-coding RNAs, RNA-edited transcripts or alternative mRNA splice variants implicated in or connected to human disease in complex human nucleic acid samples, e.g. from cancer patients. The oligonucleotide compositions and probe sequences are especially applicable for accurate, highly sensitive and specific detection and quantitation of microRNAs and other non-coding RNAs, which are useful as biomarkers for diagnostic purposes of human diseases, such as cancers, as well as for antisense-based intervention, targeted against tumorigenic miRNAs and other non-coding RNAs. The novel oligonucleotide compositions and probe sequences are furthermore applicable for sensitive and specific detection and quantitation of microRNAs, which can be used as biomarkers for the identification of the primary site of metastatic tumors of unknown origin.

### Brief Description Of The Drawings

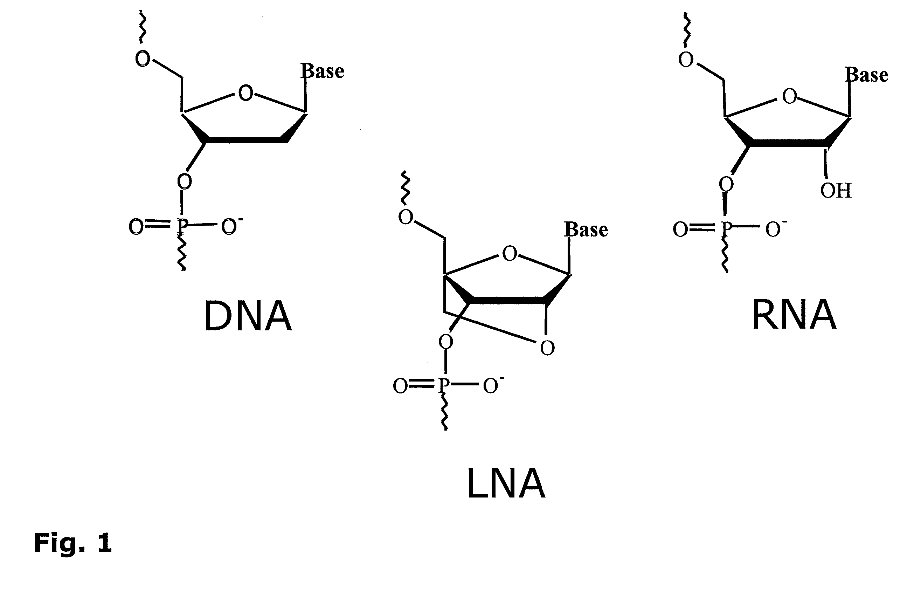

Fig. 1: The structures of DNA, LNA and RNA nucleosides.

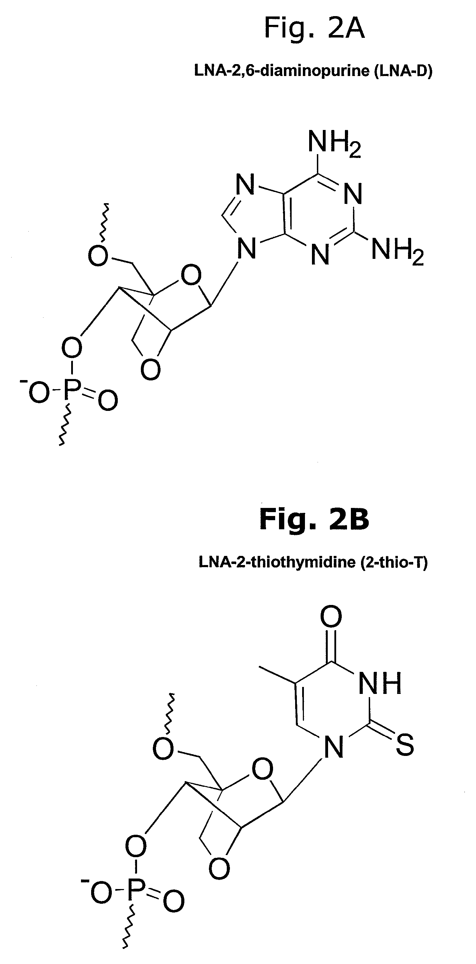

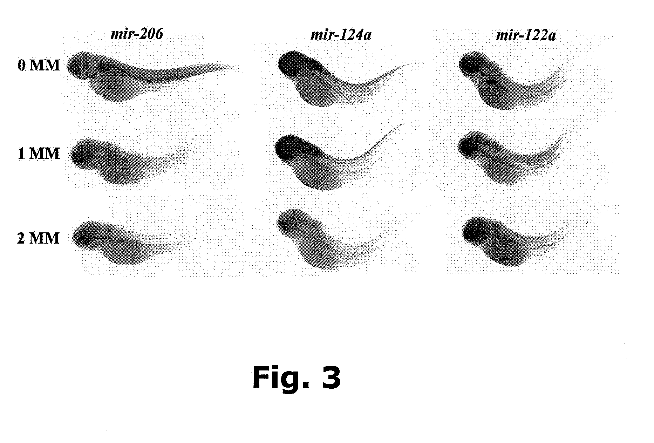

Fig. 2: The structures of LNA 2,6-diaminopurine and LNA 2-thiothymidine nucleosides.
Fig.: 3. The specificity of microRNA detection by *in situ* hybridization with LNA-substituted probes.
   The LNA probes containing one 1 MM) or two (2 MM) mismatches were designed for the three different miRNAs miR-206, miR-124a and miR-122a (see Table 3 below). The hybridizations were performed on embryos at 72 hours post fertilization at the same temperature as the perfect match probe (0 MM).

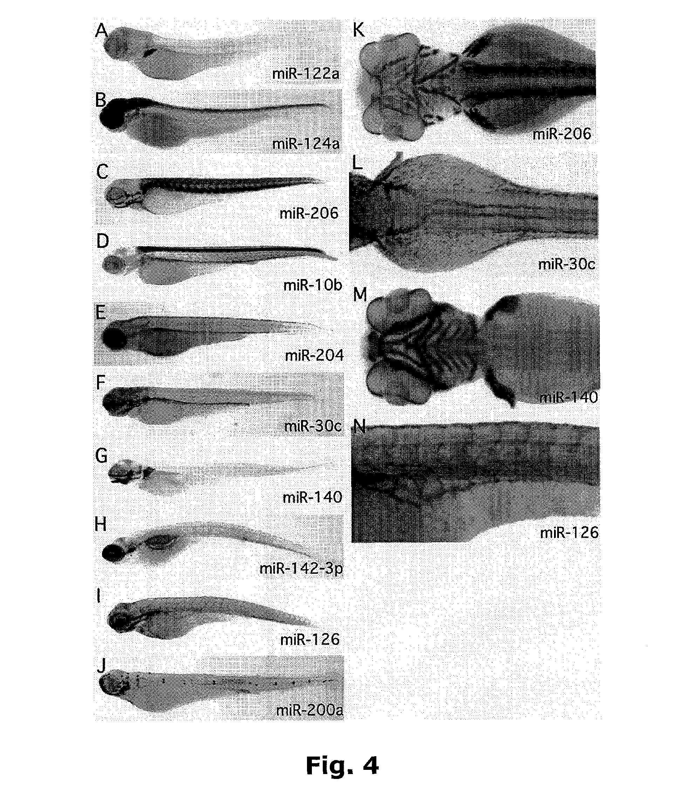

Fig. 4: Examples of miRNA whole-mount in situ expression patterns in zebrafish detected by LNA-substituted probes.
   Representatives for miRNAs expressed in the organ systems are shown. miRNAs were expressed in: (A) liver of the digestive system, (B) brain, spinal cord and cranial nerves/ganglia of the central and peripheral nervous systems, (C, M) muscles, (D) restricted parts along the head-to-tail axis, (E) pigment cells of the skin, (F, L) pronephros and presumably mucous cells of the excretory system, (G, M) cartilage of the skeletal system, (H) thymus, (I, N) blood vessels of the circulatory system, (J) lateral line system of the sensory organs. Embryos in (K, L, M, N) are higher magnifications of the embryos in (C, D, G, I), respectively. (A-J, N) are lateral views; (K-M) are dorsal views. All embryos are 72 hours post fertilization, except for (H), which is a five-day old larva.

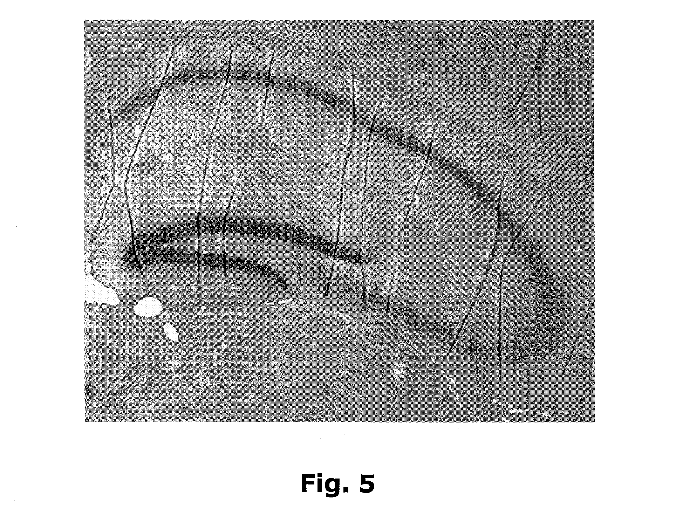

Fig. 5: Detection of let-7a miRNA by in situ hybridization in paraffin-embedded mouse brain sections using 3' digoxigenin-labeled LNA probe.
   Part of the hippocampus can be seen as an arrow-like structure.

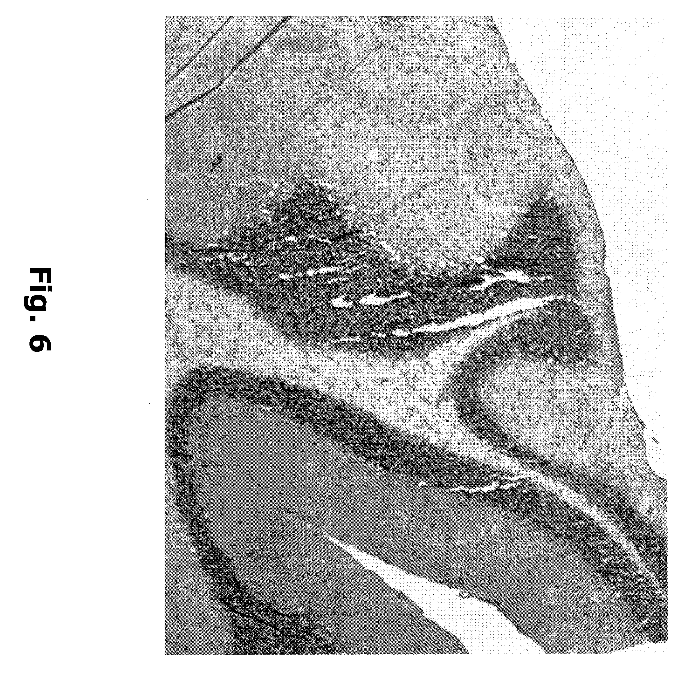

Fig. 6: Detection of let-7a miRNA by in situ hybridization in paraffin-embedded mouse brain sections using 3' digoxigenin-labeled LNA probe.
   The Purkinje cells can be seen in the cerebellum.

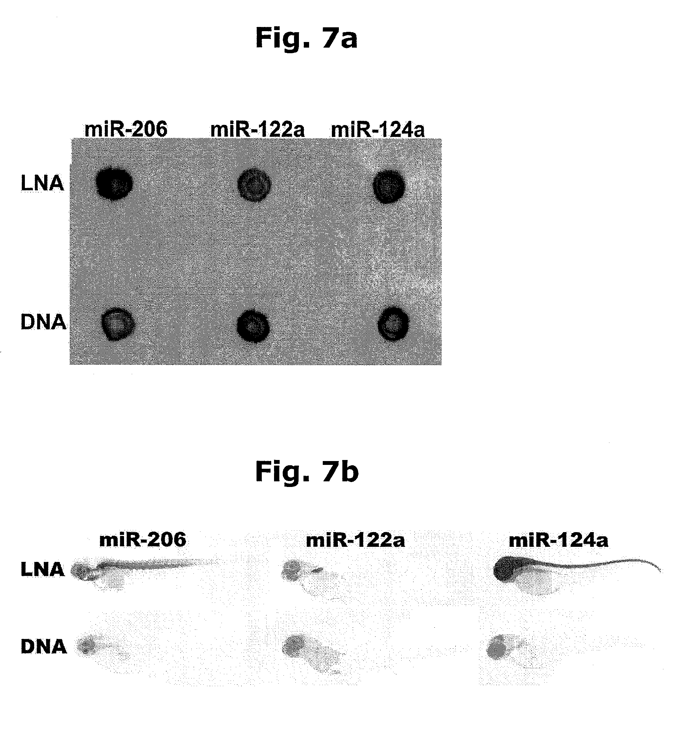

Fig. 7: Detection of miR-124a, miR-122a and miR-206 with DIG-labeled DNA and LNA probes in 72h zebrafish embryos.
   (a) Dot-blot of DIG labeled DNA and LNA probes. Per probe, 1 pmol was spotted on a positively charged nylon membrane. All probes show approximately equal incorporation of the DIG-label.
   (b) Only LNA probes give clear staining. LNA probes were hybridized at 59 °C (miR-122a and miR-124a) and 54 °C (miR-206). DNA probes were hybridized at 45°C.

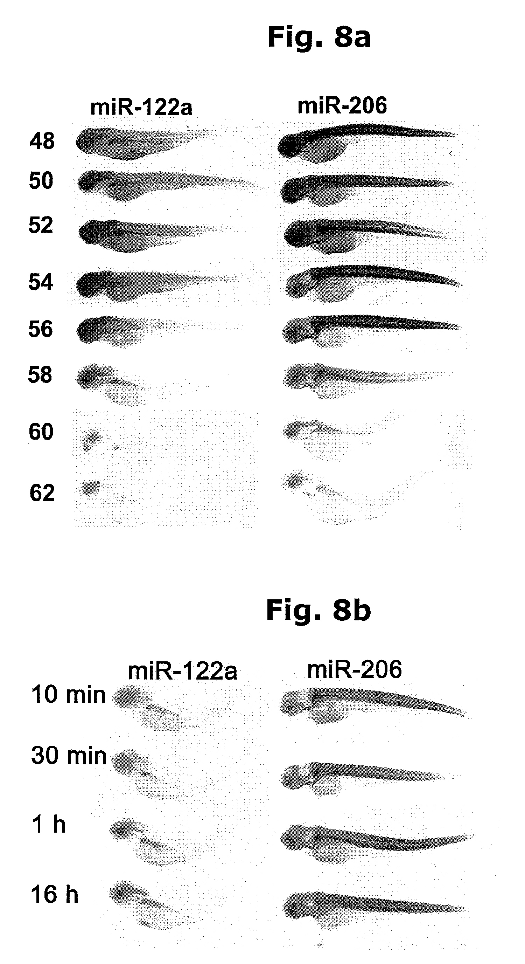

Fig. 8: Determination of the optimal hybridization temperature and time for in situ hybridization on 72h zebrafish embryos using LNA probes.
   (a) LNA probes for miR-122a and miR-206 were hybridized at different temperatures. The optimal hybridization temperature lies around 21 °C below the calculated Tm of the probe. While specific staining remains at the lower temperatures, background increases significantly. At higher temperatures staining is completely lost.
   (b) Hybridization time series with probes for miR-122a and miR-206. An incubation time of 10 min is already sufficient to get a detectable signal, while increasing the hybridization time beyond one hour does not increase the signal significantly. All in situ hybridizations were performed in parallel.

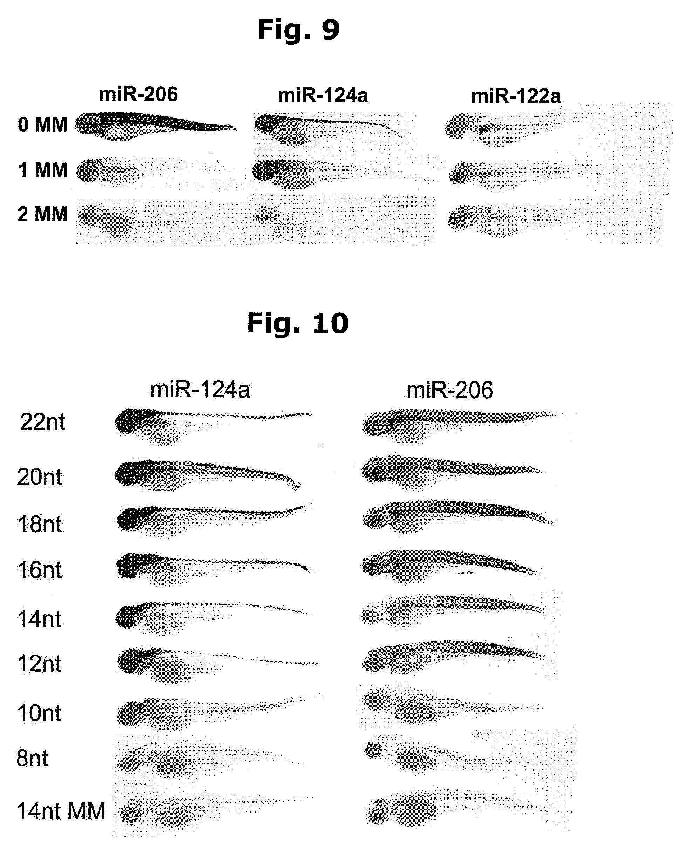

Fig. 9: Assessment of the specificity of LNA probes using perfectly matched and mismatched probes for the detection of miR-124a, miR-122a and miR-206 by in situ hybridization on 72h zebrafish embryos.
   Mismatched probes were hybridized under the same conditions as the perfectly matching probe. In most cases a central single mismatch is sufficient to loose signal. For the very highly expressed miR-124a specific staining was only lost upon introduction of two consecutive central mismatches in the probe.
Fig. 10: In situ detection of miR-124a and miR-206 in 72h zebrafish embryos using shorter LNA probe versions.
   In situ hybridizations were performed with probes of 2, 4, 6, 8, 10, 12 and 14 nt shorter than the original 22nt probes. Signals of probes that were 14 nt in length still resulted in readily detectable and specific signals. A single central mismatch in the 14 nt probes for miR-124a and miR-206 prevents hybridization. Probes that were 12 nt in length gave slightly reduced staining for both miR-124a and miR-206. Staining was virtually lost when 10 and 8 nt probes were used, although weak staining in the brain could still be observed for the highly expressed miR-124a.

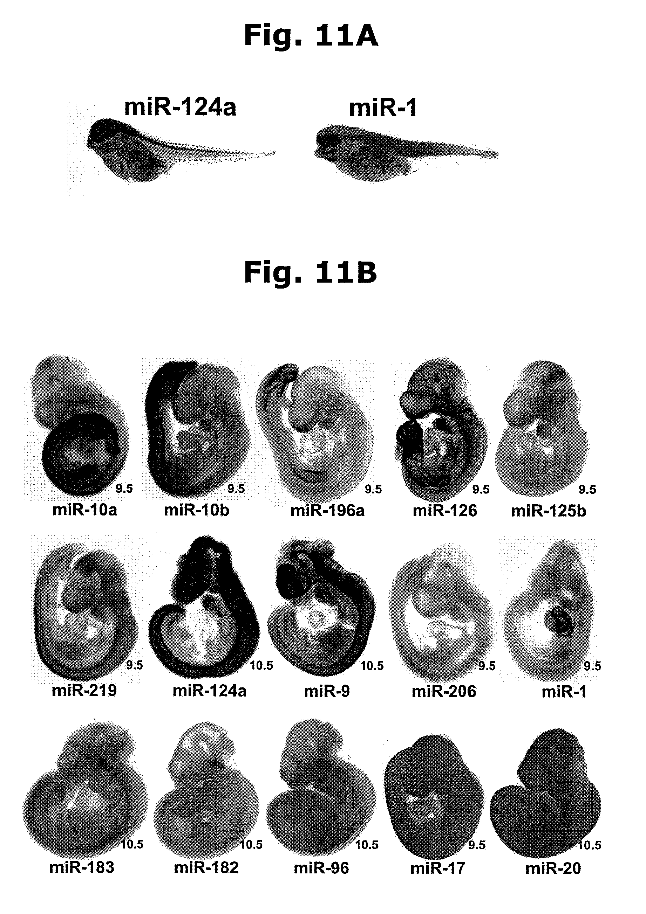

Fig. 11: In situ hybridizations for miRNAs on Xenopus tropicalis and mouse embryos.
   (a) Expression of miR-1 is restricted to the muscles in the body and the head in X. tropicalis. miR-124a is expressed throughout the central nervous system.
   (b) Expression of 15 miRNAs in 9.5 and 10.5 dpc (days post coitum) mouse embryos: miR-10a and 10b, posterior trunk; miR-196a, tailbud; miR-126, blood vessels; miR-125b, midbrain hindbrain boundary; miR-219, midbrain, hindbrain and spinal cord; miR-124a, central nervous system; miR-9, forebrain and the spinal cord; miR-206, somites; miR-1, heart and somites; miR-182, miR-96 and miR-183, cranial and dorsal root ganglia; miR-17-5p and miR-20 are expressed ubiquitously, like the other members of its genomic cluster.

### Definitions

For the purposes of the subsequent detailed description of the invention the following definitions are provided for specific terms, which are used in the disclosure of the present invention:

In the present context "ligand" means something, which binds. Ligands comprise biotin and functional groups such as: aromatic groups (such as benzene, pyridine, naphtalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, C₁-C₂₀ alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-β-alanine, polyglycine, polylysine, peptides, oligo/polysaccharides, oligo/polyphosphates, toxins, antibiotics, cell poisons, and steroids, and also "affinity ligands", i.e. functional groups or biomolecules that have a specific affinity for sites on particular proteins, antibodies, poly- and oligosaccharides, and other biomolecules.

The singular form "a", "an" and "the" include plural references unless the context clearly dictates otherwise. For example, the term "a cell" includes a plurality of cells, including mixtures thereof. The term "a nucleic acid molecule" includes a plurality of nucleic acid molecules.

"Transcriptome" refers to the complete collection of transcriptional units of the genome of any species. In addition to protein-coding mRNAs, it also represents non-coding RNAs, such as small nucleolar RNAs, siRNAs, microRNAs and antisense RNAs, which comprise important structural and regulatory roles in the cell.

A "multi-probe library" or "library of multi-probes" comprises a plurality of multi- probes, such that the sum of the probes in the library are able to recognise a major proportion of a transcriptome, including the most abundant sequences, such that about 60%, about 70%, about 80%, about 85%, more preferably about 90%, and still more preferably 95%, of the target nucleic acids in the transcriptome, are detected by the probes.

"Sample" refers to a sample of cells, or tissue or fluid isolated from an organism or organisms, including but not limited to, for example, skin, plasma, serum, spinal fluid, lymph fluid, synovial fluid, urine, tears, blood cells, organs, tumours, and also to samples of *in vitro* cell culture constituents (including but not limited to conditioned medium resulting from the growth of cells in cell culture medium, recombinant cells and cell components).

An "organism" refers to a living entity, including but not limited to, for example, human, mouse, rat, *Drosophila, C. elegans,* yeast, *Arabidopsis thaliana,* maize, rice, zebra fish, primates, domestic animals, etc.

The terms "Detection probes" or "detection probe" or "detection probe sequence" refer to an oligonucleotide, which oligonucleotide comprises a recognition sequence complementary to a RNA (or DNA) target sequence, which said recognition sequence is substituted with high-affinity nucleotide analogues, e.g. LNA, to increase the sensitivity and specificity of conventional oligonucleotides, such as DNA oligonucleotides, for hybridization to short target sequences, e.g. mature miRNAs, stem-loop precursor miRNAs, pri-miRNAs, siRNAs or other non-coding RNAs as well as miRNA binding sites in their cognate mRNA targets, mRNAs, mRNA splice variants, RNA-edited mRNAs and antisense RNAs.

The terms "miRNA" and "microRNA" refer to 18-25 nt non-coding RNAs derived from endogenous genes. They are processed from longer (ca 75 nt) hairpin-like precursors termed pre-miRNAs. MicroRNAs assemble in complexes termed miRNPs and recognize their targets by antisense complementarity. If the microRNAs match 100% their target, i.e. the complementarity is complete, the target mRNA is cleaved, and the miRNA acts like a siRNA. If the match is incomplete, i.e. the complementarity is partial, then the translation of the target mRNA is blocked.

The terms "Small interfering RNAs" or "siRNAs" refer to 21-25 nt RNAs derived from processing of linear double-stranded RNA. siRNAs assemble in complexes termed RISC (RNA-induced silencing complex) and target homologous RNA sequences for endonucleolytic cleavage. Synthetic siRNAs also recruit RISCs and are capable of cleaving homologous RNA sequences

The term "RNA interference" (RNAi) refers to a phenomenon where double-stranded RNA homologous to a target mRNA leads to degradation of the targeted mRNA. More broadly defined as degradation of target mRNAs by homologous siRNAs.

The term "Recognition sequence" refers to a nucleotide sequence that is complementary to a region within the target nucleotide sequence essential for sequence-specific hybridization between the target nucleotide sequence and the recognition sequence.

The term "label" as used herein refers to any atom or molecule which can be used to provide a detectable (preferably quantifiable) signal, and which can be attached to a nucleic acid or protein. Labels may provide signals detectable by fluorescence, radioactivity, colorimetric, X-ray diffraction or absorption, magnetism, enzymatic activity, and the like.

As used herein, the terms "nucleic acid", "polynucleotide" and "oligonucleotide" refer to primers, probes, oligomer fragments to be detected, oligomer controls and unlabelled blocking oligomers and shall be generic to polydeoxyribonucleotides (containing 2-deoxy-D-ribose), to polyribonucleotides (containing D-ribose), and to any other type of polynucleotide which is an N glycoside of a purine or pyrimidine base, or modified purine or pyrimidine bases. There is no intended distinction in length between the term "nucleic acid", "polynucleotide" and "oligonucleotide", and these terms will be used interchangeably. These terms refer only to the primary structure of the molecule. Thus, these terms include double-and single-stranded DNA, as well as double- and single stranded RNA. The oligonucleotide is comprised of a sequence of approximately at least 3 nucleotides, preferably at least about 6 nucleotides, and more preferably at least about 8 - 30 nucleotides corresponding to a region of the designated target nucleotide sequence. "Corresponding" means identical to or complementary to the designated sequence. The oligonucleotide is not necessarily physically derived from any existing or natural sequence but may be generated in any manner, including chemical synthesis, DNA replication, reverse transcription or a combination thereof.

The terms "oligonucleotide" or "nucleic acid" intend a polynucleotide of genomic DNA or RNA, cDNA, semi synthetic, or synthetic origin which, by virtue of its origin or manipulation: (1) is not associated with all or a portion of the polynucleotide with which it is associated in nature; and/or (2) is linked to a polynucleotide other than that to which it is linked in nature; and (3) is not found in nature. Because mononucleotides are reacted to make oligonucleotides in a manner such that the 5'-phosphate of one mononucleotide pentose ring is attached to the 3' oxygen of its neighbour in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have a 5' and 3' ends. When two different, non-overlapping oligonucleotides anneal to different regions of the same linear complementary nucleic acid sequence, the 3' end of one oligonucleotide points toward the 5' end of the other; the former may be called the "upstream" oligonucleotide and the latter the "downstream" oligonucleotide.

By the term "SBC nucleobases" is meant "Selective Binding Complementary" nucleobases, i.e. modified nucleobases that can make stable hydrogen bonds to their complementary nucleobases, but are unable to make stable hydrogen bonds to other SBC nucleobases. As an example, the SBC nucleobase A', can make a stable hydrogen bonded pair with its complementary unmodified nucleobase, T. Likewise, the SBC nucleobase T' can make a stable hydrogen bonded pair with its complementary unmodified nucleobase, A. However, the SBC nucleobases A' and T' will form an unstable hydrogen bonded pair as compared to the base pairs A'-T and A-T'. Likewise, a SBC nucleobase of C is designated C' and can make a stable hydrogen bonded pair with its complementary unmodified nucleobase G, and a SBC nucleobase of G is designated G' and can make a stable hydrogen bonded pair with its complementary unmodified nucleobase C, yet C' and G' will form an unstable hydrogen bonded pair as compared to the base pairs C'-G and C-G'. A stable hydrogen bonded pair is obtained when 2 or more hydrogen bonds are formed e.g. the pair between A' and T, A and T', C and G', and C' and G. An unstable hydrogen bonded pair is obtained when 1 or no hydrogen bonds is formed e.g. the pair between A' and T', and C' and G'. Especially interesting SBC nucleobases are 2,6-diaminopurine (A', also called D) together with 2-thio-uracil (U', also called ^{2S}U) (2-thio-4-oxo-pyrimidine) and 2-thio-thymine (T', also called ^{2S}T)(2-thio-4-oxo-5-methyl-pyrimidine). Figure 4 in PCT Publication No. WO 2004/024314 illustrates that the pairs A-^{2S}T and D-T have 2 or more than 2 hydrogen bonds whereas the D-^{2S}T pair forms a single (unstable) hydrogen bond. Likewise the SBC nucleobases pyrrolo-[2,3-d]pyrimidine-2(3H)-one (C', also called PyrroloPyr) and hypoxanthine (G', also called I)(6-oxo-purine) are shown in Figure 4 in PCT Publication No. WO 2004/024314 where the pairs PyrroloPyr-G and C-I have 2 hydrogen bonds each whereas the PyrroloPyr-I pair forms a single hydrogen bond.

"SBC LNA oligomer" refers to a "LNA oligomer" containing at least one LNA monomer where the nucleobase is a "SBC nucleobase". By "LNA monomer with an SBC nucleobase" is meant a "SBC LNA monomer". Generally speaking SBC LNA oligomers include oligomers that besides the SBC LNA monomer(s) contain other modified or naturally occurring nucleotides or nucleosides. By "SBC monomer" is meant a non-LNA monomer with a SBC nucleobase. By "isosequential oligonucleotide" is meant an oligonucleotide with the same sequence in a Watson-Crick sense as the corresponding modified oligonucleotide e.g. the sequences agTtcATg is equal to agTscD^{2S}Ug where s is equal to the SBC DNA monomer 2-thio-t or 2-thio-u, D is equal to the SBC LNA monomer LNA-D and ^{2S}U is equal to the SBC LNA monomer LNA ^{2S}U.

The complement of a nucleic acid sequence as used herein refers to an oligonucleotide which, when aligned with the nucleic acid sequence such that the 5' end of one sequence is paired with the 3' end of the other, is in "antiparallel association." Bases not commonly found in natural nucleic acids may be included in the nucleic acids of the present invention include, for example, inosine and 7-deazaguanine. Complementarity may not be perfect; stable duplexes may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

Stability of a nucleic acid duplex is measured by the melting temperature, or "Tₘ". The Tₘ of a particular nucleic acid duplex under specified conditions is the temperature at which half of the duplexes have disassociated.

The term "nucleobase" covers the naturally occurring nucleobases adenine (A), guanine (G), cytosine (C), thymine (T) and uracil (U) as well as non-naturally occurring nucleobases such as xanthine, diaminopurine, 8-oxo-N⁶-methyladenine, 7-deazaxanthine, 7-deazaguanine, N⁴,N⁴-ethanocytosin, N⁶,N⁶-ethano-2,6-diaminopurine, 5-methylcytosine, 5-(C³-C⁶)-alkynyl-cytosine, 5-fluorouracil, 5-bromouracil, pseudoisocytosine, 2-hydroxy-5-methyl-4-triazolopyridin, isocytosine, isoguanine, inosine and the "non-naturally occurring" nucleobases described in Benner et al., U.S. Patent No. 5,432,272 and Susan M. Freier and Karl-Heinz Altmann, Nucleic Acid Research,25: 4429-4443, 1997. The term "nucleobase" thus includes not only the known purine and pyrimidine heterocycles, but also heterocyclic analogues and tautomers thereof. Further naturally and non naturally occurring nucleobases include those disclosed in U.S. Patent No. 3,687,808; in chapter 15 by Sanghvi, in Antisense Research and Application, Ed. S. T. Crooke and B. Lebleu, CRC Press, 1993; in Englisch, et al., Angewandte Chemie, International Edition, 30: 613-722, 1991 (see, especially pages 622 and 623, and in the Concise Encyclopedia of Polymer Science and Engineering, J. I. Kroschwitz Ed., John Wiley & Sons, pages 858-859, 1990, Cook, Anti-Cancer DrugDesign 6: 585-607, 1991, each of which are hereby incorporated by reference in their entirety).

The term "nucleosidic base" or "nucleobase analogue" is further intended to include heterocyclic compounds that can serve as like nucleosidic bases including certain "universal bases" that are not nucleosidic bases in the most classical sense but serve as nucleosidic bases. Especially mentioned as a universal base is 3-nitropyrrole or a 5-nitroindole. Other preferred compounds include pyrene and pyridyloxazole derivatives, pyrenyl, pyrenylmethylglycerol derivatives and the like. Other preferred universal bases include, pyrrole, diazole or triazole derivatives, including those universal bases known in the art.

By "oligonucleotide," "oligomer," or "oligo" is meant a successive chain of monomers (e.g., glycosides of heterocyclic bases) connected via internucleoside linkages. The linkage between two successive monomers in the oligo consist of 2 to 4, desirably 3, groups/atoms selected from -CH₂-, -O-, -S-, -NR^{H}-, >C=O, >C=NR^{H}, >C=S, -Si(R")₂-, -SO-, -S(O)₂-, -P(O)₂-, -PO(BH₃)-, -P(O,S)-, -P(S)₂-, -PO(R")-, -PO(OCH₃)-, and -PO(NHR^{H})-, where R^{H} is selected from hydrogen and C₁₋₄-alkyl, and R" is selected from C₁₋₆-alkyl and phenyl. Illustrative examples of such linkages are -CH₂-CH₂-CH₂-, -CH₂-CO-CH₂-, -CH₂-CHOH-CH₂-, -O-CH₂-O-, -O-CH₂-CH₂-, -O-CH₂-CH= (including R⁵ when used as a linkage to a succeeding monomer), -CH₂-CH₂-O-, -NR^{H}-CH₂-CH₂-, -CH₂-CH₂-NR^{H}-, -CH₂-NR^{H}-CH₂-, -O-CH₂-CH₂-NR^{H}-, -NR^{H}-CO-O-, -NR^{H}-CO-NR^{H}-, -NR^{H}-CS-NR^{H}-, -NR^{H}-C(=NR^{H})-NR^{H}-, -NR^{H} -CO-CH₂-NR^{H}-, -O-CO-O-, -O-CO-CH₂-O-, -O-CH₂-CO-O-, -CH₂-CO-NR^{H}-, -O-CO-NR^{H}-, -NR^{H}-CO-CH₂-, -O-CH₂-CO-NR^{H}-, -O-CH₂CH₂NR^{H}-, -CH=N-O-, -CH₂-NR^{H}-O-, -CH₂-O-N= (including R⁵ when used as a linkage to a succeeding monomer), -CH₂-O-NR^{H}-, -CO-NR^{H}-CH₂-, -CH₂-NR^{H}-O- , -CH₂-NR^{H}-CO-, -O-NR^{H}-CH₂-, -O-NR^{H}-, -O-CH₂-S-, -S-CH₂-O-, -CH₂-CH₂-S-, -O-CH₂-CH₂-S-, -S-CH₂-CH= (including R⁵ when used as a linkage to a succeeding monomer), -S-CH₂-CH₂-, -S-CH₂-CH₂-O-, -S-CH₂-CH₂-S-, -CH₂-S-CH₂-, -CH₂-SO-CH₂-, -CH₂-SO₂-CH₂-, -O-SO-O-, -O-S(O)₂-O-, -O-S(O)₂-CH₂-, -O-S(O)₂-NR^{H}-, -NR^{H}-S(O)₂-CH₂-, -O-S(O)₂-CH₂-, -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -O-P(S)₂-S-, -S-P(O)₂-S-, -S-P(O,S)-S-, -S-P(S)₂-S-, -O-PO(R")-O-, -O-PO(OCH₃)-O-, -O-PO(OCH₂CH₃)-O-, -O-PO(OCH₂CH₂S-R)-O-, -O-PO(BH₃)-O-, -O-PO(NHR^{N})-O-, -O-P(O)₂-NR^{H}-, -NR^{H}-P(O)₂-O-, O-P(O,NR^{H})-O-, -CH₂-P(O)₂-O-, -O-P(O)₂-CH₂-, and -O-Si(R")₂-O-; among which -CH₂-CO-NR^{H}-, -CH₂-NR^{H}-O-, -S-CH₂-O-, -O-P(O)₂-O-, -O-P(O,S)-O-, -O-P(S)₂-O-, -NR"-P(O)₂-O-, -O-P(O,NR^{H})-O-, -O-PO(R")-O-, -O-PO(CH₃)-O-, and -O-PO(NHR^{N})-O-, where R^{H} is selected form hydrogen and C₁₋₄-alkyl, and R" is selected from C₁₋₆-alkyl and phenyl, are especially desirable. Further illustrative examples are given in Mesmaeker et. al., Current Opinion in Structural Biology 1995, 5, 343-355 and Susan M. Freier and Karl-Heinz Altmann, Nucleic Acids Research, 1997, vol 25, pp 4429-4443. The left-hand side of the internucleoside linkage is bound to the 5-membered ring as substituent P* at the 3'-position, whereas the right-hand side is bound to the 5'-position of a preceding monomer.

By "LNA" or "LNA monomer" (e.g., an LNA nucleoside or LNA nucleotide) or an LNA oligomer (e.g., an oligonucleotide or nucleic acid) is meant a nucleoside or nucleotide analogue that includes at least one LNA monomer. LNA monomers as disclosed in PCT Publication WO 99/14226 are in general particularly desirable modified nucleic acids for incorporation into an oligonucleotide of the invention. Additionally, the nucleic acids may be modified at either the 3' and/or 5' end by any type of modification known in the art. For example, either or both ends may be capped with a protecting group, attached to a flexible linking group, attached to a reactive group to aid in attachment to the substrate surface, etc. Desirable LNA monomers and their method of synthesis also are disclosed in US 6,043,060, US 6,268,490, PCT Publications WO 01/07455, WO 01/00641, WO 98/39352, WO 00/56746, WO 00/56748 and WO 00/66604 as well as in the following papers: Morita et al., Bioorg. Med. Chem. Lett. 12(1):73-76, 2002; Hakansson et al., Bioorg. Med. Chem. Lett. 11(7):935-938, 2001; Koshkin et al., J. Org. Chem. 66(25):8504-8512, 2001; Kvaerno et al., J. Org. Chem. 66(16):5498-5503, 2001; Hakansson et al., J. Org. Chem. 65(17):5161-5166, 2000; Kvaerno et al., J. Org. Chem. 65(17):5167-5176, 2000; Pfundheller et al., Nucleosides Nucleotides 18(9):2017-2030, 1999; and Kumar et al., Bioorg. Med. Chem. Lett. 8(16):2219-2222, 1998.

Preferred LNA monomers, also referred to as "oxy-LNA" are LNA monomers which include bicyclic compounds as disclosed in PCT Publication WO 03/020739 wherein the bridge between R^{4'} and R^{2'} as shown in formula (I) below together designate -CH₂-O- or -CH₂-CH₂-O-.

By "LNA modified oligonucleotide" or "LNA substituted oligonucleotide" is meant a oligonucleotide comprising at least one LNA monomer of formula (I), described *infra,* having the below described illustrative examples of modifications: 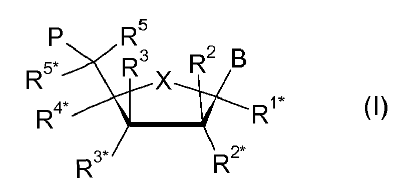 wherein X is selected from -O-, -S-, -N(R^{N})-, -CCR⁶R^{6*})-, -O-C(R⁷R^{7*})-, -C(R⁶R^{6*})-O-, -S-CCR⁷R^{7*})-, -CCR⁶R^{6*})-S-, -N(R^{N*})-CCR⁷R^{7*})-, -C(R⁶R^{6*}) -N(R^{N*}) -, and -C(R⁶R^{6*})-C(R⁷R^{7*}).

B is selected from a modified base as discussed above e.g. an optionally substituted carbocyclic aryl such as optionally substituted pyrene or optionally substituted pyrenylmethylglycerol, or an optionally substituted heteroalicylic or optionally substituted heteroaromatic such as optionally substituted pyridyloxazole, optionally substituted pyrrole, optionally substituted diazole or optionally substituted triazole moieties; hydrogen, hydroxy, optionally substituted C₁₋₄-alkoxy, optionally substituted C₁₋₄-alkyl, optionally substituted C₁₋₄-acyloxy, nucleobases, DNA intercalators, photochemically active groups, thermochemically active groups, chelating groups, reporter groups, and ligands.

P designates the radical position for an internucleoside linkage to a succeeding monomer, or a 5'-terminal group, such internucleoside linkage or 5'-terminal group optionally including the substituent R⁵. One of the substituents R², R^{2*}, R³, and R^{3*} is a group P* which designates an internucleoside linkage to a preceding monomer, or a 2'/3'-terminal group. The substituents of R^{1*} R^{4*}, R⁵ R^{5*} R⁶ R^{6*} , R⁷, R^{7*}, R^{N} and the ones of R², R^{2*}', R³ and R^{3*} not designating P^{*} each designates a biradical comprising about 1-8 groups/atoms selected from -C(R^{a}R^{b})-, -C(R^{a})=C(R^{a})-, -C(R^{a})=N-, -C(R^{a})-O-, -O -Si(R^{c})₂-, -C(R^{a})-S, -S-, -SO₂-, -C(R^{a}) -N(R^{b})-, -N(R^{a})-, and >C=Q, wherein Q is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, hetero-aryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents R^{a} and R^{b} together may designate optionally substituted methylene (=CH₂), and wherein two non-geminal or geminal substituents selected from R^{a}, R^{b}, and any of the substituents R^{1*}, R², R^{2*} R³ R^{3*}, R^{4*}, R⁵, R^{5*} R⁶ and R^{6*} R⁷, and R^{7*} which are present and not involved in P, P^{*} or the biradical(s) together may form an associated biradical selected from biradicals of the same kind as defined before; the pair(s) of non-geminal substituents thereby forming a mono- or bicyclic entity together with (i) the atoms to which said non-geminal substituents are bound and (ii) any intervening atoms.

Each of the substituents R^{1*}, R², R^{2*} R³ R^{4*}, R⁵ R^{5*} R⁶ and R^{6*}, R⁷, and R^{7*} which are present and not involved in P, P^{*} or the biradical(s), is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di-(C₁₋₆-alkyl)amino, carbamoyl, mono- and di(C₁₋₆-alkyl)-amino-carbonyl, amino-C₁₋₆-alkylaminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof.

Exemplary 5', 3', and/or 2' terminal groups include -H, -OH, halo (e.g., chloro, fluoro, iodo, or bromo), optionally substituted aryl, (e.g., phenyl or benzyl), alkyl (e.g., methyl or ethyl), alkoxy (e.g., methoxy), acyl (e.g. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamino, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, amidino, amino, carbamoyl, sulfamoyl, alkene, alkyne, protecting groups (e.g., silyl, 4,4'-dimethoxytrityl, monomethoxytrityl, or trityl(triphenylmethyl)), linkers (e.g., a linker containing an amine, ethylene glycol, quinone such as anthraquinone), detectable labels (e.g., radiolabels or fluorescent labels), and biotin.

It is understood that references herein to a nucleic acid unit, nucleic acid residue, LNA monomer, or similar term are inclusive of both individual nucleoside units and nucleotide units and nucleoside units and nucleotide units within an oligonucleotide.

A "modified base" or other similar terms refer to a composition (e.g., a non-naturally occurring nucleobase or nucleosidic base), which can pair with a natural base (e.g., adenine, guanine, cytosine, uracil, and/or thymine) and/or can pair with a non-naturally occurring nucleobase or nucleosidic base. Desirably, the modified base provides a Tₘ differential of 15, 12, 10, 8, 6, 4, or 2°C or less as described herein. Exemplary modified bases are described in EP 1 072 679 and WO 97/12896.

The term "chemical moiety" refers to a part of a molecule. "Modified by a chemical moiety" thus refer to a modification of the standard molecular structure by inclusion of an unusual chemical structure. The attachment of said structure can be covalent or non-covalent.

The term "inclusion of a chemical moiety" in an oligonucleotide probe thus refers to attachment of a molecular structure. Such as chemical moiety include but are not limited to covalently and/or non-covalently bound minor groove binders (MGB) and/or intercalating nucleic acids (INA) selected from a group consisting of asymmetric cyanine dyes, DAPI, SYBR Green I, SYBR Green II, SYBR Gold, PicoGreen, thiazole orange, Hoechst 33342, Ethidium Bromide, 1-O-(1-pyrenylmethyl)glycerol and Hoechst 33258. Other chemical moieties include the modified nucleobases, nucleosidic bases or LNA modified oligonucleotides. "Oligonucleotide analogue" refers to a nucleic acid binding molecule capable of recognizing a particular target nucleotide sequence. A particular oligonucleotide analogue is peptide nucleic acid (PNA) in which the sugar phosphate backbone of an oligonucleotide is replaced by a protein like backbone. In PNA, nucleobases are attached to the uncharged polyamide backbone yielding a chimeric pseudopeptide-nucleic acid structure, which is homomorphous to nucleic acid forms.

"High affinity nucleotide analogue" or "affinity-enhancing nucleotide analogue" refers to a non-naturally occurring nucleotide analogue that increases the "binding affinity" of an oligonucleotide probe to its complementary recognition sequence when substituted with at least one such high-affinity nucleotide analogue.

As used herein, a probe with an increased "binding affinity" for a recognition sequence compared to a probe which comprises the same sequence but does not comprise a stabilizing nucleotide, refers to a probe for which the association constant (Kₐ) of the probe recognition segment is higher than the association constant of the complementary strands of a double-stranded molecule. In another preferred embodiment, the association constant of the probe recognition segment is higher than the dissociation constant (K_{d}) of the complementary strand of the recognition sequence in the target sequence in a double stranded molecule.

Monomers are referred to as being "complementary" if they contain nucleobases that can form hydrogen bonds according to Watson-Crick base-pairing rules (e.g. G with C, A with T or A with U) or other hydrogen bonding motifs such as for example diaminopurine with T, 5-methyl C with G, 2-thiothymidine with A, inosine with C, pseudoisocytosine with G, etc.

The term "succeeding monomer" relates to the neighbouring monomer in the 5'-terminal direction and the "preceding monomer" relates to the neighbouring monomer in the 3'-terminal direction.

The term "target nucleic acid" or "target ribonucleic acid" refers to any relevant nucleic acid of a single specific sequence, e. g., a biological nucleic acid, e. g., derived from a patient, an animal (a human or non-human animal), a plant, a bacteria, a fungi, an archae, a cell, a tissue, an organism, etc. For example, where the target ribonucleic acid or nucleic acid is derived from a bacteria, archae, plant, non-human animal, cell, fungi, or non-human organism, the method optionally further comprises selecting the bacteria, archae, plant, non-human animal, cell, fungi, or non-human organism based upon detection of the target nucleic acid. In one embodiment, the target nucleic acid is derived from a patient, e.g., a human patient. In this embodiment, the invention optionally further includes selecting a treatment, diagnosing a disease, or diagnosing a genetic predisposition to a disease, based upon detection of the target nucleic acid.

"Target sequence" refers to a specific nucleic acid sequence within any target nucleic acid.

The term "stringent conditions", as used herein, is the "stringency" which occurs within a range from about Tₘ-5° C. (5° C. below the melting temperature (Tₘ) of the probe) to about 20° C. to 25° C. below Tₘ. As will be understood by those skilled in the art, the stringency of hybridization may be altered in order to identify or detect identical or related polynucleotide sequences. Hybridization techniques are generally described in Nucleic Acid Hybridization, A Practical Approach, Ed. Hames, B. D. and Higgins, S. J., IRL Press, 1985; Gall and Pardue, Proc. Natl. Acad. Sci., USA 63: 378-383, 1969; and John, et al. Nature 223: 582-587, 1969.

### Detailed Description of the Invention

### Collection of probes of the invention

As briefly stated above, the probe collections or libraries of the present invention are so designed each member of said collection comprises a recognition sequence consisting of nucleobases and affinity enhancing nucleobase analogues, and wherein the recognition sequences exhibit a combination of high melting temperatures and low self-complementarity scores, said melting temperatures being the melting temperature of the duplex between the recognition sequence and its complementary DNA or RNA sequence.

This design provides for probes which are highly specific for their target sequences but which at the same time exhibits a very low risk of self-annealing (as evidenced by a low self-complementarity score) - self-annealing is, due to the presence of affinity enhancing nucleobases (such as LNA monomers) a problem which is more serious than when using conventional deoxyribonucleotide probes.

In one embodiment the recognition sequences exhibit a melting temperature (or a measure of melting temperature) corresponding to at least 5°C higher than a melting temperature or a measure of melting temperature of the self-complementarity score under condtions where the probe hybridizes specifically to its complementary target sequence (alternatively, one can quantify the "risk of self-annealing" feature by requiring that the melting temperature of the probe-target duplex must be at least 5°C higher than the melting temperature of duplexes between the probes or the probes internally). The collection may be so constituted that at least 90% (such as at least 95%) of the recognition sequences exhibit a melting temperature or a measure of melting temperature corresponding to at least 5°C higher than a melting temperature or a measure of melting temperature of the self-complementarity score under condtions where the probe hybridizes specifically to its complementary target sequence (or that at least the same percentages of probes exhibit a melting temperature of the probe-target duplex of at least 5°C more than the melting temperature of duplexes between the probes or the probes internally). In a preferred embodiment all of the detection probes include recognition sequences which exhibit a melting temperature or a measure of melting temperature corresponding to at least 5°C higher than a melting temperature or a measure of melting temperature of the self-complementarity score under condtions where the probe hybridizes specifically to its complementary target sequence.

However, it is preferred that this temperature difference is higher, such as at least least 10°C, such as at least 15, at least 20, at least 25, at least 30, at least 35, at least 40, at least 45, and at least 50°C higher than a melting temperature or measure of melting temperature of the self-complementarity score.

In one embodiment a collection of probes according to the present invention comprises at least 10 detection probes, 15 detection probes, such as at least 20, at least 25, at least 50, at least 75, at least 100, at least 200, at least 500, at least 1000, and at least 2000 members.

It if preferred that the collection of probes of the invention is capable of specifically detecting all or substantially all members of the transcriptome of an organism.

In another preferred embodiment, the collection of probes is capable of specifically detecting all small non-coding RNAs of an organism, such as all miRNAs or siRNAs.

The organism is selected from the group consisting of a bacterium, a yeast, a fungus, a protozoan, a plant, and an animal. Specific examples of genuses and species of such organisms are mentioned herein, and the inventive collection of probes may by designed for all of these specific genuses and species.

In one embodiment, the affinity-enhancing nucleobase analogues are regularly spaced between the nucleobases in at least 80% of the members of said collection, such as in at least 90% or at least 95% of said collection (in one embodiment, all members of the collection contains regularly spaced affinity-enhancing nucleobase analogues). One reason for this is that the time needed for adding each nucleobase or analogue during synthesis of the probes of the invention is dependent on whether or not a nucleobase analogue is added. By using the "regular spacing strategy" considerable production benefits are achieved. Specifically for LNA nucleobases, the required coupling times for incorporating LNA amidites during synthesis may exceed that required for incorporating DNA amidites. Hence, in cases involving simultaneous parallel synthesis of multiple oligonucleotides on the same instrument, it is advantageous if the nucleotide analogues such as LNA are spaced evenly in the same pattern as derived from the 3'-end, to allow reduced cumulative coupling times for the sytnthesis. The affinity enhancing nucleobase analogues are conveniently regularly spaced as every 2^{nd}, every 3^{rd}, every 4^{th} or every 5^{th} nucleobase in the recognition sequence, and preferably as every 3^{rd} nucleobase.

In one embodiment of the the collection of probes, all members contain affinity enhancing nucleobase analogues with the same regular spacing in the recognition sequences.

The presence of the affinity enhancing nucleobases in the recognition sequence preferably confers an increase in the binding affinity between a probe and its complementary target nucleotide sequence relative to the binding affinity exhibited by a corresponding probe, which only include nucleobases. Since LNA nucleobases/monomers have this ability, it is preferred that the affinity enhancing nucleobase analogues are LNA nucleobases.

In some embodiments, the 3' and 5' nucleobases are not substituted by affinity enhancing nucleobase analogues.

As detailed herein, one huge advantage of the probes of the invention is their short lengths which surprisingly provides for high target specificity and advantages in detecting small RNAs and detecting nucleic acids in samples not normally suitable for hybridization detection strategies. It is, however, preferred that the probes comprise a recognition sequence is at least a 6-mer, such as at least a 7-mer, at least an 8-mer, at least a 9-mer, at least a 10-mer, at least an 11-mer, at least a 12-mer, at least a 13-mer, at least a 14-mer, at least a 15-mer, at least a 16-mer, at least a 17-mer, at least an 18-mer, at least a 19-mer, at least a 20-mer, at least a 21-mer, at least a 22-mer, at least a 23-mer, and at least a 24-mer. On the other hand, the recognition sequence is preferably at most a 25-mer, such as at most a 24-mer, at most a 23-mer, at most a 22-mer, at most a 21-mer, at most a 20-mer, at most a 19-mer, at most an 18-mer, at most a 17-mer, at most a 16-mer, at most a 15-mer, at most a 14-mer, at most a 13-mer, at most a 12-mer, at most an 11-mer, at most a 10-mer, at most a 9-mer, at most an 8-mer, at most a 7-mer, and at most a 6-mer.

Also for production purposes, it is an advantage that a majority of the probes in a collection are of the same length. In preferred embodiments, the collection of probes of the invention is one wherein at least 80% of the members comprise recognition sequences of the same length, such as at least 90% or at least 95%.

As discussed above, it is advantageous, in order ot avoid self-annealing, that at least one of the nucleobases in the recognition sequence is substituted with its corresponding selectively binding complementary (SBC) nucleobase.

Typically, the nucleobases in the sequence are selected from ribonucleotides and deoxyribonucleotides, preferably deoxyribonucleotides. It is preferred that the recognition sequence consists of affinity enhancing nucleobase analogues together with either ribonucleotides or deoxyribonucleotides.

In certain embodiments, each member of a collection is covalently bonded to a solid support. Such a solid support may be selected from a bead, a microarray, a chip, a strip, a chromatographic matrix, a microtiter plate, a fiber or any other convenient solid support generally accepted in the art in order to facilitate the exercise of the methods discussed generally and specficially

As also detailed herein, each detection probe in a collection of the invention may include a detection moiety and/or a ligand, optionally placed in the recognition sequence but also placed outside the recognition sequence. The detection probe may thus include a photochemically active group, a thermochemically active group, a chelating group, a reporter group, or a ligand that facilitates the direct of indirect detection of the probe or the immobilisation of the oligonucleotide probe onto a solid support.

### Probes of the invention

The present invention provides novel oligonucleotide compositions and probe sequences for the use in detection, isolation, purification, amplification, identification, quantification, or capture of miRNAs, their target mRNAs, stem-loop precursor miRNAs, siRNAs, other non-coding RNAs, RNA-edited transcripts or alternative mRNA splice variants or single stranded DNA (e.g. viral DNA) characterized in that the probe sequences contain a number of nucleoside analogues.

In a preferred embodiment the number of nucleoside analogue corresponds to from 20 to 40% of the oligonucleotide of the invention.

In a preferred embodiment the probe sequences are substituted with a nucleoside analogue with regular spacing between the substitutions

In another preferred embodiment the probe sequences are substituted with a nucleoside analogue with irregular spacing between the substitutions

In a preferred embodiment the nucleoside analogue is LNA.

In a further preferred embodiment the detection probe sequences comprise a photochemically active group, a thermochemically active group, a chelating group, a reporter group, or a ligand that facilitates the direct of indirect detection of the probe or the immobilisation of the oligonucleotide probe onto a solid support.

In a further preferred embodiment:
(a) the photochemically active group, the thermochemically active group, the chelating group, the reporter group, or the ligand includes a spacer (K), said spacer comprising a chemically cleavable group; or
(b) the photochemically active group, the thermochemically active group, the chelating group, the reporter group, or the ligand is attached via the biradical of at least one of the LNA(s) of the oligonucleotide.

Especially preferred detection probes of the invention are those that include the LNA containing recognition sequences set forth in tables A-K, 1, 3 and 15-I herein.

### Methods for defining and preparing probes an probe collections

The invention relates to a method for expanding or building a collection defined above, comprising
A) defining a reference nucleotide sequence consisting of nucleobases, said reference nucleotide sequence being complementary to a target sequence for which the collection does not contain a detection probe,
B) substituting the reference nucleotide sequence's nucleobases with affinity enhancing nucleobase analogues to provide a set of chimeric sequences wherein,
C) determining usefulness of each of the chimeric sequences based on assessment of their ability to self-anneal and their melting temperature, and
D) synthesizing and adding, to the collection, a probe comprising as its recognition sequence the chimeric sequence with the optimum combination of high melting temperature and low self-annealing.

In order to ensure that the optimum probes are added to the library, step B preferably includes provision of all possible chimeric sequences which include a particular set of affinity enhancing nucleobase analogues. By this is meant that prior to exercise of the method, it is decided which affinity enhancing nucleobases should be used in the design phase (typically one for each of the 4 naturally occurring nucleobases). After this choice has been made, step B runs through an iterative process in order to define all possible chimeric sequences. In order to reduce the comprehensive nature of this step, it can also be decided to utilize the "regular spacing" strategy referred to above, since this will inherently reduce the number of chimeric sequences to evaluate in step C. So, basically this means that only chimeric sequences, wherein the affinity enhancing nucleobase analogues are regularly spaced between the nucleobases, are added to the collection in step D.

Step C comprises the herein-discussed evaluation of melting temperature diffences of at least 5°C between melting temperature for the duplex between the potential probe and its target and the melting temperature characterizing self-annealing. Hence, all disclosures relating to these preferred differences in melting temperature referred to above in the discussion of the probe collections apply mutatis mutandis to the determination in step C.

Preferably, the melting temperature difference used for the determination in step C is at least 15°C.

Apart from that, all disclosures relating to the characteristics of the probes in the collections of the invention apply mutatis mutandis to the above referenced method, meaning that the probes designed/produced may further include all the features characterizing the probes of the present invention.

A similar method may be utilized to design single probes, comprising
1) defining a reference nucleotide sequence consisting of nucleobases, said reference nucleotide sequence being complementary to said target nucleotide sequence,
2) substituting the reference nucleotide sequence's nucleobases with affinity enhancing nucleobase analogues to provide a set of chimeric sequences
3) determining usefulness of each of the chimeric sequences based on assessment of their ability to self-anneal and their melting temperatures, and
4) defining the optimized detection probe as the one in the set having as its recognition sequence the chimeric sequence with the optimum combination of high melting temperature and low self-annealing.

As above, step 2 may include provision of all possible chimeric sequences which include a particular set of affinity enhancing nucleobase analogues and as above only chimeric sequences, wherein the affinity enhancing nucleobase analogues are regularly spaced between the nucleobases, are defined in step 4 or, if applicable, are synthesized - this is because the method may also entail synthesizing the optimized detection probe. And, in general, all disclosures herein relating to the characteristics of the probes in the collections of the invention apply mutatis mutandis to the above referenced method for design of single probes, meaning that the probes designed/produced may further include all the features characterizing the probes of the present invention. This e.g. includes that the detection probe may be further modified by containing at least one SBC nucleobase as one of the nucleobases, and in general, the detection probe designed may be any detection probe disclosed herein.

Both of the above-referenced methods may be performed partly in silico, i.e. all steps relating to the design phase. Since sequence alignments and melting temperature calculations may be accomplished by the use of software, the present methods are preferably exercised at least partially in a software environment. That is, above-referenced steps A-C or 1-4, may be performed in silico and the invention also relates to a computer system comprising a computer program product/executable code which can perform such a method.

Hence, the present invention also relates to a computer system for designing an optimized detection probe for a target nucleic acid sequence, said system comprising
a) input means for inputting the target nucleotide (can be a manual input interface such as a keyboard but conveniently simple queries in a database or input from a source file)
b) storage means for storing the target nucleotide sequence (RAM, a harddisk or any other suitable volatile memory),
c) optionally executable code which can calculate a reference nucleotide sequence being complementary to said target nucleotide sequence and/or input means for inputting the reference nucleotide sequence,
d) optionally storage means for storing the reference nucleotide sequence (features c and d are optional because these, although convenient, are not necessary in order to create a chimeric sequence, cf. next step),
e) executable code which can generate chimeric sequences from the reference nucleotide sequence or the target nucleic acid sequence, wherein said chimeric sequences comprise the reference nucleotide sequence, wherein has been in-substituted affinity enhancing nucleobase analogues (typically, this code will generate a complete list of possible chimeric sequences which are then examined for usefulness and at the same time removed from the list in order to avoid double testing of the same chimeric sequence),
f) executable code which can determine the usefulness of such chimeric sequences based on assessment of their ability to self-anneal and their melting temperatures and either rank such chimeric sequences according to their usefulness (this code is executed after execution of the code in step e, and basically functions as a iteration which tests each and every chimeric sequence genereated by feature e),
g) storage means for storing at least one chimeric sequence (depending on the desired output, this storage means may hold a ranked list of chimeric sequences or one single chimeric sequence, namely the one which has the highest degree of usefulness after each execution of one iteration in step f), and
h) output means for presenting the sequence of at least one optimized detection probe (will typically be a disk drive, a monitor or a printer).

Typcially the target nucleic acid sequences stored in step b will be sequences of non-coding small RNAs as discussed herein.

Also a storage means embedding executable code (e.g. a computer program) which executes the design steps of the method referred to above is part of the present invention.

### Methods/uses of probes and probe collections

Preferred methods/uses include:Specific isolation, purification, amplification, detection, identification, quantification, inhibition or capture of a target nucleotide sequence in a sample, by contacting said sample with a member of a collection of probes or a probe defined herein under conditions that facilitate hybridization between said member/probe and said target nucleotide sequence. Since the probes are typically shorter than the complete molecule wherein they form part, the inventive methods/uses include isolation, purification, amplification, detection, identification, quantification, inhibition or capture of a molecule comprising the target nucleotide sequence.

Typically, the molecule which is isolated, purified, amplified, detected, identified, quantified, inhibited or captured is a small, non-coding RNA, e.g. a miRNA such as a mature miRNA. A very surprising finding of the present invention is that it is possible to effect specific hybridization with miRNAs using probes of very short lengths, such as those lengths discussed herein when discussing the collection of probes. Typically the small, non-coding RNA has a length of at most 30 residues, such as at most 29, 28, 27, 26, 25, 24, 23, 22, 21, 20, 19, or 18 residues. The small non-coding RNA typically also has a length of at least 15 residues, such as at least 16, 17, 18, 19, 20, 21, 22, 23, 24, 25, 26, 27, 28, 29 or 30 residues.

As detailed in the examples herein, the specific hybridization between the short probes of the present invention to miRNA and the fact that miRNA can be mapped to various tissue origins, allows for an embodiment of the uses/methods of the present invention comprising identification of the primary site of metastatic tumors of unknown origin.

As also discussed in the examples herein, the short, but highly specific probes of the present invention allows hybridization assays to be performed on fixated embedded tissue sections, such as formalin fixated paraffine embedded sections. Hence, an embodiment of the uses/methods of the present invention are those where the molecule, which is isolated, purified, amplified, detected, identified, quantified, inhibited or captured, is DNA (single stranded such as viral DNA) or RNA present in a fixated, embedded sample such as a formalin fixated paraffine embedded sample.

Other uses include:
(a) capture and detection of naturally occurring or synthetic single stranded nucleic acids such as miRNAs, their target mRNAs, stem-loop precursor miRNAs, siRNAs, other non-coding RNAs, RNA-edited transcripts or alternative mRNA splice variants or viral DNA; or
(b) purification of naturally occurring single stranded nucleic acids such as miRNAs, their target mRNAs, stem-loop precursor miRNAs, siRNAs, other non-coding RNAs, RNA-edited transcripts or alternative mRNA splice variants or viral DNA; or
(c) detection and assessment of expression patterns for naturally occurring single stranded nucleic acids such as miRNAs, their target mRNAs, stem-loop precursor miRNAs, siRNAs, other non-coding RNAs, RNA-edited transcripts or alternative mRNA splice variants by RNA in-situ hybridisation, dot blot hybridisation, reverse dot blot hybridisation, or in Northern blot analysis or expression profiling by microarrays
(d) functional analysis of naturally occurring single stranded nucleic acids such as miRNAs, their target mRNAs, stem-loop precursor miRNAs, siRNAs, other non-coding RNAs, RNA-edited transcripts or alternative mRNA splice variants or viral DNA in vitro and in vivo in plants or animals, such as human, mouse, rat, zebrafish, *Caenorhabditis elegans, Drosophila melanogaster, Arabidopsis thaliana,* rice and maize, by inhibiting their mode of action, e.g. the binding of mature miRNAs to their cognate target mRNAs.
(e) antisense-based intervention, targeted against tumorigenic single stranded nucleic acids such as miRNAs, their target mRNAs, stem-loop precursor miRNAs, siRNAs, other non-coding RNAs, RNA-edited transcripts or alternative mRNA splice variants or viral DNA in vivo in plants or animals, such as human, mouse, rat, zebrafish, *Caenorhabditis elegans, Drosophila melanogaster, Arabidopsis thaliana,* rice and maize, by inhibiting their mode of action, e.g. the binding of mature miRNAs to their cognate target mRNAs.

Further embodiments includes the use of an LNA modified oligonucleotide probe as an aptamer in molecular diagnostics or (b) as an aptamer in RNA mediated catalytic processes or (c) as an aptamer in specific binding of antibiotics, drugs, amino acids, peptides, structural proteins, protein receptors, protein enzymes, saccharides, polysaccharides, biological cofactors, nucleic acids, or triphosphates or (d) as an aptamer in the separation of enantiomers from racemic mixtures by stereospecific binding or (e) for labelling cells or (f) to hybridise to non-protein coding cellular RNAs, such as tRNA, rRNA, snRNA and scRNA, in vivo or in-vitro or (g) to hybridise to non-protein coding cellular RNAs, such as tRNA, rRNA, snRNA and scRNA, in vivo or in-vitro or (h) in the construction of Taqman probes or Molecular Beacons.

The present invention also provides a kit for the isolation, purification, amplification, detection, identification, quantification, or capture of natural or synthetic nucleic acids, where the kit comprises a reaction body and one or more LNAs as defined herein. The LNAs are preferably immobilised onto said reactions body (e.g. by using the immobilising techniques described above).

For the kits according to the invention, the reaction body is preferably a solid support material, e.g. selected from borosilicate glass, soda-lime glass, polystyrene, polycarbonate, polypropylene, polyethylene, polyethyleneglycol terephthalate, polyvinylacetate, polyvinylpyrrolidinone, polymethylmethacrylate and polyvinylchloride, preferably polystyrene and polycarbonate. The reaction body may be in the form of a specimen tube, a vial, a slide, a sheet, a film, a bead, a pellet, a disc, a plate, a ring, a rod, a net, a filter, a tray, a microtitre plate, a stick, or a multi-bladed stick.

A written instruction sheet stating the optimal conditions for use of the kit typically accompanies the kits.

### Further aspects of the invention

Once the appropriate target RNA sequences have been selected, LNA substituted detection probes are preferably chemically synthesized using commercially available methods and equipment as described in the art (Tetrahedron 54: 3607-30, 1998). For example, the solid phase phosphoramidite method can be used to produce short LNA probes (Caruthers, et al., Cold Spring Harbor Symp. Quant. Biol. 47:411-418, 1982, Adams, et al., J. Am. Chem. Soc. 105: 661 (1983).

LNA-containing-probes can be labelled during synthesis. The flexibility of the phosphoramidite synthesis approach furthermore facilitates the easy production of LNAs carrying all commercially available linkers, fluorophores and labelling-molecules available for this standard chemistry. LNA-modified probes may also be labelled by enzymatic reactions e.g. by kinasing using T4 polynucleotide kinase and gamma-³²P-ATP or by using terminal deoxynucleotidyl transferase (TDT) and any given digoxygenin-conjugated nucleotide triphosphate (dNTP) or dideoxynucleotide triphosphate (ddNTP).

Detection probes according to the invention can comprise single labels or a plurality of labels. In one aspect, the plurality of labels comprise a pair of labels which interact with each other either to produce a signal or to produce a change in a signal when hybridization of the detection probe to a target sequence occurs.

In another aspect, the detection probe comprises a fluorophore moiety and a quencher moiety, positioned in such a way that the hybridized state of the probe can be distinguished from the unhybridized state of the probe by an increase in the fluorescent signal from the nucleotide. In one aspect, the detection probe comprises, in addition to the recognition element, first and second complementary sequences, which specifically hybridize to each other, when the probe is not hybridized to a recognition sequence in a target molecule, bringing the quencher molecule in sufficient proximity to said reporter molecule to quench fluorescence of the reporter molecule. Hybridization of the target molecule distances the quencher from the reporter molecule and results in a signal, which is proportional to the amount of hybridization.

In the present context, the term "label" means a reporter group, which is detectable either by itself or as a part of a detection series. Examples of functional parts of reporter groups are biotin, digoxigenin, fluorescent groups (groups which are able to absorb electromagnetic radiation, e.g. light or X-rays, of a certain wavelength, and which subsequently reemits the energy absorbed as radiation of longer wavelength; illustrative examples are DANSYL (5-dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erythrosine, coumaric acid, umbelliferone, Texas red, rhodamine, tetramethyl rhodamine, Rox, 7-nitrobenzo-2-oxa-1-diazole (NBD), pyrene, fluorescein, Europium, Ruthenium, Samarium, and other rare earth metals), radio isotopic labels, chemiluminescence labels (labels that are detectable via the emission of light during a chemical reaction), spin labels (a free radical (e.g. substituted organic nitroxides) or other paramagnetic probes (e.g. Cu²⁺, Mg²⁺) bound to a biological molecule being detectable by the use of electron spin resonance spectroscopy). Especially interesting examples are biotin, fluorescein, Texas Red, rhodamine, dinitrophenyl, digoxigenin, Ruthenium, Europium, Cy5, Cy3, etc.

Suitable samples of target nucleic acid molecules may comprise a wide range of eukaryotic and prokaryotic cells, including protoplasts; or other biological materials, which may harbour target nucleic acids. The methods are thus applicable to tissue culture animal cells, animal cells (e.g., blood, serum, plasma, reticulocytes, lymphocytes, urine, bone marrow tissue, cerebrospinal fluid or any product prepared from blood or lymph) or any type of tissue biopsy (e.g. a muscle biopsy, a liver biopsy, a kidney biopsy, a bladder biopsy, a bone biopsy, a cartilage biopsy, a skin biopsy, a pancreas biopsy, a biopsy of the intestinal tract, a thymus biopsy, a mammae biopsy, a uterus biopsy, a testicular biopsy, an eye biopsy or a brain biopsy, e.g., homogenized in lysis buffer), archival tissue nucleic acids, plant cells or other cells sensitive to osmotic shock and cells of bacteria, yeasts, viruses, mycoplasmas, protozoa, rickettsia, fungi and other small microbial cells and the like.

Preferably, the detection probes of the invention are modified in order to increase the binding affinity of the probes for the target sequence by at least two-fold compared to probes of the same sequence without the modification, under the same conditions for hybridization or stringent hybridization conditions. The preferred modifications include, but are not limited to, inclusion of nucleobases, nucleosidic bases or nucleotides that have been modified by a chemical moiety or replaced by an analogue to increase the binding affinity. The preferred modifications may also include attachment of duplex-stabilizing agents e.g., such as minor-groove-binders (MGB) or intercalating nucleic acids (INA). Additionally, the preferred modifications may also include addition of non-discriminatory bases e.g., such as 5-nitroindole, which are capable of stabilizing duplex formation regardless of the nucleobase at the opposing position on the target strand. Finally, multi-probes composed of a non-sugar-phosphate backbone, e.g. such as PNA, that are capable of binding sequence specifically to a target sequence are also considered as a modification. All the different binding affinity-increasing modifications mentioned above will in the following be referred to as "the stabilizing modification(s)", and the tagging probes and the detection probes will in the following also be referred to as "modified oligonucleotide". More preferably the binding affinity of the modified oligonucleotide is at least about 3-fold, 4-fold, 5-fold, or 20-fold higher than the binding of a probe of the same sequence but without the stabilizing modification(s).

Most preferably, the stabilizing modification(s) is inclusion of one or more LNA nucleotide analogs. Probes from 6 to 30 nucleotides according to the invention may comprise from 1 to 8 stabilizing nucleotides, such as LNA nucleotides. When at least two LNA nucleotides are included, these may be consecutive or separated by one or more non-LNA nucleotides. In one aspect, LNA nucleotides are alpha-L-LNA and/or xylo LNA nucleotides as disclosed in PCT Publications No. WO 2000/66604 and WO 2000/56748.

The problems with existing detection, quantification and knock-down of miRNAs and siRNAs as outlined above are addressed by the use of the novel oligonucleotide probes of the invention in combination with any of the methods of the invention selected so as to recognize or detect a majority of all discovered and detected miRNAs, in a given cell type from a given organism. In one aspect, the probe sequences comprise probes that detect mammalian mature miRNAs, e.g., such as mouse, rat, rabbit, monkey, or human miRNAs. By providing a sensitive and specific method for detection of mature miRNAs, the present invention overcomes the limitations discussed above especially for conventional miRNA assays and siRNA assays. The detection element of the detection probes according to the invention may be single or double labelled (e.g. by comprising a label at each end of the probe, or an internal position). In one aspect, the detection probe comprises two labels capable of interacting with each other to produce a signal or to modify a signal, such that a signal or a change in a signal may be detected when the probe hybridizes to a target sequence. A particular aspect is when the two labels comprise a quencher and a reporter molecule.

In another aspect, the probe comprises a target-specific recognition segment capable of specifically hybridizing to a target molecule comprising the complementary recognition sequence. A particular detection aspect of the invention referred to as a "molecular beacon with a stem region" is when the recognition segment is flanked by first and second complementary hairpin-forming sequences which may anneal to form a hairpin. A reporter label is attached to the end of one complementary sequence and a quenching moiety is attached to the end of the other complementary sequence. The stem formed when the first and second complementary sequences are hybridized (i.e., when the probe recognition segment is not hybridized to its target) keeps these two labels in close proximity to each other, causing a signal produced by the reporter to be quenched by fluorescence resonance energy transfer (FRET). The proximity of the two labels is reduced when the probe is hybridized to a target sequence and the change in proximity produces a change in the interaction between the labels. Hybridization of the probe thus results in a signal (e.g. fluorescence) being produced by the reporter molecule, which can be detected and/or quantified.

As mentioned above, the invention also provides a method, system and computer program embedded in a computer readable medium ("a computer program product") for designing detection probes comprising at least one stabilizing nucleobase. The method comprises querying a database of target sequences (e.g., such as the miRNA registry at http://www.sanger.ac.uk/Software/Rfam/mirna/index.shtml) and designing probes which: i) have sufficient binding stability to bind their respective target sequence under stringent hybridization conditions, ii) have limited propensity to form duplex structures with itself, and iii) are capable of binding to and detecting/quantifying at least about 60%, at least about 70%, at least about 80%, at least about 90% or at least about 95% of all the target sequences in the given database of miRNAs or other RNA sequences.

In one preferred aspect, the target sequence database comprises nucleic acid sequences corresponding to human, mouse, rat, *Drosophila melanogaster, C. elegans, Arabidopsis thaliana,* maize or rice miRNAs.

In another aspect, the method further comprises calculating stability based on the assumption that the recognition sequence comprises at least one stabilizing nucleotide, such as an LNA molecule. In one preferred aspect the calculated stability is used to eliminate probes with inadequate stability from the database of virtual candidate probes prior to the initial query against the database of target sequence to initiate the identification of optimal probe recognition sequences.

In another aspect, the method further comprises calculating the capability for a given probe sequence to form a duplex structure with itself based on the assumption that the sequence comprises at least one stabilizing nucleotide, such as an LNA molecule. In one preferred aspect the calculated propensity is used to eliminate probe sequences that are likely to form probe duplexes from the database of virtual candidate probes.

A preferred embodiment of the invention are kits for the detection or quantification of target miRNAs, siRNAs, RNA-edited transcripts, non-coding antisense transcripts or alternative splice variants comprising libraries of detection probes. In one aspect, the kit comprises in silico protocols for their use. The detection probes contained within these kits may have any or all of the characteristics described above. In one preferred aspect, a plurality of probes comprises at least one stabilizing nucleotide, such as an LNA nucleotide. In another aspect, the plurality of probes comprises a nucleotide coupled to or stably associated with at least one chemical moiety for increasing the stability of binding of the probe. The kits according to the invention allow a user to quickly and efficiently develop an assay for different miRNA targets, siRNA targets, RNA-edited transcripts, non-coding antisense transcripts or alternative splice variants.

The invention also provides a method, system and computer program embedded in a computer readable medium ("a computer program product") for designing detection probes comprising at least one stabilizing nucleobase. The method comprises querying a database of target sequences (e.g., such as the miRNA registry at http://www.sanger.ac.uk/Software/Rfam/mirnal/index.shtml) and designing probes which: i) have sufficient binding stability to bind their respective target sequence under stringent hybridization conditions, ii) have limited propensity to form duplex structures with itself, and iii) are capable of binding to and detecting/quantifying at least about 60%, at least about 70%, at least about 80%, at least about 90% or at least about 95% of all the target sequences in the given database of miRNAs or other RNA sequences.

In one preferred aspect, the target sequence database comprises nucleic acid sequences corresponding to human, mouse, rat, *Drosophila melanogaster, C. elegans, Arabidopsis thaliana,* maize or rice miRNAs.

In another aspect, the method further comprises calculating stability based on the assumption that the recognition sequence comprises at least one stabilizing nucleotide, such as an LNA molecule. In one preferred aspect the calculated stability is used to eliminate probes with inadequate stability from the database of virtual candidate probes prior to the initial query against the database of target sequence to initiate the identification of optimal probe recognition sequences.

In another aspect, the method further comprises calculating the capability for a given probe sequence to form a duplex structure with itself based on the assumption that the sequence comprises at least one stabilizing nucleotide, such as an LNA molecule. In one preferred aspect the calculated propensity is used to eliminate probe sequences that are likely to form probe duplexes from the database of virtual candidate probes.

In general, the invention features the design of high affinity oligonucleotide probes that have duplex stabilizing properties and methods highly useful for a variety of target nucleic acid detection methods (e.g., monitoring spatiotemporal expression of microRNAs or siRNAs or knock-down of miRNAs). Some of these oligonucleotide probes contain novel nucleotides created by combining specialized synthetic nucleobases with an LNA backbone, thus creating high affinity oligonucleotides with specialized properties such as reduced sequence discrimination for the complementary strand or reduced ability to form intramolecular double stranded structures. The invention also provides improved methods for detecting and quantifying ribonucleic acids in complex nucleic acid sample. Other desirable modified bases have decreased ability to self-anneal or to form duplexes with oligonucleotide probes containing one or more modified bases.

### EXAMPLES

The invention will now be further illustrated with reference to the following examples. It will be appreciated that what follows is by way of example only and that modifications to detail may be made while still falling within the scope of the invention.

### EXAMPLE 1

### Synthesis, deprotection and purification of LNA-substituted oligonucleotide probes

The LNA-substituted probes of Example 2 to 11 were prepared on an automated DNA synthesizer (Expedite 8909 DNA synthesizer, PerSeptive Biosystems, 0.2 µmol scale) using the phosphoramidite approach (Beaucage and Caruthers, Tetrahedron Lett. 22: 1859-1862, 1981) with 2-cyanoethyl protected LNA and DNA phosphoramidites, (Sinha, et al., Tetrahedron Lett.24: 5843-5846, 1983). CPG solid supports derivatised with a suitable quencher and 5'-fluorescein phosphoramidite (GLEN Research, Sterling, Virginia, USA). The synthesis cycle was modified for LNA phosphoramidites (250s coupling time) compared to DNA phosphoramidites. 1H-tetrazole or 4,5-dicyanoimidazole (Proligo, Hamburg, Germany) was used as activator in the coupling step.

The probes were deprotected using 32% aqueous ammonia (1h at room temperature, then 2 hours at 60°C) and purified by HPLC (Shimadzu-SpectraChrom series; Xterra™ RP18 column, 10?m 7.8 x 150 mm (Waters). Buffers: A: 0.05M Triethylammonium acetate pH 7.4. B. 50% acetonitrile in water. Eluent: 0-25 min: 10-80% B; 25-30 min: 80% B). The composition and purity of the probes were verified by MALDI-MS (PerSeptive Biosystem, Voyager DE-PRO) analysis.

### EXAMPLE 2

### List of LNA-substituted detection probes for detection of fully conserved vertebrate microRNAs in all vertebrates

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE A**

| **LNA probe name** | **Sequence 5'-3'** | **Self-comp score** | **Calculated Tm** |
|---|---|---|---|
| hsa-let7f/LNA | aamCtaTacAatmCtamCtamCctmCa | 16 | 67 |
| hsa-miR19b/LNA | tmCagTttTgcAtgGatTtgmCaca | 34 | 75 |
| hsa-miR17-5p/LNA | actAccTgcActGtaAgcActTtg | 39 | 74 |
| hsa-miR217/LNA | atcmCaaTcaGttmCctGatGcaGta | 49 | 75 |
| hsa-miR218/LNA | acAtgGttAgaTcaAgcAcaa | 40 | 70 |
| hsa-miR222/LNA | gaGacmCcaGtaGccAgaTgtAgct | 38 | 80 |
| hsa-let7i/LNA | agmCacAaamCtamCtamCctmCa | 18 | 71 |
| hsa-miR27b/LNA | cagAacTtaGccActGtgAa | 35 | 68 |
| hsa-miR301/LNA | gctTtgAcaAtamCtaTtgmCacTg | 36 | 70 |
| hsa-miR30b/LNA | gcTgaGtgTagGatGttTaca | 33 | 70 |
| hsa-miR100/LNA | cacAagTtcGgaTctAcgGgtt | 38 | 77 |
| hsa-miR34a/LNA | aamCaamCcaGctAagAcamCtgmCca | 27 | 80 |
| hsa-miR7/LNA | aacAaaAtcActAgtmCttmCca | 30 | 66 |
| hsa-miRl25b/LNA | tcamCaaGttAggGtcTcaGgga | 35 | 77 |
| hsa-miRl33a/LNA | acAgcTggTtgAagGggAccAa | 41 | 82 |
| hsa-miR101/LNA | cttmCagTtaTcamCagTacTgta | 54 | 68 |
| hsa-miR108/LNA | aatGccmCctAaaAatmCctTat | 23 | 66 |
| hsa-miR107/LNA | tGatAgcmCctGtamCaaTgcTgct | 63 | 80 |
| hsa-miR153/LNA | tcamCttTtgTgamCtaTgcAa | 35 | 68 |
| hsa-miR10b/LNA | amCaaAttmCggTtcTacAggGta | 35 | 73 |
| mmu-miR10b/LNA | acamCaaAttmCggTtcTacAggg | 27 | 73 |
| hsa-miR194/LNA | tccAcaTggAgtTgcTgtTaca | 41 | 75 |
| hsa-miR199a/LNA | gaAcaGgtAgtmCtgAacActGgg | 40 | 78 |
| hsa-miR199a*/LNA | aacmCaaTgtGcaGacTacTgta | 39 | 74 |
| hsa-miR20/LNA | ctAccTgcActAtaAgcActTta | 26 | 70 |
| hsa-miR214/LNA | ctGccTgtmCtgTgcmCtgmCtgt | 30 | 81 |
| hsa-miR219/LNA | agAatTgcGttTggAcaAtca | 35 | 70 |
| hsa-miR223/LNA | gGggTatTtgAcaAacTgamCa | 40 | 73 |
| hsa-miR23a/LNA | gGaaAtcmCctGgcAatGtgAt | 37 | 76 |
| hsa-miR24/LNA | cTgtTccTgcTgaActGagmCca | 35 | 80 |
| hsa-miR26a/LNA | agcmCtaTccTggAttActTgaa | 34 | 70 |
| hsa-miR126/LNA | gcAttAttActmCacGgtAcga | 25 | 71 |
| hsa-miR126*/LNA | cgmCgtAccAaaAgtAatAatg | 28 | 68 |
| hsa-miR128a/LNA | aaAagAgamCcgGttmCacTgtGa | 47 | 77 |
| mmu-miR7b/LNA | aamCaaAatmCacAagTctTcca | 24 | 68 |
| hsa-let7c/LNA | aamCcaTacAacmCtamCtamCctmCa | 11 | 74 |
| hsa-let7b/LNA | aamCcamCacAacmCtamCtamCctmCa | 6 | 77 |
| hsa-miR103/LNA | tmCatAgcmCctGtamCaaTgcTgct | 63 | 80 |
| hsa-miR129/LNA | agcAagmCccAgamCcgmCaaAaag | 21 | 80 |
| rno-miR129*/LNA | aTgcTttTtgGggTaaGggmCtt | 37 | 78 |
| hsa-miR130a/LNA | gcmCctTttAacAttGcamCtg | 34 | 70 |
| hsa-miR132/LNA | cgAccAtgGctGtaGacTgtTa | 48 | 76 |
| hsa-miR135a/LNA | tcamCatAggAatAaaAagmCcaTa | 22 | 69 |
| hsa-miR137/LNA | cTacGcgTatTctTaaGcaAta | 48 | 68 |
| hsa-miR200a/LNA | acaTcgTtamCcaGacAgtGtta | 39 | 72 |
| hsa-miR142-3p/LNA | tmCcaTaaAgtAggAaamCacTaca | 29 | 72 |
| hsa-miR142-5p/LNA | gtaGtgmCttTctActTtaTg | 36 | 63 |
| hsa-miR181b/LNA | aamCccAccGacAgcAatGaaTgtt | 30 | 81 |
| hsa-miR183/LNA | caGtgAatTctAccAgtGccAta | 32 | 73 |
| hsa-miR190/LNA | acmCtaAtaTatmCaaAcaTatmCa | 31 | 62 |
| hsa-miR193/LNA | ctGggActTtgTagGccAgtt | 31 | 76 |
| hsa-miR19a/LNA | tmCagTttTgcAtaGatTtgmCaca | 37 | 72 |
| hsa-miR204/LNA | cagGcaTagGatGacAaaGggAa | 25 | 78 |
| hsa-miR205/LNA | caGacTccGgtGgaAtgAagGa | 39 | 81 |
| hsa-miR216/LNA | camCagTtgmCcaGctGagAtta | 64 | 74 |
| hsa-miR221/LNA | gAaamCccAgcAgamCaaTgtAgct | 31 | 80 |
| hsa-miR25/LNA | tcaGacmCgaGacAagTgcAatg | 27 | 77 |
| hsa-miR29c/LNA | taamCcgAttTcaAatGgtGcta | 47 | 70 |
| hsa-miR29b/LNA | amCacTgaTttmCaaAtgGtgmCta | 47 | 71 |
| hsa-miR30c/LNA | gmCtgAgaGtgTagGatGttTaca | 33 | 73 |
| hsa-miR140/LNA | ctAccAtaGggTaaAacmCact | 43 | 71 |
| hsa-miR9*/LNA | acTttmCggTtaTctAgcTtta | 27 | 65 |
| hsa-miR92/LNA | amCagGccGggAcaAgtGcaAta | 36 | 81 |
| hsa-miR96/LNA | aGcaAaaAtgTgcTagTgcmCaaa | 38 | 75 |
| hsa-miR99a/LNA | cacAagAtcGgaTctAcgGgtt | 42 | 77 |
| hsa-miR145/LNA | aAggGatTccTggGaaAacTggAc | 50 | 79 |
| hsa-miR155/LNA | ccmCctAtcAcgAttAgcAttAa | 29 | 71 |
| hsa-miR29a/LNA | aamCcgAttTcaAatGgtGctAg | 47 | 75 |
| rno-miR140*/LNA | gtcmCgtGgtTctAccmCtgTggTa | 49 | 81 |
| hsa-miR206/LNA | ccamCacActTccTtamCatTcca | 11 | 73 |
| hsa-miR124a/LNA | tggmCatTcamCcgmCgtGccTtaa | 43 | 80 |
| hsa-miR122a/LNA | acAaamCacmCatTgtmCacActmCca | 25 | 78 |
| hsa-miR1/LNA | tamCatActTctTtamCatTcca | 11 | 64 |
| hsa-miR181a/LNA | acTcamCcgAcaGcgTtgAatGtt | 49 | 77 |
| hsa-miR10a/LNA | cAcaAatTcgGatmCtamCagGgta | 37 | 74 |
| hsa-miR196a/LNA | ccaAcaAcaTgaAacTacmCta | 20 | 67 |
| hsa-let7a/LNA | aamCtaTacAacmCtamCtamCctmCa | 16 | 70 |
| hsa-miR9/LNA | tcAtamCagmCtaGatAacmCaaAga | 34 | 71 |

### EXAMPLE 3

### List of LNA-substituted detection probes for detection of fully conserved vertebrate microRNAs in all vertebrates

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE B**

| **Probe name** | **Sequence 5'-3'** | **Self-compl score** | **Calculated Tm** |
|---|---|---|---|
| hsa-miR-210 | agcmCgcTgtmCacAcgmCacAg | 37 | 84 |
| hsa-miR-144 | taGtamCatmCatmCtaTacTgta | 37 | 64 |
| hsa-miR-338 | caAcaAaaTcamCtgAtgmCtgGa | 33 | 72 |
| hsa-miR-187 | ggcTgcAacAcaAga mCacGa | 30 | 79 |
| hsa-miR-200b | cAtcAttAccAggmCagTatTaga | 29 | 71 |
| hsa-miR-184 | cmCctTatmCagTtcTccGtcmCa | 23 | 75 |
| hsa-miR-27a | gcGgaActTagmCcamCtgTgaa | 35 | 77 |
| hsa-miR-215 | ctgTcaAttmCatAggTcat | 38 | 65 |
| hsa-miR-203 | agTggTccTaaAcaTttmCac | 23 | 68 |
| hsa-miR-16 | ccaAtaTttAcgTgcTgcTa | 30 | 68 |
| hsa-miR-152 | aAgtTctGtcAtgmCacTga | 29 | 72 |

### EXAMPLE 4

### List of LNA-substituted detection probes for detection of zebra fish microRNAs

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH2-C6- or a NH2-C6-hexaethylene glycol monomer or dimer group at the 5'-end or at the 3'-end of the probes during synthesis.

**TABLE C**

| **Probe name** | **Sequence 5'-3'** | **Self-comp score** | **Calculated Tm** |
|---|---|---|---|
| dre-miR-93 | ctAccTgcAcaAacAgcActTt | 26 | 73 |
| dre-miR-22 | acaGttmCttmCagmCtgGcaGctt | 62 | 76 |
| dre-miR-213 | gGtamCagTcaAcgGtcGatGgt | 63 | 80 |
| dre-miR-31 | cagmCtaTgcmCaamCatmCttGcc | 34 | 76 |
| dre-miR-189 | amCtgTtaTcaGctmCagTagGcac | 41 | 75 |
| dre-miR-18 | tatmCtgmCacTaaAtgmCacmCtta | 45 | 69 |
| dre-miR-15a | cAcaAacmCatTctGtgmCtgmCta | 35 | 74 |
| dre-miR-34b | cAatmCagmCtaAcaAcamCtgmCcta | 24 | 74 |
| dre-miR-148a | acaAagTtcTgtAatGcamCtga | 44 | 69 |
| dre-miR-125a | camCagGttAagGgtmCtcAggGa | 38 | 80 |
| dre-miR-139 | agAcamCatGcamCtgTaga | 34 | 69 |
| dre-miR-150 | cacTggTacAagGatTggGaga | 30 | 75 |
| dre-miR-192 | ggcTgtmCaaTtcAtaGgtmCa | 46 | 73 |
| dre-miR-98 | aacAacAcaActTacTacmCtca | 17 | 68 |
| dre-let-7g | amCtgTacAaamCaamCtamCctmCa | 30 | 73 |
| dre-miR-30a-5p | gctTccAgtmCggGgaTgtTtamCa | 45 | 80 |
| dre-miR-26b | aacmCtaTccTggAttActTgaa | 36 | 68 |
| dre-miR-21 | cAacAccAgtmCtgAtaAgcTa | 35 | 72 |
| dre-miR-146 | accmCttGgaAttmCagTtcTca | 40 | 72 |
| dre-miR-182 | tgtGagTtcTacmCatTgcmCaaa | 32 | 72 |
| dre-miR-182* | taGttGgcAagTctAgaAcca | 32 | 72 |
| dre-miR-220 | aAgtGtcmCgaTacGgtTgtGg | 47 | 81 |

### EXAMPLE 5

### List of LNA-substituted detection probes for detection of Drosophila melanogaster microRNAs.

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH2-C6- or a NH2-C6-hexaethylene glycol monomer or dimer group at the 5'-end or at the 3'-end of the probes during synthesis.

**TABLE D**

| **Probe name** | **Sequence 5'-3'** | **Self-compl score** | **Calculated Tm** |
|---|---|---|---|
| dme-miR-2c | gcmCcaTcaAagmCtgGctGtgAta | 68 | 78 |
| dme-miR-6 | aaaAagAacAgcmCacTgtGata | 36 | 71 |
| dme-miR-7 | amCaamCaaAatmCacTagTctTcca | 30 | 71 |
| dme-miR-14 | tAggAgaGagAaaAagActGa | 15 | 71 |
| dme-miR-277 | tgTcgTacmCagAtaGtgmCatTta | 38 | 72 |
| dme-miR-278 | aaAcgGacGaaAgtmCccAccGa | 41 | 80 |
| dme-miR-279 | tTaaTgaGtgTggAtcTagTca | 40 | 70 |
| dme-miR-309 | tAggAcaAacTttAccmCagTgc | 37 | 74 |
| dme-miR-310 | aAagGccGggAagTgtGcaAta | 28 | 79 |
| dme-miR-318 | tgaGatAaamCaaAgcmCcaGtga | 25 | 73 |
| dme-miR-bantam | aaTcaGctTtcAaaAtgAtcTca | 40 | 66 |

### EXAMPLE 6

### List of LNA-substituted detection probes for detection of Drosophila melanogaster and Caenorhabditis elegans microRNAs

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE E**

| **Probe name** | **Sequence 5'-3'** | **Self-comp score** | **Calculated Tm** |
|---|---|---|---|
| dme_cel-miR1/LNA | cAtamCttmCttTacAttmCca | 14 | 62 |
| dme_cel-miR2/LNA | tcaAagmCtgGctGtgAta | 56 | 67 |
| cel-lin4/LNA | tcAcamCttGagGtcTcag | 50 | 68 |

### EXAMPLE 7

### List of LNA-substituted detection probes for detection of Arabidopsis thaliana microRNAs

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE F**

| **Probe name** | **Sequence 5'-3'** | **Self-comp score** | **Calculated Tm** |
|---|---|---|---|
| ath-MIR171_LNA2 | gAtAtTgGcGcGgmCtmCaAtmCa | 64 | 83 |
| ath-MIR171_LNA3 | gAtaTtgGcgmCggmCtcAatmCa | 54 | 78 |
| ath-MIR159_LNA2 | tAgAgmCtmCcmCtTcAaTcmCaAa | 46 | 79 |
| ath-MIR159_LNA3 | tAgaGctmCccTtcAatmCcaAa | 43 | 72 |
| ath-MIR161LNA3 | cmCccGatGtaGtcActTtcAa | 34 | 73 |
| ath-MIR167LNA3 | tAgaTcaTgcTggmCagmCttmCa | 53 | 79 |
| ath-MIR319LNA3 | ggGagmCtcmCctTcaGtcmCaa | 70 | 78 |

### EXAMPLE 8

### List of LNA-substituted detection probes for detection of Arabidopsis thaliana microRNAs

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE G**

| **Oligo name** | **Sequence 5'-3'** | **Predicted Tm °C** |
|---|---|---|
| ath-miR159a/LNA | tAgaGctmCccTtcAatmCcaAa | 145 |
| ath-miR319a/LNA | ggGagmCtcmCctTcaGtcmCaa | 183 |
| ath-miR396a/LNA | gTtcAagAaaGctGtgGaa | 242 |
| ath-miR156a/LNA | gtgmCtcActmCtcTtcTgtmCa | 235 |
| ath-miR172a/LNA | atgmCagmCatmCatmCaaGatTct | 228 |

### EXAMPLE 9

### List of LNA-substituted detection probes useful as controls in detection of vertebrate microRNAs

LNA nucleotides are depicted by capital letters, DNA nucleotides by lowercase letters, mC denotes LNA methyl-cytosine. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE H**

| **Probe name** | **Sequence 5'-3'** | **Self-comp score** |
|---|---|---|
| hsa-miR206/LNA/2MM | ccamCacActmCtcTtamCatTcca | 8 |
| hsa-miR206/LNA/MM10 | ccamCacActmCccTtamCatTcca | 8 |
| hsa-miR124a/LNA/2MM | tggmCatTcaAagmCgtGccTtaa | 60 |
| hsa-miR124a/LNA/MM10 | tggmCatTcaAcgmCgtGccTtaa | 60 |
| hsa-miR122a/LNA/2MM | acAaamCacmCacmCgtmCacActmCca | 18 |
| hsa-miR122a/LNA/MM11 | acAaamCacmCatmCgtmCacActmCca | 18 |

### EXAMPLE 10

### List of LNA-substituted detection probes for detection of human microRNAs

LNA nucleotides are depicted by capital letters, DNA nucleotides by lowercase letters, mC denotes LNA methyl-cytosine, PM perfect match to the miRNA, MM one mismatch at the central position of the probe sequence. The detection probes can be used to detect and analyze conserved vertebrate miRNAs by RNA in situ hybridization, Northern blot analysis and by silencing using the probes as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE I**

| **Probe name** | **Sequence 5'-3'** |
|---|---|
| hsa-let7a/LNA_PM | aamCtaTacAacmCtamCtamCctmCa |
| hsa-let7f/LNA_PM | aamCtaTacAatmCtamCtamCctmCa |
| hsa-miR143LNA_PM | tGagmCtamCagTgcTtcAtcTca |
| hsa-miR145/LNA_PM | aAggGatTccTggGaaAacTggAc |
| hsa-miR320/LNA_PM | tTcgmCccTctmCaamCccAgcTttt |
| hsa-miR26a/LNA_PM | agcmCtaTccTggAttActTgaa |
| hsa-miR99a/LNA_PM | cacAagAtcGgaTctAcgGgtt |
| hsa-miR15a/LNA_PM | cAcaAacmCatTatGtgmCtgmCta |
| hsa-miRl6-1/LNA PM | cgmCcaAtaTttAcgTgcTgcTa |
| hsa-miR24/LNA_PM | cTgtTccTgcTgaActGagmCca |
| hsa-let7g/LNA_PM | amCtgTacAaamCtamCtamCctmCa |
| hsa-let7a/LNA_MM | aamCtaTacAacAtamCtamCctmCa |
| hsa-let7f/LNA_MM | aamCtaTacAatAtamCtamCctmCa |
| hsa-miR143LNA_MM | tGagmCtamCagmCgcTtcAtcTca |
| hsa-miR145/LNA_MM | aAggGatTccTcgGaaAacTggAc |
| hsa-miR320/LNA_MM | tTcgmCccTctAaamCccAgcTttt |
| hsa-miR26a/LNA_MM | agcmCtaTccTcgAttActTgaa |
| hsa-miR99a/LNA_MM | cacAagAtcGcaTctAcgGgtt |
| hsa-miR15a/LNA_MM | cAcaAacmCatmCatGtgmCtgmCta |
| hsa-miR16-1/LNA_MM | cgmCcaAtaTttTcgTgcTgcTa |
| hsa-miR24/LNA_MM | cTgtTccTgcmCgaActGagmCca |
| hsa-let7g/LNA_MM | amCtgTacAaaAtamCtamCctmCa |

### EXAMPLE 11

### List of LNA-substituted detection probes for expression profiling of human and mouse microRNAs by oligonucleotide microarrays

LNA nucleotides are depicted by capital letters, DNA nucleotides by lowercase letters, mC denotes LNA methyl-cytosine, PM perfect match to the miRNA, MM one mismatch at the central position of the probe sequence, dir denotes the probe sequence corresponding to the mature miRNA sequence, rev denotes the probe sequence complementary to the mature miRNA sequence in question. The detection probes can be used t as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group at the 5'-end or at the 3'-end of the probes during synthesis.

**TABLE J**

| **Probe name** | **Sequence 5'-3'** | **Self-comp score** |
|---|---|---|
| mmu-let7adirPM/LNA | tgaGgtAgtAggTtgTatAgtt | 30 |
| mmu-miR1dirPM/LNA | tgGaaTgtAaaGaaGtaTgta | 18 |
| mmu-miR16dirPM/LNA | tagmCagmCacGtaAatAttGgcg | 46 |
| mmu-miR22dirPM/LNA | aagmCtgmCcaGttGaaGaamCtgt | 48 |
| mmu-miR26bdirPM/LNA | tTcaAgtAatTcaGgaTagGtt | 35 |
| mmu-miR30cdirPM/LNA | tgtAaamCatmCctAcamCtcTcaGc | 27 |
| mmu-miRl22adirPM/LNA | tggAgtGtgAcaAtgGtgTttg | 32 |
| mmu-miR126stardirPM/LNA | catTatTacTttTggTacGcg | 28 |
| mmu-miR126dirPM/LNA | tcgTacmCgtGagTaaTaaTgc | 32 |
| mmu-miR133dirPM/LNA | tTggTccmCctTcaAccAgcTgt | 37 |
| mmu-miR143dirPM/LNA | tGagAtgAagmCacTgtAgcTca | 49 |
| mmu-miR144dirPM/LNA | tAcaGtaTagAtgAtgTacTag | 41 |
| mmu-let7arevPM/LNA | aamCtaTacAacmCtamCtamCctmCa | 16 |
| mmu-miR1revPM/LNA | tamCatActTctTtamCatTcca | 11 |
| mmu-miR16revPM/LNA | cgmCcaAtaTttAcgTgcTgcTa | 34 |
| mmu-miR22revPM/LNA | acaGttmCttmCaamCtgGcaGctt | 48 |
| mmu-miR26brevPM/LNA | aacmCtaTccTgaAttActTgaa | 28 |
| mmu-miR30crevPM/LNA | gmCtgAgaGtgTagGatGttTaca | 33 |
| mmu-miR122arevPM/LNA | cAaamCacmCatTgtmCacActmCca | 25 |
| mmu-miR126starrevPM/LNA | cgmCgtAccAaaAgtAatAatg | 28 |
| mmu-miR126revPM/LNA | gcAttAttActmCacGgtAcga | 25 |
| mmu-miR133revPM/LNA | acAgcTggTtgAagGggAccAa | 41 |
| mmu-miR143revPM/LNA | tGagmCtamCagTgcTtcAtcTca | 56 |
| mmu-miR144revPM/LNA | ctaGtamCatmCatmCtaTacTgta | 37 |
| mmu-let7adirMM/LNA | tgaGgtAgtAagTtgTatAgtt | 34 |
| mmu-miR1dirMM/LNA | tgGaaTgtAagGaaGtaTgta | 18 |
| mmu-miR16dirMM/LNA | tAgcAgcAcgGaaAtaTtgGcg | 33 |
| mmu-miR22dirMM/LNA | aaGctGccAggTgaAgaActGt | 35 |
| mmu-miR26bdirMM/LNA | tTcaAgtAatGcaGgaTagGtt | 27 |
| mmu-miR30cdirMM/LNA | tgtAaamCatmCatAcamCtcTcaGc | 27 |
| mmu-miR122adirMM/LNA | tggAgtGtgAaaAtgGtgTttg | 29 |
| mmu-miR126stardirMM/LNA | catTatTacTgtTggTacGcg | 35 |
| mmu-miR126dirMM/LNA | tmCgtAccGtgGgtAatAatGc | 39 |
| mmu-miR133dirMM/LNA | ttgGtcmCccTgcAacmCagmCtgt | 42 |
| mmu-miR143dirMM/LNA | tGagAtgAagAacTgtAgcTca | 49 |
| mmu-miR144dirMM/LNA | tAcaGtaTagGtgAtgTacTag | 41 |
| mmu-let7arevMM/LNA | aActAtamCaamCttActAccTca | 17 |
| mmu-miRirevMM/LNA | tacAtamCttmCctTacAttmCca | 11 |
| mmu-miR16revMM/LNA | cgmCcaAtaTttmCcgTgcTgcTa | 34 |
| mmu-miR22revMM/LNA | amCagTtcTtcAccTggmCagmCtt | 35 |
| mmu-miR26brevMM/LNA | aamCctAtcmCtgmCatTacTtgAa | 24 |
| mmu-miR30crevMM/LNA | gmCtgAgaGtgTatGatGttTaca | 29 |
| mmu-miR122arevMM/LNA | cAaamCacmCatTttmCacActmCca | 13 |
| mmu-miR126starrevMM/LNA | cgmCgtAccAacAgtAatAatg | 31 |
| mmu-miR126revMM/LNA | gmCatTatTacmCcamCggTacGa | 39 |
| mmu-miR133revMM/LNA | acaGctGgtTgcAggGgamCcaa | 45 |
| mmu-miR143revMM/LNA | tgAgcTacAgtTctTcaTctmCa | 49 |
| mmu-miR144revMM/LNA | ctAgtAcaTcamCctAtamCtgTa | 31 |

### EXAMPLE 12

### List of LNA-substituted detection probes for detection of all microRNAs listed in the miRNA registry database release 5.1 from December 2004 at http://www.sanger.ac.uk/Software/Rfam/mirna/index.shtml

LNA nucleotides are depicted by capital letters, DNA nucleotides by lowercase letters, mC denotes LNA methyl-cytosine. The detection probes can be used to detect and analyze miRNAs by RNA in situ hybridization, Northern blot analysis and by silencing using the oligonucleotides as miRNA inhibitors. The LNA-modified probes can be conjugated with a variety of haptens or fluorochromes for miRNA in situ hybridization using standard methods. 5'-end labeling using T4 polynucleotide kinase and gamma-32P-ATP can be carried out by standard methods for Northern blot analysis. In addition, the LNA-modified probe sequences can be used as capture sequences for expression profiling by LNA oligonucleotide microarrays. Covalent attachment to the solid surfaces of the capture probes can be accomplished by incorporating a NH₂-C₆- or a NH₂-C₆-hexaethylene glycol monomer or dimer group, or a NH₂-C₆-random N₂₀ sequence at the 5'-end or at the 3'-end of the probes during synthesis. Ath, Arabidopsis thaliana; cbr, Caenorhabditis briggsae; cel, Caenorhabditis elegans; dme, Drosophila melanogaster, dps, Drosophila pseudoobscura; dre, Danio rerio; ebr, Eppstein Barr Virus; gga, Gallus gallus; has, Homo sapiens; mmu, Mus musculus; osa, Oryza sativa; rno, Rattus norvegicus; zma, Zea mays.

**TABLE K**

| **Probe name** | **Probe sequence (5'-3')** | **Calc Tm °C** | **Self-complem. score** |
|---|---|---|---|
| ath-miR156a | gtgmCtcActmCtcTtcTgtmCa | 71 | 25 |
| ath-miR156b | gtgmCtcActmCtcTtcTgtmCa | 71 | 25 |
| ath-miR156c | gtgmCtcActmCtcTtcTgtmCa | 71 | 25 |
| ath-miR156d | gtgmCtcActmCtcTtcTgtmCa | 71 | 25 |
| ath-miR156e | gtgmCtcActmCtcTtcTgtmCa | 71 | 25 |
| ath-miR156f | gtgmCtcActmCtcTtcTgtmCa | 71 | 25 |
| ath-miR156g | tgTgcTcamCtcTctTctGtcg | 74 | 31 |
| ath-miR156h | gtgmCtcTctTtcTtcTgtmCaa | 68 | 25 |
| ath-miR157a | gtgmCtcTctAtcTtcTgtmCaa | 68 | 25 |
| ath-miR157b | gtgmCtcTctAtcTtcTgtmCaa | 68 | 25 |
| ath-miR157c | gtgmCtcTctAtcTtcTgtmCaa | 68 | 25 |
| ath-miR157d | gTgcTctmCtaTctTctGtca | 69 | 21 |
| ath-miR158a | tgmCttTgtmCtamCatTtgGga | 71 | 28 |
| ath-miR158b | tgmCttTgtmCtamCatTtgGgg | 72 | 28 |
| ath-miR159a | tagAgcTccmCttmCaaTccAaa | 71 | 36 |
| ath-miR159b | aagAgcTccmCttmCaaTccAaa | 72 | 36 |
| ath-miR159c | aggAgcTccmCttmCaaTccAaa | 74 | 46 |
| ath-miR160a | tggmCatAcaGggAgcmCagGca | 85 | 49 |
| ath-miR160b | tggmCatAcaGggAgcmCagGca | 85 | 49 |
| ath-miR160c | tggmCatAcaGggAgcmCagGca | 85 | 49 |
| ath-miR161 | cccmCgaTgtAgtmCacTttmCaa | 75 | 27 |
| ath-miR162a | ctgGatGcaGagGttTatmCga | 73 | 34 |
| ath-miR162b | ctgGatGcaGagGttTatmCga | 73 | 34 |
| ath-miR163 | aTcgAagTtcmCaaGtcmCtcTtcAa | 74 | 29 |
| ath-miR164a | tgcAcgTgcmCctGctTctmCca | 82 | 46 |
| ath-miR164b | tgcAcgTgcmCctGctTctmCca | 82 | 46 |
| ath-miR164c | cgcAcgTgcmCctGctTctmCca | 83 | 46 |
| ath-miR165a | gggGgaTgaAgcmCtgGtcmCga | 84 | 46 |
| ath-miR165b | gggGgaTgaAgcmCtgGtcmCga | 84 | 46 |
| ath-miR166a | gGggAatGaaGccTggTccGa | 84 | 33 |
| ath-miR166b | gGggAatGaaGccTggTccGa | 84 | 33 |
| ath-miR166c | gGggAatGaaGccTggTccGa | 84 | 33 |
| ath-miR166d | gGggAatGaaGccTggTccGa | 84 | 33 |
| ath-miR166e | gGggAatGaaGccTggTccGa | 84 | 33 |
| ath-miR166f | gGggAatGaaGccTggTccGa | 84 | 33 |
| ath-miR166g | gGggAatGaaGccTggTccGa | 84 | 33 |
| ath-miR167a | tAgaTcaTgcTggmCagmCttmCa | 79 | 53 |
| ath-miR167b | tAgaTcaTgcTggmCagmCttmCa | 79 | 53 |
| ath-miR167c | aAgaTcaTgcTggmCagmCttAa | 76 | 53 |
| ath-miR167d | ccAgaTcaTgcTggmCagmCttmCa | 82 | 53 |
| ath-miR168a | ttcmCcgAccTgcAccAagmCga | 82 | 26 |
| ath-miR168b | ttcmCcgAccTgcAccAagmCga | 82 | 26 |
| ath-miR169a | tcGgcAagTcaTccTtgGctg | 78 | 40 |
| ath-miR169b | ccGgcAagTcaTccTtgGctg | 79 | 40 |
| ath-miR169c | ccGgcAagTcaTccTtgGctg | 79 | 40 |
| ath-miR169d | cGgcAagTcaTccTtgGctmCa | 80 | 35 |
| ath-miR169e | cGgcAagTcaTccTtgGctmCa | 80 | 35 |
| ath-miR169f | cGgcAagTcaTccTtgGctmCa | 80 | 35 |
| ath-miR169g | cGgcAagTcaTccTtgGctmCa | 80 | 35 |
| ath-miR169g* | aGccAagGtcAacTtgmCcgGa | 81 | 45 |
| ath-miR169h | caGgcAagTcaTccTtgGcta | 76 | 41 |
| ath-miR169i | caGgcAagTcaTccTtgGcta | 76 | 41 |
| ath-miR169j | caGgcAagTcaTccTtgGcta | 76 | 41 |
| ath-miR169k | caGgcAagTcaTccTtgGcta | 76 | 41 |
| ath-miR1691 | caGgcAagTcaTccTtgGcta | 76 | 41 |
| ath-miR169m | caGgcAagTcaTccTtgGcta | 76 | 41 |
| ath-miR169n | caGgcAagTcaTccTtgGcta | 76 | 41 |
| ath-miR170 | gAtaTtgAcamCggmCtcAatmCa | 72 | 52 |
| ath-miR171a | gAtaTtgGcgmCggmCtcAatmCa | 78 | 54 |
| ath-miR171b | cGtgAtaTtgGcamCggmCtcAa | 77 | 43 |
| ath-miR171c | cGtgAtaTtgGcamCggmCtcAa | 77 | 43 |
| ath-miR172a | atgmCagmCatmCatmCaaGatTct | 73 | 45 |
| ath-miR172b | atgmCagmCatmCatmCaaGatTct | 73 | 45 |
| ath-miR172b* | gtgAatmCttAatGgtGctGc | 72 | 33 |
| ath-miR172c | ctgmCagmCatmCatmCaaGatTct | 73 | 39 |
| ath-miR172d | ctgmCagmCatmCatmCaaGatTct | 73 | 39 |
| ath-miRl72e | aTgcAgcAtcAtcAagAttmCc | 74 | 39 |
| ath-miRl73 | gtgAttTctmCtcTgcAagmCgaa | 72 | 38 |
| ath-miR319a | gggAgcTccmCttmCagTccAa | 77 | 64 |
| ath-miR319b | gggAgcTccmCttmCagTccAa | 77 | 64 |
| ath-miR319c | aggAgcTccmCttmCagTccAa | 76 | 46 |
| ath-miR393a | gAtcAatGcgAtcmCctTtgGa | 74 | 56 |
| ath-miR393b | gAtcAatGcgAtcmCctTtgGa | 74 | 56 |
| ath-miR394a | gGagGtgGacAgaAtgmCcaa | 77 | 29 |
| ath-miR394b | gGagGtgGacAgaAtgmCcaa | 77 | 29 |
| ath-miR395a | gAgtTccmCccAaamCacTtcAg | 77 | 28 |
| ath-miR395b | gagTccmCccmCaaAcamCttmCag | 77 | 21 |
| ath-miR395c | gagTccmCccmCaaAcamCttmCag | 77 | 21 |
| ath-miR395d | gAgtTccmCccAaamCacTtcAg | 77 | 28 |
| ath-miR395e | gAgtTccmCccAaamCacTtcAg | 77 | 28 |
| ath-miR395f | gagTccmCccmCaaAcamCttmCag | 77 | 21 |
| ath-miR396a | cagTtcAagAaaGctGtgGaa | 70 | 35 |
| ath-miR396b | aagTtcAagAaaGctGtgGaa | 69 | 24 |
| ath-miR397a | caTcaAcgmCtgmCacTcaAtga | 73 | 39 |
| ath-miR397b | caTcaAcgAtgmCacTcaAtga | 70 | 35 |
| ath-miR398a | aagGggTgamCctGagAacAca | 80 | 39 |
| ath-miR398b | cagGggTgamCctGagAacAca | 81 | 51 |
| ath-miR398c | cagGggTgamCctGagAacAca | 81 | 51 |
| ath-miR399a | cAggGcaAatmCtcmCttTggmCa | 78 | 48 |
| ath-miR399b | caGggmCaamCtcTccTttGgca | 81 | 39 |
| ath-miR399c | caGggmCaamCtcTccTttGgca | 81 | 39 |
| ath-miR399d | cggGgcAaaTctmCctTtgGca | 79 | 47 |
| ath-miR399e | cgaGgcAaaTctmCctTtgGca | 76 | 41 |
| ath-miR399f | cmCggGcaAatmCtcmCttTggmCa | 80 | 41 |
| ath-miR400 | gTgamCttAtaAtamCtcTcaTa | 63 | 32 |
| ath-miR401 | tgtmCggTcgAcamCcaGttTcg | 78 | 59 |
| ath-miR402 | cAgaGgtTtaAtaGgcmCtcGaa | 76 | 68 |
| ath-miR403 | cgAgtTtgTgcGtgAatmCtaa | 71 | 46 |
| ath-miR404 | gmCtgmCcgmCaamCcgmCcaGcgTtaAt | 88 | 55 |
| ath-miR405a | agTtaTggGttAgamCccAacTcat | 74 | 64 |
| ath-miR405b | agTtaTggGttAgamCccAacTcat | 74 | 64 |
| ath-miR405d | agTtaTggGttAgamCccAacTcat | 74 | 64 |
| ath-miR406 | ctGgaTtamCaaTagmCatTcta | 67 | 38 |
| ath-miR407 | amCcaAaaGtaTatGatTtaAa | 61 | 36 |
| ath-miR408 | gmCcaGggAagAggmCagTgcAt | 87 | 35 |
| ath-miR413 | gtgmCagAacAagAgaAacTat | 69 | 24 |
| ath-miR414 | tGacGatGatGatGaaGatGa | 75 | 22 |
| ath-miR415 | atgTtcTgtTtcTgcTctGtt | 68 | 15 |
| ath-miR416 | tGaamCagTgtAcgTacGaamCc | 78 | 52 |
| ath-miR417 | tcgAacAaaTtcActAccTtc | 65 | 21 |
| ath-miR418 | ggtmCagTtcAtcAtcAcaTta | 66 | 22 |
| ath-miR419 | caamCatmCctmCagmCatTcaTaa | 71 | 18 |
| ath-miR420 | tGcaTttmCcgTgaTtaGttTa | 68 | 27 |
| ath-miR426 | cgTaaGgamCaaAttTccAaaa | 68 | 31 |
| cbr-let-7 | aamCtaTacAacmCtamCtamCctmCa | 70 | 16 |
| cbr-lin-4 | tcamCacTtgAggTctmCagGga | 78 | 70 |
| cbr-16 | cggAatGcgTctmCatAcaAaa | 71 | 40 |
| cbr-mi R-1 | tamCatActTctTtamCatTcca | 64 | 11 |
| cbr-miR-124 | tggmCatTcamCcgmCgtGccTta | 80 | 43 |
| cbr-miR-228 | ccGtgAatTcaTgcAgtGccAtt | 78 | 56 |
| cbr-miR-230 | ttTccTggTcgmCacAacTaaTac | 74 | 27 |
| cbr-miR-231 | tccTgcmCtgTtgTtcAcgAgcTta | 77 | 39 |
| cbr-miR-232 | tcAccGcaGttAagAtgmCatTta | 71 | 44 |
| cbr-miR-233 | tccmCgcAcaTgcGcaTtgmCtcAa | 83 | 59 |
| cbr-miR-234 | aAggGtaTtcTcgAgcAatAa | 70 | 46 |
| cbr-miR-236 | agmCgtmCatTacmCtgAcaGtaTta | 71 | 36 |
| cbr-miR-239a | cmCagTacmCtaAttGtaGtamCaaa | 68 | 44 |
| cbr-miR-239b | caGtamCttTtgTgcAgtAcaa | 68 | 51 |
| cbr-miR-240 | agcGaaAatTtgGagGccAgta | 74 | 33 |
| cbr-miR-241 | tmCatTtcTcamCacmCtamCctmCa | 74 | 7 |
| cbr-miR-244 | catAccActTtgTacAacmCaaAga | 70 | 40 |
| cbr-miR-245 | gaGctActTggAggGgamCcaAt | 80 | 33 |
| cbr-miR-246 | aGctmCctAccmCaaTacAtgTaa | 73 | 40 |
| cbr-miR-248 | tGagmCgtTatmCcgAgcAcgTgta | 82 | 59 |
| cbr-miR-249 | gmCaamCacTcaAaaAtcmCtgTga | 73 | 23 |
| cbr-miR-250 | cmCgtGccAacAgtTgamCtgTga | 81 | 58 |
| cbr-miR-251 | aatAagAgcGgcAccActActTaa | 74 | 41 |
| cbr-miR-252 | gttAccTgcGgcActActActTa | 75 | 28 |
| cbr-miR-253 | agtTagTgtTagTgaGgtGtg | 72 | 32 |
| cbr-miR-254 | tAtamCagTtgmCaaAagAttTgca | 69 | 51 |
| cbr-miR-259 | aacmCagAttAggAtgAgaTtt | 67 | 31 |
| cbr-miR-268 | amCcaAaamCtgmCttmCtaAttmCttGcc | 73 | 23 |
| cbr-miR-34 | cAacmCagmCtaAccAcamCtgmCct | 80 | 24 |
| cbr-miR-35 | cTtgmCaaGttTtcAccmCggTga | 77 | 52 |
| cbr-miR-353 | gaTacmCaamCacAtgAtamCttg | 68 | 23 |
| cbr-miR-354 | aggAgcAgcAacAaamCaaGgt | 79 | 23 |
| cbr-miR-355 | catAgcTcaGgcTaaAacAaa | 70 | 45 |
| cbr-miR-356 | ggAttTgtTcgmCgtTgcTcat | 74 | 29 |
| cbr-miR-357 | tccGtcAatGacTggmCatTtt | 73 | 52 |
| cbr-miR-358 | ccamCgamCtaAggAtamCcaAttg | 72 | 26 |
| cbr-miR-36 | aTtgmCgaAttTtcAccmCggTga | 76 | 44 |
| cbr-miR-360 | ttGtgAacGggAttAcgGtca | 75 | 46 |
| cbr-miR-38 | aTacmCagGttGtcTccmCggTga | 80 | 53 |
| cbr-miR-39 | cTaamCcgTttTtcAccmCggTga | 76 | 49 |
| cbr-miR-40 | ctAgcTgaTtgAcamCccGgtGa | 81 | 57 |
| cbr-miR-41 | tggGagTttTtcAccmCggTga | 76 | 44 |
| cbr-miR-42 | cTgtAgaTgtTaamCccGgtg | 76 | 39 |
| cbr-miR-43 | gcGacAgcAagTaaActGtgAta | 74 | 32 |
| cbr-miR-44 | agcTgaAtgTgtmCtcTagTca | 70 | 30 |
| cbr-miR-45 | agcTgaAtgTgtmCtcTagTca | 70 | 30 |
| cbr-miR-46 | tgAagAgaGcgActmCcaTgamCa | 79 | 33 |
| cbr-miR-47 | tGaaGagAgcGccTccAtgAca | 80 | 38 |
| cbr-miR-48 | tcgmCatmCtamCtgAgcmCtamCctmCa | 79 | 31 |
| cbr-miR-49 | tcTgcAgcTtcTcgTggTgcTt | 80 | 36 |
| cbr-miR-50 | aamCccAagAatAtcAgamCatAtca | 71 | 23 |
| cbr-miR-51 | aacAtgGcaAggAgcTacGggTa | 80 | 34 |
| cbr-miR-52 | agmCacGgaAacAtaTgtAcgGgtg | 81 | 44 |
| cbr-miR-55 | ctcGgcAgaAaaAtaTacGggTa | 75 | 32 |
| cbr-miR-57 | acamCacAgcTcgAtcTacAggGta | 78 | 47 |
| cbr-miR-58 | aTtgmCcgTacTgaAcgAtcTca | 75 | 32 |
| cbr-miR-60 | tgGacTagAaaAtgTgcAtaAta | 67 | 34 |
| cbr-miR-61 | gAgcAgaGtcAagGttmCtaGtca | 74 | 53 |
| cbr-miR-62 | ctgTaaGctAgaTtamCatAtca | 65 | 60 |
| cbr-miR-64 | tccGtamCacGctTcaGtgTcaTg | 79 | 41 |
| cbr-miR-67 | tctActmCttTctAggAggTtgTga | 73 | 54 |
| cbr-miR-70 | ctGggAacAccAatmCacGtaTta | 74 | 29 |
| cbr-miR-71 | tcamCtamCccAtgTctTtca | 67 | 20 |
| cbr-miR-72 | gmCtaTgcmCaamCatmCtgmCct | 77 | 29 |
| cbr-miR-73 | amCtgAacTgcmCaamCatmCttGcca | 79 | 44 |
| cbr-miR-74 | tctAgamCtgmCcaTttmCttGcca | 74 | 28 |
| cbr-miR-75 | tGaaGgcGgtTggTagmCttTaa | 79 | 48 |
| cbr-miR-77 | tggAcaGctAtgGccTgaTgaa | 76 | 48 |
| cbr-miR-79 | aGctTtgGtaAccTagmCttTat | 67 | 52 |
| cbr-miR-80 | tcGgcTttmCaamCtaAtgAtcTca | 72 | 27 |
| cbr-miR-81 | acTagmCttTcamCgaTgaTctmCa | 73 | 27 |
| cbr-miR-82 | amCtgGctTtcAcgAtgAtcTca | 73 | 30 |
| cbr-miR-83 | acamCtgAatTtaTatGgtGcta | 67 | 47 |
| cbr-miR-84 | gacAgcAttGcaAacTacmCtca | 73 | 36 |
| cbr-miR-85 | gmCacGccTttTcaAatActTtgTa | 71 | 33 |
| cbr-miR-86 | gActGtgGcaAagmCatTcamCtta | 73 | 44 |
| cbr-miR-87 | amCacmCtgAaamCttTgcTcac | 72 | 20 |
| cbr-miR-90 | gGggmCatTcaAacAacAtaTca | 73 | 23 |
| cel-let-7 | aamCtaTacAacmCtamCtamCctmCa | 70 | 16 |
| cel-lin-4 | tcamCacTtgAggTctmCagGga | 78 | 70 |
| cel-16 | cgaAatGcgTctmCatAcaAaa | 69 | 44 |
| cel-mi R-1 | tamCatActTctTtamCatTcca | 64 | 11 |
| cel-miR-124 | tggmCatTcamCcgmCgtGccTta | 80 | 43 |
| cel-miR-2 | gcAcaTcaAagmCtgGctGtgAta | 75 | 68 |
| cel-miR-227 | gttmCagAatmCatGtcGaaAgct | 71 | 34 |
| cel-miR-228 | ccGtgAatTcaTgcAgtGccAtt | 78 | 56 |
| cel-miR-229 | acgAtgGaaAagAtaAccAgtGtcAtt | 74 | 43 |
| cel-miR-230 | tcTccTggTcgmCacAacTaaTac | 76 | 27 |
| cel-miR-231 | ttcTgcmCtgTtgAtcAcgAgcTta | 75 | 46 |
| cel-miR-232 | tcAccGcaGttAagAtgmCatTta | 71 | 44 |
| cel-miR-233 | tccmCgcAcaTgcGcaTtgmCtcAa | 83 | 59 |
| cel-miR-234 | aAggGtaTtcTcgAgcAatAa | 70 | 46 |
| cel-miR-235 | tcAggmCcgGggAgaGtgmCaaTa | 85 | 39 |
| cel-miR-236 | agmCgtmCatTacmCtgAcaGtaTta | 71 | 36 |
| cel-miR-237 | aAgcTgtTcgAgaAttmCtcAggGa | 78 | 54 |
| cel-miR-238 | tcTgaAtgGcaTcgGagTacAaa | 75 | 34 |
| cel-miR-239a | ccaGtamCctAtgTgtAgtAcaAa | 71 | 50 |
| cel-miR-239b | cAgtActTttGtgTagTacAa | 68 | 45 |
| cel-miR-240 | agcGaaGatTtgGggGccAgta | 80 | 33 |
| cel-miR-241 | tmCatTtcTcgmCacmCtamCctmCa | 76 | 18 |
| cel-miR-242 | tmCgaAgcAaaGgcmCtamCgcAa | 82 | 49 |
| cel-miR-243 | gatAtcmCcgmCcgmCgaTcgTacmCg | 84 | 58 |
| cel-miR-244 | catAccActTtgTacAacmCaaAga | 70 | 40 |
| cel-miR-245 | gaGctActTggAggGgamCcaAt | 80 | 33 |
| cel-miR-246 | aGctmCctAccmCgaAacAtgTaa | 75 | 30 |
| cel-miR-247 | aAgaAgaGaaTagGctmCtaGtca | 71 | 50 |
| cel-miR-248 | tGagmCgtTatmCcgTgcAcgTgta | 82 | 48 |
| cel-miR-249 | gcaAcgmCtcAaaAgtmCctGtga | 74 | 35 |
| cel-miR-250 | cmCatGccAacAgtTgamCtgTga | 79 | 58 |
| cel-miR-251 | aatAagAgcGgcAccActActTaa | 74 | 41 |
| cel-miR-252 | gttAccTgcGgcActActActTa | 75 | 28 |
| cel-miR-253 | ggTcaGtgTtaGtgAggTgtg | 74 | 20 |
| cel-miR-254 | cmCtamCagTcgmCgaAagAttTgca | 76 | 44 |
| cel-miR-256 | tacAgtmCttmCtaTgcAttmCca | 69 | 32 |
| cel-miR-257 | tcActGggTacTccTgaTacTc | 76 | 42 |
| cel-miR-258 | aaaAggAttmCctmCtcAaaAcc | 67 | 45 |
| cel-miR-259 | tacmCagAttAggAtgAgaTtt | 67 | 30 |
| cel-miR-260 | ctamCaaGagTtcGacAtcAc | 70 | 34 |
| cel-miR-261 | cgtGaaAacTaaAaaGcta | 61 | 24 |
| cel-miR-262 | aTcaGaaAacAtcGagAaac | 67 | 25 |
| cel-miR-264 | catAacAacAacmCacmCcgmCc | 77 | 18 |
| cel-miR-265 | atamCcamCccTtcmCtcmCctmCa | 77 | 6 |
| cel-miR-266 | gctTtgmCcaAagTctTgcmCt | 74 | 44 |
| cel-miR-267 | tgcAgcAgamCacTtcAcgGg | 81 | 29 |
| cel-miR-268 | amCcaAacTgcTtcTaaTtcTtgmCc | 74 | 19 |
| cel-miR-269 | aGttTtgmCcaGagTctTgcc | 74 | 49 |
| cel-miR-270 | cTccActGctAcaTcaTgcc | 75 | 27 |
| cel-miR-271 | aaTgcTttmCccAccmCggmCga | 82 | 33 |
| cel-miR-272 | cAaamCacmCcaTgcmCtamCa | 75 | 20 |
| cel-miR-273 | cAgcmCgamCacAgtAcgGgca | 85 | 37 |
| cel-miR-34 | cAacmCagmCtaAccAcamCtgmCct | 80 | 24 |
| cel-miR-35 | amCtgmCtaGttTccAccmCggTga | 80 | 39 |
| cel-miR-353 | aaTacmCaamCacAtgGcaAttg | 70 | 33 |
| cel-miR-354 | aggAgcAgcAacAaamCaaGgt | 79 | 23 |
| cel-miR-355 | catAgcTcaGgcTaaAacAaa | 70 | 45 |
| cel-miR-356 | tgAttTgtTcgmCgtTgcTcaa | 73 | 29 |
| cel-miR-357 | tmCctGcaAcgActGgcAttTa | 77 | 33 |
| cel-miR-358 | ccTtgAcaGggAtamCcaAttg | 72 | 42 |
| cel-miR-359 | tmCgtmCagAgaAagAccAgtGa | 78 | 25 |
| cel-miR-36 | cAtgmCgaAttTtcAccmCggTga | 77 | 44 |
| cel-miR-360 | ttGtgAacGggAttAcgGtca | 75 | 46 |
| cel-miR-37 | amCtgmCaaGtgTtcAccmCggTga | 82 | 46 |
| cel-miR-38 | amCtcmCagTttTtcTccmCggTga | 77 | 28 |
| cel-miR-39 | cAagmCtgAttTacAccmCggTga | 77 | 38 |
| cel-miR-392 | tcAtcAcamCgtGatmCgaTgaTa | 75 | 59 |
| cel-miR-40 | tTagmCtgAtgTacAccmCggTga | 78 | 52 |
| cel-miR-41 | tAggTgaTttTtcAccmCggTga | 76 | 44 |
| cel-miR-42 | cTgtAgaTgtTaamCccGgtg | 76 | 39 |
| cel-miR-43 | gcGacAgcAagTaaActGtgAta | 74 | 32 |
| cel-miR-44 | agcTgaAtgTgtmCtcTagTca | 70 | 30 |
| cel-miR-45 | agcTgaAtgTgtmCtcTagTca | 70 | 30 |
| cel-miR-46 | tgAagAgaGcgActmCcaTgamCa | 79 | 33 |
| cel-miR-47 | tGaaGagAgcGccTccAtgAca | 80 | 38 |
| cel-miR-48 | tcgmCatmCtamCtgAgcmCtamCctmCa | 79 | 31 |
| cel-miR-49 | tcTgcAgcTtcTcgTggTgcTt | 80 | 36 |
| cel-miR-50 | aamCccAagAatAccAgamCatAtca | 73 | 16 |
| cel-miR-51 | aacAtgGatAggAgcTacGggTa | 79 | 31 |
| cel-miR-52 | agmCacGgaAacAtaTgtAcgGgtg | 81 | 44 |
| cel-miR-53 | agmCacGgaAacAaaTgtAcgGgtg | 82 | 33 |
| cel-miR-54 | cTcgGatTatGaaGatTacGggTa | 75 | 35 |
| cel-miR-55 | ctcAgcAgaAacTtaTacGggTa | 74 | 33 |
| cel-miR-56 | ctcAgcGgaAacAttAcgGgta | 77 | 25 |
| cel-miR-56* | tacAacmCcaAaaTggAtcmCgcmCa | 78 | 42 |
| cel-miR-57 | acamCacAgcTcgAtcTacAggGta | 78 | 47 |
| cel-miR-58 | aTtgmCcgTacTgaAcgAtcTca | 75 | 32 |
| cel-miR-59 | cAtcAtcmCtgAtaAacGatTcga | 70 | 35 |
| cel-miR-60 | tgAacTagAaaAtgTgcAtaAta | 65 | 34 |
| cel-miR-61 | gagAtgAgtAacGgtTctAgtmCa | 75 | 52 |
| cel-miR-62 | ctgTaaGctAgaTtamCatAtca | 65 | 60 |
| cel-miR-63 | ttTccAacTcgmCttmCagTgtmCata | 75 | 31 |
| cel-miR-64 | ttcGgtAacGctTcaGtgTcaTa | 76 | 41 |
| cel-miR-65 | ttcGgtTacGctTcaGtgTcaTa | 75 | 41 |
| cel-miR-66 | tmCacAtcmCctAatmCagTgtmCatg | 75 | 27 |
| cel-miR-67 | tctActmCttTctAggAggTtgTga | 73 | 54 |
| cel-miR-68 | tmCtamCacTttTgaGtcTtcGa | 69 | 33 |
| cel-miR-69 | tcTacActTttTaaTttTcga | 59 | 20 |
| cel-miR-70 | atgGaaAcamCcaAcgAcgTatTa | 73 | 33 |
| cel-miR-71 | tcamCtamCccAtgTctTtca | 67 | 20 |
| cel-miR-72 | gmCtaTgcmCaamCatmCttGcct | 76 | 34 |
| cel-miR-73 | actGaamCtgmCctAcaTctTgcmCa | 79 | 28 |
| cel-miR-74 | tgTagActGccAttTctTgcmCa | 76 | 43 |
| cel-miR-75 | tgAagmCcgGttGgtAgcTttAa | 77 | 48 |
| cel-miR-76 | tcaAggmCttmCatmCaamCaamCgaa | 75 | 31 |
| cel-miR-77 | tggAcaGctAtgGccTgaTgaa | 76 | 48 |
| cel-miR-78 | gcamCaaAcaAccAggmCctmCca | 79 | 38 |
| cel-miR-79 | aGctTtgGtaAccTagmCttTat | 67 | 52 |
| cel-miR-80 | tcGgcTttmCaamCtaAtgAtcTca | 72 | 27 |
| cel-miR-81 | acTagmCttTcamCgaTgaTctmCa | 73 | 27 |
| cel-miR-82 | amCtgGctTtcAcgAtgAtcTca | 73 | 30 |
| cel-miR-83 | ttamCtgAatTtaTatGgtGcta | 65 | 33 |
| cel-miR-84 | tamCaaTatTacAtamCtamCctmCa | 66 | 26 |
| cel-miR-85 | gmCacGacTttTcaAatActTtgTa | 70 | 35 |
| cel-miR-86 | gActGtgGcaAagmCatTcamCtta | 73 | 44 |
| cel-miR-87 | amCacmCtgAaamCttTgcTcac | 72 | 20 |
| cel-miR-90 | gGggmCatTcaAacAacAtaTca | 73 | 23 |
| dme-bantam | aaTcaGctTtcAaaAtgAtcTca | 66 | 40 |
| dme-let-7 | amCtaTacAacmCtamCtamCctmCa | 71 | 16 |
| dme-miR-1 | ctcmCatActTctTtamCatTcca | 67 | 11 |
| dme-miR-10 | acaAatTcgGatmCtamCagGgt | 73 | 37 |
| dme-miR-100 | cAcaAgtTcgGatTtamCggGtt | 74 | 48 |
| dme-miR-11 | gcaAgaActmCagActGtgAtg | 71 | 40 |
| dme-miR-12 | accAgtAccTgaTgtAatActmCa | 73 | 33 |
| dme-miR-124 | ctTggmCatTcamCcgmCgtGccTta | 81 | 43 |
| dme-miR-125 | tcamCaaGttAggGtcTcaGgga | 77 | 35 |
| dme-miR-133 | acAgcTggTtgAagGggAccAa | 82 | 41 |
| dme-miR-13a | acTcaTcaAaaTggmCtgTgaTa | 72 | 34 |
| dme-miR-13b | acTcgTcaAaaTggmCtgTgaTa | 74 | 34 |
| dme-miR-14 | tAggAgaGagAaaAagActGa | 71 | 15 |
| dme-miR-184 | gcmCctTatmCagTtcTccGtcmCa | 77 | 23 |
| dme-miR-184* | cGggGcgAgaGaaTgaTaaGg | 83 | 19 |
| dme-miR-210 | tAgcmCgcTgtmCacAcgmCacAa | 84 | 37 |
| dme-miR-219 | cAagAatTgcGttTggAcaAtca | 72 | 35 |
| dme-miR-263a | gtgAatTctTccAgtGccAttAac | 72 | 37 |
| dme-miR-263b | gTgaAttmCtcmCcaGtgmCcaAg | 77 | 34 |
| dme-miR-274 | aTtamCccGttAgtGtcGgtmCacAaaa | 79 | 51 |
| dme-miR-275 | cGcgmCgcTacTtcAggTacmCtga | 82 | 64 |
| dme-miR-276a | agAgcAcgGtaTgaAgtTccTa | 75 | 33 |
| dme-miR-276a* | cgtAggAacTctAtamCctmCgcTg | 76 | 30 |
| dme-miR-276b | agAgcAcgGtaTtaAgtTccTa | 71 | 40 |
| dme-miR-276b* | cgtAggAacTctAtamCctmCgcTg | 76 | 30 |
| dme-miR-277 | tgTcgTacmCagAtaGtgmCatTta | 72 | 38 |
| dme-miR-278 | aaAcgGacGaaAgtmCccAccGa | 80 | 41 |
| dme-miR-279 | tTaaTgaGtgTggAtcTagTca | 70 | 40 |
| dme-miR-280 | tAtcAttTcaTatGcaAcgTaaAtamCa | 70 | 40 |
| dme-miR-281 | acAaaGagAgcAatTccAtgAca | 74 | 26 |
| dme-miR-281-1* | actGtcGacGgamCagmCtcTctt | 80 | 56 |
| dme-miR-281-2* | actGtcGacGgaTagmCtcTctt | 77 | 56 |
| dme-miR-282 | amCagAcaAagmCctAgtAgaGgcTagAtt | 80 | 49 |
| dme-miR-283 | aGaaTtamCcaGctGatAttTa | 67 | 54 |
| dme-miR-284 | caAttGctGgaAtcAagTtgmCtgActTca | 78 | 45 |
| dme-miR-285 | gcamCtgAttTcgAatGgtGcta | 74 | 55 |
| dme-miR-286 | agcAcgAgtGttmCggTctAgtmCa | 80 | 46 |
| dme-miR-287 | gtgmCaaAcgAttTtcAacAca | 68 | 27 |
| dme-miR-288 | caTgaAatGaaAtcGacAtgAaa | 68 | 27 |
| dme-miR-289 | agtmCgcAggmCtcmCacTtaAatAttTa | 74 | 42 |
| dme-miR-2a | gcTcaTcaAagmCtgGctGtgAta | 75 | 68 |
| dme-miR-2b | gcTccTcaAagmCtgGctGtgAta | 76 | 62 |
| dme-miR-2c | gcmCcaTcaAagmCtgGctGtgAta | 78 | 68 |
| dme-miR-3 | tgaGacAcamCttTgcmCcaGtga | 77 | 45 |
| dme-miR-303 | accAgtTtcmCtgTgaAacmCtaAa | 72 | 45 |
| dme-miR-304 | ctcAcaTttAcaAatTgaGatTa | 64 | 55 |
| dme-miR-305 | cagAgcAccTgaTgaAgtAcaAt | 74 | 31 |
| dme-miR-306 | tTgaGagTcamCtaAgtAccTga | 72 | 42 |
| dme-miR-306* | gmCacAggmCacAgaGtgAccmCcc | 86 | 37 |
| dme-miR-307 | ctmCacTcaAggAggTtgTga | 74 | 33 |
| dme-miR-308 | cTcamCagTatAatmCctGtgAtt | 69 | 64 |
| dme-miR-309 | tAggAcaAacTttAccmCagTgc | 74 | 37 |
| dme-miR-310 | aAagGccGggAagTgtGcaAta | 79 | 28 |
| dme-miR-311 | tmCagGccGgtGaaTgtGcaAta | 81 | 36 |
| dme-miR-312 | tmCagGccGtcTcaAgtGcaAta | 77 | 39 |
| dme-miR-313 | tcgGgcTgtGaaAagTgcAata | 77 | 29 |
| dme-miR-314 | cmCgaActTatTggmCtcGaaTa | 72 | 30 |
| dme-miR-315 | gmCttTctGagmCaamCaaTcaAaa | 72 | 37 |
| dme-miR-316 | cgcmCagTaaGcgGaaAaaGaca | 76 | 35 |
| dme-miR-317 | amCtgGatAccAccAgcTgtGttmCa | 82 | 47 |
| dme-miR-318 | tgaGatAaamCaaAgcmCcaGtga | 73 | 25 |
| dme-miR-31a | tcaGctAtgmCcgAcaTctTgcmCa | 80 | 45 |
| dme-miR-31b | cagmCtaTtcmCgamCatmCttGcca | 75 | 31 |
| dme-miR-33 | cAatGcgActAcaAtgmCacmCt | 75 | 26 |
| dme-miR-34 | cAacmCagmCtaAccAcamCtgmCca | 80 | 24 |
| dme-miR-4 | tcAatGgtTgtmCtaGctTtat | 67 | 34 |
| dme-miR-5 | catAtcAcaAcgAtcGttmCctTt | 69 | 54 |
| dme-miR-6 | aaaAagAacAgcmCacTgtGata | 71 | 36 |
| dme-miR-7 | amCaamCaaAatmCacTagTctTcca | 71 | 30 |
| dme-miR-79 | atgmCttTggTaaTctAgcTtta | 66 | 34 |
| dme-miR-8 | gamCatmCttTacmCtgAcaGtaTta | 67 | 36 |
| dme-miR-87 | camCacmCtgAaaTttTgcTcaa | 69 | 32 |
| dme-miR-92a | aTagGccGggAcaAgtGcaAtg | 80 | 28 |
| dme-miR-92b | gmCagGccGggActAgtGcaAtt | 83 | 36 |
| dme-miR-9a | tcAtamCagmCtaGatAacmCaaAga | 71 | 34 |
| dme-miR-9b | catAcaGctAaaAtcAccAaaGa | 69 | 24 |
| dme-miR-9c | tctAcaGctAgaAtamCcaAaga | 68 | 27 |
| dme-miR-iab-4-3p | gttAcgTatActGaaGgtAtamCcg | 73 | 59 |
| dme-miR-iab-4-5p | tmCagGatAcaTtcAgtAtamCgt | 72 | 34 |
| dps-bantam | aaTcaGctTtcAaaAtgAtcTca | 66 | 40 |
| dps-let-7 | amCtaTacAacmCtamCtamCctmCa | 71 | 16 |
| dps-miR-1 | ctcmCatActTctTtamCatTcca | 67 | 11 |
| dps-miR-10 | acaAatTcgGatmCtamCagGgt | 73 | 37 |
| dps-miR-100 | cacAagTtcGgaAttAcgGgtt | 74 | 50 |
| dps-miR-11 | gcaAgaActmCagActGtgAtg | 71 | 40 |
| dps-miR-12 | accAgtAccTgaTgtAatActmCa | 73 | 33 |
| dps-miR-124 | ctTggmCatTcamCcgmCgtGccTta | 81 | 43 |
| dps-miR-125 | tcamCaaGttAggGtcTcaGgga | 77 | 35 |
| dps-miR-133 | acAgcTggTtgAagGggAccAa | 82 | 41 |
| dps-miR-13a | acTcaTcaAaaTggmCtgTgaTa | 72 | 34 |
| dps-miR-13b | acTcgTcaAaaTggmCtgTgaTa | 74 | 34 |
| dps-miR-14 | tAggAgaGagAaaAagActGa | 71 | 15 |
| dps-miR-184 | gcmCctTatmCagTtcTccGtcmCa | 77 | 23 |
| dps-miR-210 | tAgcmCgcTgtmCacAcgmCacAa | 84 | 37 |
| dps-miR-219 | cAagAatTgcGttTggAcaAtca | 72 | 35 |
| dps-miR-263a | gtgAatTctTccAgtGccAttAac | 72 | 37 |
| dps-miR-263b | gTgaAttmCtcmCcaGtgmCcaAg | 77 | 34 |
| dps-miR-274 | aTtamCccGttAgtGtcGgtmCacAaaa | 79 | 51 |
| dps-miR-275 | cGcgmCgcTacTtcAggTacmCtga | 82 | 64 |
| dps-miR-276a | agAgcAcgGtaTgaAgtTccTa | 75 | 33 |
| dps-miR-276b | agAgcAcgGtaTtaAgtTccTa | 71 | 40 |
| dps-miR-277 | tgTcgTacmCagAtaGtgmCatTta | 72 | 38 |
| dps-miR-278 | aAacGgamCgaAagTccmCtcmCga | 81 | 53 |
| dps-miR-279 | tTaaTgaGtgTggAtcTagTca | 70 | 40 |
| dps-miR-280 | tAtcAttTcaTatGcaAcgTaaAtamCa | 70 | 40 |
| dps-miR-281 | acAaaGagAgcAatTccAtgAca | 74 | 26 |
| dps-miR-282 | amCagAcaAagmCctAgtAgaGgcTagAtt | 80 | 49 |
| dps-miR-283 | aGaaTtamCcaGctGatAttTa | 67 | 54 |
| dps-miR-284 | caAttGctGgaAtcAagTtgmCtgActTca | 78 | 45 |
| dps-miR-285 | gcamCtgAttTcgAatGgtGcta | 74 | 55 |
| dps-miR-286 | agcAcgAgtGttmCggTctAgtmCa | 80 | 46 |
| dps-miR-287 | gtgmCaaAcgAttTtcAacAca | 68 | 27 |
| dps-miR-288 | caTgaAatGaaAtcGacAtgAaa | 68 | 27 |
| dps-miR-289 | agtmCgcAggmCtcmCacTtaAatAttTa | 74 | 42 |
| dps-miR-2a | gcTcaTcaAagmCtgGctGtgAta | 75 | 68 |
| dps-miR-2b | gcTccTcaAagmCtgGctGtgAta | 76 | 62 |
| dps-miR-2c | gcmCcaTcaAagmCtgGctGtgAta | 78 | 68 |
| dps-miR-3 | tgaGacAcamCttTgcmCcaGtga | 77 | 45 |
| dps-miR-304 | ctcAcaTttAcaAatTgaGatTa | 64 | 55 |
| dps-miR-305 | cagAgcAccTgaTgaAgtAcaAt | 74 | 31 |
| dps-miR-306 | tTgaGagTcamCtaAgtAccTga | 72 | 42 |
| dps-miR-307 | ctmCacTcaAggAggTtgTga | 74 | 33 |
| dps-miR-308 | cTcamCagTatAatmCctGtgAtt | 69 | 64 |
| dps-miR-309 | tAagAcaAacTtcAccmCagTgc | 74 | 29 |
| dps-miR-314 | cmCgaActTatTggmCtcGaaTa | 72 | 30 |
| dps-miR-315 | gmCttTctGagmCaamCaaTcaAaa | 72 | 37 |
| dps-miR-316 | cgcmCagTaaGcgGaaAaaGaca | 76 | 35 |
| dps-miR-317 | aTtgGatAccAccAgcTgtGttmCa | 79 | 47 |
| dps-miR-318 | tgaGatAaamCaaAgcmCcaGtga | 73 | 25 |
| dps-miR-31a | tcaGctAtgmCcgAcaTctTgcmCa | 80 | 45 |
| dps-miR-31b | tcaGctAttmCcgAcaTctTgcmCa | 77 | 31 |
| dps-miR-33 | cAatGcgActAcaAtgmCacmCt | 75 | 26 |
| dps-miR-34 | cAacmCagmCtaAccAcamCtgmCca | 80 | 24 |
| dps-miR-4 | tcAatGgtTgtmCtaGctTtat | 67 | 34 |
| dps-miR-5 | catAtcAcaAcgAtcGttmCctTt | 69 | 54 |
| dps-miR-6 | aaaAagAacAgcmCacTgtGata | 71 | 36 |
| dps-miR-7 | amCaamCaaAatmCacTagTctTcca | 71 | 30 |
| dps-miR-79 | atgmCttTggTaaTctAgcTtta | 66 | 34 |
| dps-miR-8 | gamCatmCttTacmCtgAcaGtaTta | 67 | 36 |
| dps-miR-87 | camCacmCtgAaaTttTgcTcaa | 69 | 32 |
| dps-miR-92a | aTagGccGggAcaAgtGcaAtg | 80 | 28 |
| dps-miR-92b | gmCagGccGggActAgtGcaAtt | 83 | 36 |
| dps-miR-9a | tcAtamCagmCtaGatAacmCaaAga | 71 | 34 |
| dps-miR-9b | catAcaGctAaaAtcAccAaaGa | 69 | 24 |
| dps-miR-9c | tctAcaGctAgaAtamCcaAaga | 68 | 27 |
| dps-miR-iab-4-3p | gttAcgTatActGaaGgtAtamCcg | 73 | 59 |
| dps-miR-iab-4-5p | tmCagGatAcaTtcAgtAtamCgt | 72 | 34 |
| dre-miR-10a | cAcaAatTcgGatmCtamCagGgta | 74 | 37 |
| dre-miR-10b | amCaaAttmCggTtcTacAggGta | 73 | 35 |
| dre-miR-181b | cccAccGacAgcAatGaaTgtt | 78 | 30 |
| dre-miR-182 | tgtGagTtcTacmCatTgcmCaaa | 72 | 32 |
| dre-miR-182* | taGttGgcAagTctAgaAcca | 72 | 32 |
| dre-miR-183 | caGtgAatTctAccAgtGccAta | 73 | 32 |
| dre-miR-187 | ggcTgcAacAcaAgamCacGa | 79 | 30 |
| dre-miR-192 | ggcTgtmCaaTtcAtaGgtmCat | 72 | 46 |
| dre-miR-196a | ccaAcaAcaTgaAacTacmCta | 67 | 20 |
| dre-miR-199a | gaAcaGgtAgtmCtgAacActGgg | 78 | 40 |
| dre-miR-203 | cAagTggTccTaaAcaTttmCac | 70 | 31 |
| dre-miR-204 | aggmCatAggAtgAcaAagGgaa | 75 | 25 |
| dre-miR-205 | caGacTccGgtGgaAtgAagGa | 81 | 39 |
| dre-miR-210 | ttAgcmCgcTgtmCacAcgmCacAg | 85 | 37 |
| dre-miR-213 | gGtamCaaTcaAcgGtcAatGgt | 75 | 43 |
| dre-miR-214 | ctGccTgtmCtgTgcmCtgmCtgt | 81 | 30 |
| dre-miR-216 | camCagTtgmCcaGctGagAtta | 74 | 64 |
| dre-miR-217 | atcmCaaTcaGttmCctGatGcaGta | 75 | 49 |
| dre-miR-219 | agAatTgcGttTggAcaAtca | 70 | 35 |
| dre-miR-220 | aAgtGtcmCgaTacGgtTgtGg | 81 | 47 |
| dre-miR-221 | gAaamCccAgcAgamCaaTgtAgct | 80 | 31 |
| dre-miR-222 | gaGacmCcaGtaGccAgaTgtAgct | 80 | 38 |
| dre-miR-223 | gGggTatTtgAcaAacTgamCa | 73 | 40 |
| dre-miR-34a | aamCaamCcaGctAagAcamCtgmCca | 80 | 27 |
| dre-miR-7 | caamCaaAatmCacTagTctTcca | 69 | 30 |
| dre-miR-7b | aamCaaAatmCacAagTctTcca | 68 | 24 |
| ebv-miR-B | aGcamCgtmCacTtcmCacTaaGa | 77 | 25 |
| ebv-miR-B | gcAagGgcGaaTgcAgaAaaTa | 78 | 27 |
| ebv-miR-BHRF1-1 | aacTccGggGctGatmCagGtta | 80 | 50 |
| ebv-miR-BHRF1-2 | tTcaAttTctGccGcaAaaGata | 70 | 52 |
| ebv-miR-BHRF1-2* | gctAtcTgcTgcAacAgaAttt | 71 | 62 |
| ebv-miR-BHRF1-3 | gtGtgmCttAcamCacTtcmCcgTta | 76 | 47 |
| gga-let-7a | aamCtaTacAacmCtamCtamCctmCa | 70 | 16 |
| gga-let-7b | aamCcamCacAacmCtamCtamCctmCa | 77 | 6 |
| gga-let-7c | aamCcaTacAacmCtamCtamCctmCa | 74 | 11 |
| gga-let-7d | actAtgmCaamCccActAccTct | 74 | 24 |
| gga-let-7f | aamCtaTacAatmCtamCtamCctmCa | 67 | 16 |
| gga-let-7g | amCtgTacAaamCtamCtamCctmCa | 71 | 30 |
| gga-let-7i | amCagmCacAaamCtamCtamCctmCa | 76 | 18 |
| gga-let-7j | aamCtaTacAacmCtamCtamCctmCa | 70 | 16 |
| gga-let-7k | aActAttmCaaTctActAccTca | 67 | 22 |
| gga-mi R-1 | tamCatActTctTtamCatTcca | 64 | 11 |
| gga-miR-100 | cacAagTtcGgaTctAcgGgtt | 77 | 38 |
| gga-miR-101 | cttmCagTtaTcamCagTacTgta | 68 | 54 |
| gga-miR-103 | tmCatAgcmCctGtamCaaTgcTgct | 80 | 63 |
| gga-miR-106 | tacmCtgmCacTgtAagmCacTttt | 72 | 37 |
| gga-miR-107 | tGatAgcmCctGtamCaaTgcTgct | 80 | 63 |
| gga-miR-10b | amCaaAttmCggTtcTacAggGta | 73 | 35 |
| gga-miR-122a | acAaamCacmCatTgtmCacActmCca | 78 | 25 |
| gga-miR-124a | tggmCatTcamCcgmCgtGccTtaa | 80 | 43 |
| gga-miR-124b | tggmCatTcamCtgmCgtGccTtaa | 77 | 48 |
| gga-miR-125b | tcamCaaGttAggGtcTcaGgga | 77 | 35 |
| gga-miR-126 | gcAttAttActmCacGgtAcga | 71 | 25 |
| gga-miR-128a | aaAagAgamCcgGttmCacTgtGa | 77 | 47 |
| gga-miR-128b | aaAagAgamCcgGttmCacTgtGa | 77 | 47 |
| gga-miR-130a | atgmCccTttTaaTatTgcActg | 68 | 42 |
| gga-miR-130b | acGccmCttTcaTtaTtgmCacTg | 75 | 26 |
| gga-miR-133a | acAgcTggTtgAagGggAccAa | 82 | 41 |
| gga-miR-133b | taGctGgtTgaAggGgamCcaa | 81 | 37 |
| gga-miR-133c | gcAgcTggTtgAagGggAccAa | 83 | 41 |
| gga-miR-135a | tcamCatAggAatAaaAagmCcaTa | 69 | 22 |
| gga-miR-137 | cTacGcgTatTctTaaGcaAta | 68 | 48 |
| gga-miR-138 | gatTcamCaamCacmCagmCt | 70 | 24 |
| gga-miR-140 | ctAccAtaGggTaaAacmCact | 71 | 43 |
| gga-miR-142-3p | tmCcaTaaAgtAggAaamCacTaca | 72 | 29 |
| gga-miR-142-5p | gtaGtgmCttTctActTtaTg | 63 | 36 |
| gga-miR-146 | aAccmCatGgaAttmCagTtcTca | 73 | 44 |
| gga-miR-148a | acaAagTtcTgtAgtGcamCtga | 72 | 54 |
| gga-miR-153 | tcamCttTtgTgamCtaTgcAa | 68 | 35 |
| gga-miR-155 | ccmCctAtcAcgAttAgcAttAa | 71 | 29 |
| gga-miR-15a | cAcaAacmCatTatGtgmCtgmCta | 73 | 35 |
| gga-miR-15b | tgcAaamCcaTgaTgtGctGcta | 77 | 52 |
| gga-miR-16 | cacmCaaTatTtamCgtGctGcta | 71 | 38 |
| gga-miR-17-3p | acAagTgcmCttmCacTgcAgt | 77 | 47 |
| gga-miR-17-5p | actAccTgcActGtaAgcActTtg | 74 | 39 |
| gga-miR-181a | acTcamCcgAcaGcgTtgAatGtt | 77 | 49 |
| gga-miR-181b | cccAccGacAgcAatGaaTgtt | 78 | 30 |
| gga-miR-183 | caGtgAatTctAccAgtGccAta | 73 | 32 |
| gga-miR-184 | acmCctTatmCagTtcTccGtcmCa | 76 | 23 |
| gga-miR-187 | ggcTgcAacAcaAgamCacGa | 79 | 30 |
| gga-miR-18a | tatmCtgmCacTagAtgmCacmCtta | 71 | 40 |
| gga-miR-18b | taamCtgmCacTagAtgmCacmCtta | 72 | 40 |
| gga-miR-190 | acmCtaAtaTatmCaaAcaTatmCa | 62 | 31 |
| gga-miR-194 | tccAcaTggAgtTgcTgtTaca | 75 | 41 |
| gga-miR-196 | ccaAcaAcaTgaAacTacmCta | 67 | 20 |
| gga-miR-199a | gaAcaGgtAgtmCtgAacActGgg | 78 | 40 |
| gga-miR-19a | tmCagTttTgcAtaGatTtgmCaca | 72 | 37 |
| gga-miR-19b | tmCagTttTgcAtgGatTtgmCaca | 75 | 34 |
| gga-miR-ib | tacAtamCttmCttAacAttmCca | 64 | 16 |
| gga-miR-20 | ctAccTgcActAtaAgcActTta | 70 | 26 |
| gga-miR-200a | acaTcgTtamCcaGacAgtGtta | 72 | 39 |
| gga-miR-200b | atcAtcAttAccAggmCagTatTa | 70 | 29 |
| gga-miR-203 | cAagTggTccTaaAcaTttmCac | 70 | 31 |
| gga-miR-204 | aggmCatAggAtgAcaAagGgaa | 75 | 25 |
| gga-miR-205a | caGacTccGgtGgaAtgAagGa | 81 | 39 |
| gga-miR-205b | cAgaTtcmCggTggAatGaaGgg | 80 | 55 |
| gga-miR-206 | ccamCacActTccTtamCatTcca | 73 | 11 |
| gga-miR-213 | gGtamCaaTcaAcgGtcGatGgt | 79 | 67 |
| gga-miR-215 | gtcTgtmCaaTtcAtaGgtmCat | 70 | 50 |
| gga-miR-216 | camCagTtgmCcaGctGagAtta | 74 | 64 |
| gga-miR-217 | atcmCaaTcaGttmCctGatGcaGta | 75 | 49 |
| gga-miR-218 | acAtgGttAgaTcaAgcAcaa | 70 | 40 |
| gga-miR-219 | agAatTgcGttTggAcaAtca | 70 | 35 |
| gga-miR-221 | gAaamCccAgcAgamCaaTgtAgct | 80 | 31 |
| gga-miR-222a | gaGacmCcaGtaGccAgaTgtAgct | 80 | 38 |
| gga-miR-222b | gAgamCccAgtAgcmCagAtgTagTt | 80 | 28 |
| gga-miR-223 | gGggTatTtgAcaAacTgamCa | 73 | 40 |
| gga-miR-23b | ggtAatmCccTggmCaaTgtGat | 76 | 38 |
| gga-miR-24 | cTgtTccTgcTgaActGagmCca | 80 | 35 |
| gga-miR-26a | gcmCtaTccTggAttActTgaa | 70 | 34 |
| gga-miR-27b | gcAgaActTagmCcamCtgTgaa | 74 | 38 |
| gga-miR-29a | aamCcgAttTcaAatGgtGcta | 71 | 47 |
| gga-miR-29b | aamCacTgaTttmCaaAtgGtgmCta | 71 | 47 |
| gga-miR-29c | amCcgAttTcaAatGgtGcta | 71 | 47 |
| gga-miR-301 | atGctTtgAcaAtaTtaTtgmCacTg | 70 | 45 |
| gga-miR-302 | tcActAaaAcaTggAagmCacTt | 71 | 23 |
| gga-miR-30a-3p | gctGcaAacAtcmCgamCtgAaag | 74 | 28 |
| gga-miR-30a-5p | cTtcmCagTcgAggAtgTttAca | 73 | 31 |
| gga-miR-30b | agcTgaGtgTagGatGttTaca | 71 | 33 |
| gga-miR-30c | gmCtgAgaGtgTagGatGttTaca | 73 | 33 |
| gga-miR-30d | cttmCcaGtcGggGatGttTaca | 76 | 44 |
| gga-miR-30e | tcmCagTcaAggAtgTttAca | 69 | 30 |
| gga-miR-31 | cagmCtaTgcmCaamCatmCttGcct | 77 | 34 |
| gga-miR-32 | gcaActTagTaaTgtGcaAta | 65 | 43 |
| gga-miR-33 | cAatGcaActAcaAtgmCac | 68 | 30 |
| gga-miR-34a | aamCaamCcaGctAagAcamCtgmCca | 80 | 27 |
| gga-miR-34b | caAtcAgcTaamCtamCacTgcmCtg | 75 | 32 |
| gga-miR-34c | gcAatmCagmCtaActAcamCtgmCct | 76 | 31 |
| gga-miR-7 | caamCaaAatmCacTagTctTcca | 69 | 30 |
| gga-miR-7b | aacAaaAatmCacTagTctTcca | 66 | 30 |
| gga-miR-9 | tcAtamCagmCtaGatAacmCaaAga | 71 | 34 |
| gga-miR-92 | cagGccGggAcaAgtGcaAta | 79 | 28 |
| gga-miR-99a | cacAagAtcGgaTctAcgGgtt | 77 | 42 |
| hsa-let-7a | aamCtaTacAacmCtamCtamCctmCa | 70 | 16 |
| hsa-let-7b | aamCcamCacAacmCtamCtamCctmCa | 77 | 6 |
| hsa-let-7c | aamCcaTacAacmCtamCtamCctmCa | 74 | 11 |
| hsa-let-7d | actAtgmCaamCctActAccTct | 71 | 24 |
| h sa-l et-7e | actAtamCaamCctmCctAccTca | 71 | 16 |
| hsa-let-7f | aamCtaTacAatmCtamCtamCctmCa | 67 | 16 |
| hsa-let-7g | amCtgTacAaamCtamCtamCctmCa | 71 | 30 |
| hsa-let-7i | amCagmCacAaamCtamCtamCctmCa | 76 | 18 |
| hsa-miR-1 | tamCatActTctTtamCatTcca | 64 | 11 |
| hsa-miR-100 | cacAagTtcGgaTctAcgGgtt | 77 | 38 |
| hsa-miR-101 | cttmCagTtaTcamCagTacTgta | 68 | 54 |
| hsa-miR-103 | tmCatAgcmCctGtamCaaTgcTgct | 80 | 63 |
| hsa-miR-105 | acAggAgtmCtgAgcAttTga | 73 | 33 |
| hsa-miR-106a | gctAccTgcActGtaAgcActTtt | 75 | 37 |
| hsa-miR-106b | atcTgcActGtcAgcActTta | 72 | 35 |
| hsa-miR-107 | tGatAgcmCctGtamCaaTgcTgct | 80 | 63 |
| hsa-miR-108 | aatGccmCctAaaAatmCctTat | 66 | 23 |
| hsa-miR-10a | cAcaAatTcgGatmCtamCagGgta | 74 | 37 |
| hsa-miR-10b | amCaaAttmCggTtcTacAggGta | 73 | 35 |
| hsa-miR-122a | acAaamCacmCatTgtmCacActmCca | 78 | 25 |
| hsa-miR-124a | tggmCatTcamCcgmCgtGccTtaa | 80 | 43 |
| hsa-miR-125a | cAcaGgtTaaAggGtcTcaGgga | 79 | 35 |
| hsa-miR-125b | tcamCaaGttAggGtcTcaGgga | 77 | 35 |
| hsa-miR-126 | gcAttAttActmCacGgtAcga | 71 | 25 |
| hsa-miR-126* | cgmCgtAccAaaAgtAatAatg | 68 | 28 |
| hsa-miR-127 | agcmCaaGctmCagAcgGatmCcga | 81 | 54 |
| hsa-miR-128a | aaAagAgamCcgGttmCacTgtGa | 77 | 47 |
| hsa-miR-128b | gaAagAgamCcgGttmCacTgtGa | 78 | 47 |
| hsa-miR-129 | gcAagmCccAgamCcgmCaaAaag | 80 | 21 |
| hsa-miR-130a | aTgcmCctTttAacAttGcamCtg | 74 | 42 |
| hsa-miR-130b | aTgcmCctTtcAtcAttGcamCtg | 75 | 34 |
| hsa-miR-132 | cgAccAtgGctGtaGacTgtTa | 76 | 48 |
| hsa-miR-133a | acAgcTggTtgAagGggAccAa | 82 | 41 |
| hsa-miR-133b | taGctGgtTgaAggGgamCcaa | 81 | 37 |
| hsa-miR-134 | ccmCtcTggTcaAccAgtmCaca | 77 | 57 |
| hsa-miR-135a | tcamCatAggAatAaaAagmCcaTa | 69 | 22 |
| hsa-miR-135b | cacAtaGgaAtgAaaAgcmCata | 70 | 22 |
| hsa-miR-136 | tccAtcAtcAaaAcaAatGgaGt | 71 | 25 |
| hsa-miR-137 | cTacGcgTatTctTaaGcaAta | 68 | 48 |
| hsa-miR-138 | gatTcamCaamCacmCagmCt | 70 | 24 |
| hsa-miR-139 | aGacAcgTgcActGtaGa | 75 | 42 |
| hsa-miR-140 | ctAccAtaGggTaaAacmCact | 71 | 43 |
| hsa-miR-141 | cmCatmCttTacmCagAcaGtgTta | 70 | 33 |
| hsa-miR-142-3p | tmCcaTaaAgtAggAaamCacTaca | 72 | 29 |
| hsa-miR-142-5p | gtaGtgmCttTctActTtaTg | 63 | 36 |
| hsa-miR-143 | tGagmCtamCagTgcTtcAtcTca | 75 | 56 |
| hsa-miR-144 | ctaGtamCatmCatmCtaTacTgta | 64 | 37 |
| hsa-miR-145 | aAggGatTccTggGaaAacTggAc | 79 | 50 |
| hsa-miR-146 | aAccmCatGgaAttmCagTtcTca | 73 | 44 |
| hsa-miR-147 | gcAgaAgcAttTccAcamCac | 74 | 25 |
| hsa-miR-148a | acaAagTtcTgtAgtGcamCtga | 72 | 54 |
| hsa-miR-148b | acaAagTtcTgtGatGcamCtga | 72 | 39 |
| hsa-miR-149 | ggaGtgAagAcamCggAgcmCaga | 80 | 31 |
| hsa-miR-150 | cacTggTacAagGgtTggGaga | 78 | 30 |
| hsa-miR-151 | ccTcaAggAgcTtcAgtmCtaGt | 75 | 45 |
| hsa-miR-152 | cmCcaAgtTctGtcAtgmCacTga | 78 | 36 |
| hsa-miR-153 | tcamCttTtgTgamCtaTgcAa | 68 | 35 |
| hsa-miR-154 | cGaaGgcAacAcgGatAacmCta | 78 | 40 |
| hsa-miR-154* | aAtaGgtmCaamCcgTgtAtgAtt | 74 | 40 |
| hsa-miR-155 | ccmCctAtcAcgAttAgcAttAa | 71 | 29 |
| hsa-miR-15a | cAcaAacmCatTatGtgmCtgmCta | 73 | 35 |
| hsa-miR-15b | tgtAaamCcaTgaTgtGctGcta | 74 | 38 |
| hsa-miR-16 | cgmCcaAtaTttAcgTgcTgcTa | 74 | 34 |
| hsa-miR-17-3p | acAagTgcmCttmCacTgcAgt | 77 | 47 |
| hsa-miR-17-5p | actAccTgcActGtaAgcActTtg | 74 | 39 |
| hsa-miR-18 | tatmCtgmCacTagAtgmCacmCtta | 71 | 40 |
| hsa-miR-181a | acTcamCcgAcaGcgTtgAatGtt | 77 | 49 |
| hsa-miR-181b | cccAccGacAgcAatGaaTgtt | 78 | 30 |
| hsa-miR-181c | amCtcAccGacAggTtgAatGtt | 76 | 33 |
| hsa-miR-182 | tgtGagTtcTacmCatTgcmCaaa | 72 | 32 |
| hsa-miR-182* | taGttGgcAagTctAgaAcca | 72 | 32 |
| hsa-miR-183 | caGtgAatTctAccAgtGccAta | 73 | 32 |
| hsa-miR-184 | acmCctTatmCagTtcTccGtcmCa | 76 | 23 |
| hsa-miR-185 | gAacTgcmCttTctmCtcmCa | 70 | 27 |
| hsa-miR-186 | aaGccmCaaAagGagAatTctTtg | 71 | 48 |
| hsa-miR-187 | cGgcTgcAacAcaAgamCacGa | 84 | 31 |
| hsa-miR-188 | amCccTccAccAtgmCaaGggAtg | 83 | 42 |
| hsa-miR-189 | actGatAtcAgcTcaGtaGgcAc | 77 | 54 |
| hsa-miR-190 | acmCtaAtaTatmCaaAcaTatmCa | 62 | 31 |
| hsa-miR-191 | agcTgcTttTggGatTccGttg | 74 | 42 |
| hsa-miR-192 | gGctGtcAatTcaTagGtcAg | 73 | 46 |
| hsa-miR-193 | ctGggActTtgTagGccAgtt | 76 | 31 |
| hsa-miR-194 | tccAcaTggAgtTgcTgtTaca | 75 | 41 |
| hsa-miR-195 | gmCcaAtaTttmCtgTgcTgcTa | 73 | 28 |
| hsa-miR-196a | ccaAcaAcaTgaAacTacmCta | 67 | 20 |
| hsa-miR-196b | cmCaamCaamCagGaaActAccTa | 73 | 27 |
| hsa-miR-197 | gctGggTggAgaAggTggTgaa | 84 | 19 |
| hsa-miR-198 | cmCtaTctmCccmCtcTggAcc | 75 | 25 |
| hsa-miR-199a | gaAcaGgtAgtmCtgAacActGgg | 78 | 40 |
| hsa-miR-199a* | aacmCaaTgtGcaGacTacTgta | 74 | 39 |
| hsa-miR-199b | gAacAgaTagTctAaamCacTggg | 73 | 32 |
| hsa-miR-19a | tmCagTttTgcAtaGatTtgmCaca | 72 | 37 |
| hsa-miR-19b | tmCagTttTgcAtgGatTtgmCaca | 75 | 34 |
| hsa-miR-20 | ctAccTgcActAtaAgcActTta | 70 | 26 |
| hsa-miR-200a | acaTcgTtamCcaGacAgtGtta | 72 | 39 |
| hsa-miR-200b | gtcAtcAttAccAggmCagTatTa | 71 | 31 |
| hsa-miR-200c | ccAtcAttAccmCggmCagTatTa | 74 | 38 |
| hsa-miR-203 | cTagTggTccTaaAcaTttmCac | 69 | 23 |
| hsa-miR-204 | aggmCatAggAtgAcaAagGgaa | 75 | 25 |
| hsa-miR-205 | caGacTccGgtGgaAtgAagGa | 81 | 39 |
| hsa-miR-206 | ccamCacActTccTtamCatTcca | 73 | 11 |
| hsa-miR-208 | acAagmCttTttGctmCgtmCttAt | 71 | 34 |
| hsa-miR-21 | tmCaamCatmCagTctGatAagmCta | 72 | 48 |
| hsa-miR-210 | tcAgcmCgcTgtmCacAcgmCacAg | 87 | 38 |
| hsa-miR-211 | aggmCgaAggAtgAcaAagGgaa | 77 | 18 |
| hsa-miR-212 | gGccGtgActGgaGacTgtTa | 81 | 37 |
| hsa-miR-213 | gGtamCaaTcaAcgGtcGatGgt | 79 | 67 |
| hsa-miR-214 | ctGccTgtmCtgTgcmCtgmCtgt | 81 | 30 |
| hsa-miR-215 | gtcTgtmCaaTtcAtaGgtmCat | 70 | 50 |
| hsa-miR-216 | camCagTtgmCcaGctGagAtta | 74 | 64 |
| hsa-miR-217 | atcmCaaTcaGttmCctGatGcaGta | 75 | 49 |
| hsa-miR-218 | acAtgGttAgaTcaAgcAcaa | 70 | 40 |
| hsa-miR-219 | agAatTgcGttTggAcaAtca | 70 | 35 |
| hsa-miR-22 | acaGttmCttmCaamCtgGcaGctt | 74 | 48 |
| hsa-miR-220 | aaAgtGtcAgaTacGgtGtgg | 75 | 32 |
| hsa-miR-221 | gAaamCccAgcAgamCaaTgtAgct | 80 | 31 |
| hsa-miR-222 | gaGacmCcaGtaGccAgaTgtAgct | 80 | 38 |
| hsa-miR-223 | gGggTatTtgAcaAacTgamCa | 73 | 40 |
| hsa-miR-224 | tAaamCggAacmCacTagTgamCttg | 75 | 49 |
| hsa-miR-23a | gGaaAtcmCctGgcAatGtgAt | 76 | 37 |
| hsa-miR-23b | ggtAatmCccTggmCaaTgtGat | 76 | 38 |
| hsa-miR-24 | cTgtTccTgcTgaActGagmCca | 80 | 35 |
| hsa-miR-25 | tcaGacmCgaGacAagTgcAatg | 77 | 27 |
| hsa-miR-26a | gcmCtaTccTggAttActTgaa | 70 | 34 |
| hsa-miR-26b | aacmCtaTccTgaAttActTgaa | 65 | 28 |
| hsa-miR-27a | gcGgaActTagmCcamCtgTgaa | 77 | 35 |
| hsa-miR-27b | gcAgaActTagmCcamCtgTgaa | 74 | 38 |
| hsa-miR-28 | ctmCaaTagActGtgAgcTccTt | 73 | 43 |
| hsa-miR-296 | acAggAttGagGggGggmCcct | 88 | 48 |
| hsa-miR-299 | aTgtAtgTggGacGgtAaamCca | 80 | 35 |
| hsa-miR-29a | aamCcgAttTcaGatGgtGcta | 75 | 43 |
| hsa-miR-29b | aamCacTgaTttmCaaAtgGtgmCta | 71 | 47 |
| hsa-miR-29c | amCcgAttTcaAatGgtGcta | 71 | 47 |
| hsa-miR-301 | gctTtgAcaAtamCtaTtgmCacTg | 70 | 36 |
| hsa-miR-302a | tcAccAaaAcaTggAagmCacTta | 72 | 25 |
| hsa-miR-302a* | aaaGcaAgtAcaTccAcgTtta | 69 | 32 |
| hsa-miR-302b | ctActAaaAcaTggAagmCacTta | 69 | 23 |
| hsa-miR-302b* | agAaaGcamCttmCcaTgtTaaAgt | 72 | 36 |
| hsa-miR-302c | ccActGaaAcaTggAagmCacTta | 74 | 28 |
| hsa-miR-302c* | cagmCagGtamCccmCcaTgtTaaa | 76 | 44 |
| hsa-miR-302d | acActmCaaAcaTggAagmCacTta | 73 | 23 |
| hsa-miR-30a-3p | gctGcaAacAtcmCgamCtgAaag | 74 | 28 |
| hsa-miR-30a-5p | cTtcmCagTcgAggAtgTttAca | 73 | 31 |
| hsa-miR-30b | agcTgaGtgTagGatGttTaca | 71 | 33 |
| hsa-miR-30c | gmCtgAgaGtgTagGatGttTaca | 73 | 33 |
| hsa-miR-30d | cttmCcaGtcGggGatGttTaca | 76 | 44 |
| hsa-miR-30e-3p | gmCtgTaaAcaTccGacTgaAag | 73 | 27 |
| hsa-miR-30e-5p | tcmCagTcaAggAtgTttAca | 69 | 30 |
| hsa-miR-31 | cagmCtaTgcmCagmCatmCttGcc | 78 | 38 |
| hsa-miR-32 | gcaActTagTaaTgtGcaAta | 65 | 43 |
| hsa-miR-320 | tTcgmCccTctmCaamCccAgcTttt | 80 | 26 |
| hsa-miR-323 | agAggTcgAccGtgTaaTgtGc | 80 | 46 |
| hsa-miR-324-3p | ccAgcAgcAccTggGgcAgtGg | 92 | 41 |
| hsa-miR-324-5p | acAccAatGccmCtaGggGatGcg | 84 | 54 |
| hsa-miR-325 | amCacTtamCtgGacAccTacTagg | 74 | 39 |
| hsa-miR-326 | ctgGagGaaGggmCccAgaGg | 87 | 46 |
| hsa-miR-328 | acGgaAggGcaGagAggGccAg | 87 | 31 |
| hsa-miR-33 | cAatGcaActAcaAtgmCac | 68 | 30 |
| hsa-miR-330 | tmCtcTgcAggmCcgTgtGctTtgc | 84 | 53 |
| hsa-miR-331 | tTctAggAtaGgcmCcaGggGc | 84 | 51 |
| hsa-miR-335 | amCatTttTcgTtaTtgmCtcTtga | 67 | 26 |
| hsa-miR-337 | aaaGgcAtcAtaTagGagmCtgGa | 76 | 34 |
| hsa-miR-338 | tcaAcaAaaTcamCtgAtgmCtgGa | 73 | 33 |
| hsa-miR-339 | tgAgcTccTggAggAcaGgga | 83 | 47 |
| hsa-miR-340 | ggcTatAaaGtaActGagAcgGa | 72 | 34 |
| hsa-miR-342 | gacGggTgcGatTtcTgtGtgAga | 82 | 34 |
| hsa-miR-345 | gmCccTggActAggAgtmCagmCa | 84 | 40 |
| hsa-miR-346 | agaGgcAggmCatGcgGgcAgamCa | 92 | 50 |
| hsa-miR-34a | aamCaamCcaGctAagAcamCtgmCca | 80 | 27 |
| hsa-miR-34b | cAatmCagmCtaAtgAcamCtgmCcta | 74 | 30 |
| hsa-miR-34c | gcAatmCagmCtaActAcamCtgmCct | 76 | 31 |
| hsa-miR-361 | gTacmCccTggAgaTtcTgaTaa | 73 | 29 |
| hsa-miR-367 | tmCacmCatTgcTaaAgtGcaAtt | 72 | 41 |
| hsa-miR-368 | aaamCgtGgaAttTccTctAtgt | 70 | 45 |
| hsa-miR-369 | aaAgaTcaAccAtgTatTatt | 62 | 24 |
| hsa-miR-370 | ccAggTtcmCacmCccAgcAggc | 86 | 29 |
| hsa-miR-371 | acActmCaaAagAtgGcgGcac | 76 | 33 |
| hsa-miR-372 | acgmCtcAaaTgtmCgcAgcActTt | 79 | 38 |
| hsa-miR-373 | acamCccmCaaAatmCgaAgcActTc | 77 | 33 |
| hsa-miR-373* | ggaAagmCgcmCccmCatTttGagt | 78 | 31 |
| hsa-miR-374 | cacTtaTcaGgtTgtAttAtaa | 63 | 35 |
| hsa-miR-375 | tcAcgmCgaGccGaamCgaAcaAa | 81 | 39 |
| hsa-miR-376a | acgTggAttTtcmCtcTatGat | 68 | 39 |
| hsa-miR-377 | acAaaAgtTgcmCttTgtGtgAt | 73 | 48 |
| hsa-miR-378 | amCacAggAccTggAgtmCagGag | 84 | 51 |
| hsa-miR-379 | tacGttmCcaTagTctAcca | 66 | 25 |
| hsa-miR-380-3p | aagAtgTggAccAtaTtamCata | 66 | 54 |
| hsa-miR-380-5p | gmCgcAtgTtcTatGgtmCaamCca | 80 | 41 |
| hsa-miR-381 | amCagAgaGctTgcmCctTgtAta | 76 | 37 |
| hsa-miR-382 | cgaAtcmCacmCacGaamCaamCttc | 75 | 23 |
| hsa-miR-383 | agcmCacAatmCacmCttmCtgAtct | 74 | 27 |
| hsa-miR-384 | tAtgAacAatTtcTagGaat | 61 | 46 |
| hsa-miR-422a | ggcmCttmCtgAccmCtaAgtmCcag | 76 | 45 |
| hsa-miR-422b | ggmCctTctGacTccAagTccAg | 80 | 39 |
| hsa-miR-423 | ctgAggGgcmCtcAgamCcgAgct | 87 | 61 |
| hsa-miR-424 | ttcAaaAcaTgaAttGctGctg | 69 | 40 |
| hsa-miR-425 | ggmCggAcamCgamCatTccmCgat | 83 | 43 |
| hsa-miR-7 | caamCaaAatmCacTagTctTcca | 69 | 30 |
| hsa-miR-9 | tcAtamCagmCtaGatAacmCaaAga | 71 | 34 |
| hsa-miR-9* | acTttmCggTtaTctAgcTtta | 65 | 27 |
| hsa-miR-92 | cagGccGggAcaAgtGcaAta | 79 | 28 |
| hsa-miR-93 | ctAccTgcAcgAacAgcActTt | 76 | 31 |
| hsa-miR-95 | tgcTcaAtaAatAccmCgtTgaa | 68 | 36 |
| hsa-miR-96 | gcaAaaAtgTgcTagTgcmCaaa | 72 | 38 |
| hsa-miR-98 | aAcaAtamCaamCttActAccTca | 67 | 17 |
| hsa-miR-99a | cacAagAtcGgaTctAcgGgtt | 77 | 42 |
| hsa-miR-99b | cgcAagGtcGgtTctAcgGgtg | 82 | 42 |
| mmu-let-7a | amCtaTacAacmCtamCtamCctmCa | 71 | 16 |
| mmu-let-7b | aamCcamCacAacmCtamCtamCctmCa | 77 | 6 |
| mmu-let-7c | aamCcaTacAacmCtamCtamCctmCa | 74 | 11 |
| mmu-let-7d | actAtgmCaamCctActAccTct | 71 | 24 |
| mmu-let-7d* | agAaaGgcAgcAggTcgTatAg | 79 | 23 |
| mmu-let-7e | actAtamCaamCctmCctAccTca | 71 | 16 |
| mmu-let-7f | amCtaTacAatmCtamCtamCctmCa | 68 | 16 |
| mmu-let-7g | amCtgTacAaamCtamCtamCctmCa | 71 | 30 |
| mmu-let-7i | amCagmCacAaamCtamCtamCctmCa | 76 | 18 |
| mmu-miR-1 | tamCatActTctTtamCatTcca | 64 | 11 |
| mmu-miR-100 | cacAagTtcGgaTctAcgGgtt | 77 | 38 |
| mmu-miR-101a | cttmCagTtaTcamCagTacTgta | 68 | 54 |
| mmu-miR-101b | cttmCagmCtaTcamCagTacTgta | 70 | 54 |
| mmu-miR-103 | tmCatAgcmCctGtamCaaTgcTgct | 80 | 63 |
| mmu-miR-106a | tacmCtgmCacTgtTagmCacTttg | 73 | 44 |
| mmu-miR-106b | atcTgcActGtcAgcActTta | 72 | 35 |
| mmu-miR-107 | tGatAgcmCctGtamCaaTgcTgct | 80 | 63 |
| mmu-miR-10a | cAcaAatTcgGatmCtamCagGgta | 74 | 37 |
| mmu-miR-10b | acamCaaAttmCggTtcTacAggg | 73 | 27 |
| mmu-miR-122a | acAaamCacmCatTgtmCacActmCca | 78 | 25 |
| mmu-miR-124a | ggmCatTcamCcgmCgtGccTta | 80 | 43 |
| mmu-miR-125a | cAcaGgtTaaAggGtcTcaGgga | 79 | 35 |
| mmu-miR-125b | tcamCaaGttAggGtcTcaGgga | 77 | 35 |
| mmu-miR-126-3p | gcAttAttActmCacGgtAcga | 71 | 25 |
| mmu-miR-126-5p | cgmCgtAccAaaAgtAatAatg | 68 | 28 |
| mmu-miR-127 | gcmCaaGctmCagAcgGatmCcga | 80 | 54 |
| mmu-miR-128a | aaAagAgamCcgGttmCacTgtGa | 77 | 47 |
| mmu-miR-128b | gaAagAgamCcgGttmCacTgtGa | 78 | 47 |
| mmu-miR-129 | agcAagmCccAgamCcgmCaaAaag | 80 | 21 |
| mmu-miR-129-3p | aTgcTttTtgGggTaaGggmCtt | 78 | 37 |
| mmu-miR-129-5p | agcAagmCccAgamCcgmCaaAaag | 80 | 21 |
| mmu-miR-130a | aTgcmCctTttAacAttGcamCtg | 74 | 42 |
| mmu-miR-130b | aTgcmCctTtcAtcAttGcamCtg | 75 | 34 |
| mmu-miR-132 | cgAccAtgGctGtaGacTgtTa | 76 | 48 |
| mmu-miR-133a | acAgcTggTtgAagGggAccAa | 82 | 41 |
| mmu-miR-133b | taGctGgtTgaAggGgamCcaa | 81 | 37 |
| mmu-miR-134 | cccmCtcTggTcaAccAgtmCaca | 79 | 57 |
| mmu-miR-135a | tcamCatAggAatAaaAagmCcaTa | 69 | 22 |
| mmu-miR-135b | cacAtaGgaAtgAaaAgcmCata | 70 | 22 |
| mmu-miR-136 | tccAtcAtcAaaAcaAatGgaGt | 71 | 25 |
| mmu-miR-137 | cTacGcgTatTctTaaGcaAtaa | 67 | 48 |
| mmu-miR-138 | gatTcamCaamCacmCagmCt | 70 | 24 |
| mmu-miR-139 | aGacAcgTgcActGtaGa | 75 | 42 |
| mmu-miR-140 | ctamCcaTagGgtAaaAccActg | 71 | 56 |
| mmu-miR-140* | tcmCgtGgtTctAccmCtgTggTa | 81 | 49 |
| mmu-miR-141 | cmCatmCttTacmCagAcaGtgTta | 70 | 33 |
| mmu-miR-142-3p | ccaTaaAgtAggAaamCacTaca | 69 | 29 |
| mmu-miR-142-5p | gtaGtgmCttTctActTtaTg | 63 | 36 |
| mmu-miR-143 | tGagmCtamCagTgcTtcAtcTca | 75 | 56 |
| mmu-miR-144 | ctaGtamCatmCatmCtaTacTgta | 64 | 37 |
| mmu-miR-145 | aAggGatTccTggGaaAacTggAc | 79 | 50 |
| mmu-miR-146 | aAccmCatGgaAttmCagTtcTca | 73 | 44 |
| mmu-miR-148a | acaAagTtcTgtAgtGcamCtga | 72 | 54 |
| mmu-miR-148b | acaAagTtcTgtGatGcamCtga | 72 | 39 |
| mmu-miR-149 | ggaGtgAagAcamCggAgcmCaga | 80 | 31 |
| mmu-miR-150 | cacTggTacAagGgtTggGaga | 78 | 30 |
| mmu-miR-151 | cmCtcAagGagmCctmCagTctAg | 78 | 42 |
| mmu-miR-152 | cmCcaAgtTctGtcAtgmCacTga | 78 | 36 |
| mmu-miR-153 | gatmCacTttTgtGacTatGcaa | 69 | 36 |
| mmu-miR-154 | cGaaGgcAacAcgGatAacmCta | 78 | 40 |
| mmu-miR-155 | ccmCctAtcAcaAttAgcAttAa | 69 | 21 |
| mmu-miR-15a | cAcaAacmCatTatGtgmCtgmCta | 73 | 35 |
| mmu-miR-15b | tgtAaamCcaTgaTgtGctGcta | 74 | 38 |
| mmu-miR-16 | cgmCcaAtaTttAcgTgcTgcTa | 74 | 34 |
| mmu-miR-17-3p | tacAagTgcmCctmCacTgcAgt | 79 | 42 |
| mmu-miR-17-5p | actAccTgcActGtaAgcActTtg | 74 | 39 |
| mmu-miR-18 | tatmCtgmCacTagAtgmCacmCtta | 71 | 40 |
| mmu-miR-181a | acTcamCcgAcaGcgTtgAatGtt | 77 | 49 |
| mmu-miR-181b | cccAccGacAgcAatGaaTgtt | 78 | 30 |
| mmu-miR-181c | amCtcAccGacAggTtgAatGtt | 76 | 33 |
| mmu-miR-182 | tgtGagTtcTacmCatTgcmCaaa | 72 | 32 |
| mmu-miR-183 | caGtgAatTctAccAgtGccAta | 73 | 32 |
| mmu-miR-184 | acmCctTatmCagTtcTccGtcmCa | 76 | 23 |
| mmu-miR-185 | gAacTgcmCttTctmCtcmCa | 70 | 27 |
| mmu-miR-186 | aaGccmCaaAagGagAatTctTtg | 71 | 48 |
| mmu-miR-187 | ccGgcTgcAacAcaAgamCacGa | 85 | 31 |
| mmu-miR-188 | amCccTccAccAtgmCaaGggAtg | 83 | 42 |
| mmu-miR-189 | actGatAtcAgcTcaGtaGgcAc | 77 | 54 |
| mmu-miR-190 | acmCtaAtaTatmCaaAcaTatmCa | 62 | 31 |
| mmu-miR-191 | agcTgcTttTggGatTccGttg | 74 | 42 |
| mmu-miR-192 | tgTcaAttmCatAggTcag | 64 | 28 |
| mmu-miR-193 | ctGggActTtgTagGccAgtt | 76 | 31 |
| mmu-miR-194 | tccAcaTggAgtTgcTgtTaca | 75 | 41 |
| mmu-miR-195 | gmCcaAtaTttmCtgTgcTgcTa | 73 | 28 |
| mmu-miR-196a | ccaAcaAcaTgaAacTacmCta | 67 | 20 |
| mmu-miR-196b | cmCaamCaamCagGaaActAccTa | 73 | 27 |
| mmu-miR-199a | gaAcaGgtAgtmCtgAacActGgg | 78 | 40 |
| mmu-miR-199a* | aacmCaaTgtGcaGacTacTgta | 74 | 39 |
| mmu-miR-199b | gaAcaGgtAgtmCtaAacActGgg | 76 | 31 |
| mmu-miR-19a | tmCagTttTgcAtaGatTtgmCaca | 72 | 37 |
| mmu-miR-19b | tmCagTttTgcAtgGatTtgmCaca | 75 | 34 |
| mmu-miR-20 | ctAccTgcActAtaAgcActTta | 70 | 26 |
| mmu-miR-200a | acaTcgTtamCcaGacAgtGtta | 72 | 39 |
| mmu-miR-200b | gtcAtcAttAccAggmCagTatTa | 71 | 31 |
| mmu-miR-200c | ccAtcAttAccmCggmCagTatTa | 74 | 38 |
| mmu-miR-201 | agAacAatGccTtamCtgAgta | 69 | 37 |
| mmu-miR-202 | tmCttmCccAtgmCgcTatAccTct | 76 | 28 |
| mmu-miR-203 | cTagTggTccTaaAcaTttmCa | 68 | 23 |
| mmu-miR-204 | cagGcaTagGatGacAaaGggAa | 78 | 25 |
| mmu-miR-205 | caGacTccGgtGgaAtgAagGa | 81 | 39 |
| mmu-miR-206 | ccamCacActTccTtamCatTcca | 73 | 11 |
| mmu-miR-207 | gaGggAggAgaGccAggAgaAgc | 86 | 18 |
| mmu-miR-208 | acAagmCttTttGctmCgtmCttAt | 71 | 34 |
| mmu-miR-21 | tmCaamCatmCagTctGatAagmCta | 72 | 48 |
| mmu-miR-210 | tcAgcmCgcTgtmCacAcgmCacAg | 87 | 38 |
| mmu-miR-211 | aggmCaaAggAtgAcaAagGgaa | 75 | 18 |
| mmu-miR-212 | gGccGtgActGgaGacTgtTa | 81 | 37 |
| mmu-miR-213 | gGtamCaaTcaAcgGtcGatGgt | 79 | 67 |
| mmu-miR-214 | ctGccTgtmCtgTgcmCtgmCtgt | 81 | 30 |
| mmu-miR-215 | gtmCtgTcaAatmCatAggTcat | 68 | 35 |
| mmu-miR-216 | camCagTtgmCcaGctGagAtta | 74 | 64 |
| mmu-miR-217 | atmCcaGtcAgtTccTgaTgcAgta | 77 | 43 |
| mmu-miR-218 | acAtgGttAgaTcaAgcAcaa | 70 | 40 |
| mmu-miR-219 | agAatTgcGttTggAcaAtca | 70 | 35 |
| mmu-miR-22 | acaGttmCttmCaamCtgGcaGctt | 74 | 48 |
| mmu-miR-221 | aaamCccAgcAgamCaaTgtAgct | 79 | 31 |
| mmu-miR-222 | gaGacmCcaGtaGccAgaTgtAgct | 80 | 38 |
| mmu-miR-223 | gGggTatTtgAcaAacTgamCa | 73 | 40 |
| mmu-miR-224 | tAaamCggAacmCacTagTgamCtta | 74 | 49 |
| mmu-miR-23a | gGaaAtcmCctGgcAatGtgAt | 76 | 37 |
| mmu-miR-23b | ggtAatmCccTggmCaaTgtGat | 76 | 38 |
| mmu-miR-24 | cTgtTccTgcTgaActGagmCca | 80 | 35 |
| mmu-miR-25 | tcaGacmCgaGacAagTgcAatg | 77 | 27 |
| mmu-miR-26a | gcmCtaTccTggAttActTgaa | 70 | 34 |
| mmu-miR-26b | aacmCtaTccTgaAttActTgaa | 65 | 28 |
| mmu-miR-27a | gcGgaActTagmCcamCtgTgaa | 77 | 35 |
| mmu-miR-27b | gcAgaActTagmCcamCtgTgaa | 74 | 38 |
| mmu-miR-28 | ctmCaaTagActGtgAgcTccTt | 73 | 43 |
| mmu-miR-290 | aaaAagTgcmCccmCatAgtTtgAg | 75 | 29 |
| mmu-miR-291-3p | gGcamCacAaaGtgGaaGcamCttt | 78 | 52 |
| mmu-miR-291-5p | aGagAggGccTccActTtgAtg | 77 | 46 |
| mmu-miR-292-3p | acActmCaaAacmCtgGcgGcamCtt | 80 | 33 |
| mmu-miR-292-5p | caaAagAgcmCccmCagTttGagt | 76 | 32 |
| mmu-miR-293 | amCacTacAaamCtcTgcGgcAct | 81 | 30 |
| mmu-miR-294 | acAcamCaaAagGgaAgcActTt | 75 | 25 |
| mmu-miR-295 | agamCtcAaaAgtAgtAgcActTt | 70 | 44 |
| mmu-miR-296 | acAggAttGagGggGggmCcct | 88 | 48 |
| mmu-miR-297 | cAtgmCacAtgmCacAcaTacAt | 75 | 41 |
| mmu-miR-298 | gGaaGaamCagmCccTccTctGcc | 82 | 53 |
| mmu-miR-299 | aTgtAtgTggGacGgtAaamCca | 80 | 35 |
| mmu-miR-29a | aamCcgAttTcaGatGgtGcta | 75 | 43 |
| mmu-miR-29b | aamCacTgaTttmCaaAtgGtgmCta | 71 | 47 |
| mmu-miR-29c | amCcgAttTcaAatGgtGcta | 71 | 47 |
| mmu-miR-300 | gAagAgaGctTgcmCctTgcAta | 77 | 35 |
| mmu-miR-301 | gctTtgAcaAtamCtaTtgmCacTg | 70 | 36 |
| mmu-miR-302 | tcAccAaaAcaTggAagmCacTta | 72 | 25 |
| mmu-miR-30a-3p | gctGcaAacAtcmCgamCtgAaag | 74 | 28 |
| mmu-miR-30a-5p | cTtcmCagTcgAggAtgTttAca | 73 | 31 |
| mmu-miR-30b | agcTgaGtgTagGatGttTaca | 71 | 33 |
| mmu-miR-30c | gmCtgAgaGtgTagGatGttTaca | 73 | 33 |
| mmu-miR-30d | cttmCcaGtcGggGatGttTaca | 76 | 44 |
| mmu-miR-30e | tcmCagTcaAggAtgTttAca | 69 | 30 |
| mmu-miR-30e* | ctgTaaAcaTccGacTgaAag | 69 | 27 |
| mmu-miR-31 | cagmCtaTgcmCagmCatmCttGcct | 79 | 38 |
| mmu-miR-32 | gcaActTagTaaTgtGcaAta | 65 | 43 |
| mmu-miR-320 | tTcgmCccTctmCaamCccAgcTttt | 80 | 26 |
| mmu-miR-322-3p | tgtTgcAgcGctTcaTgtTt | 74 | 48 |
| mmu-miR-322-5p | tccAaaAcaTgaAttGctGctg | 71 | 40 |
| mmu-miR-323 | agAggTcgAccGtgTaaTgtGc | 80 | 46 |
| mmu-miR-324-3p | ccAgcAgcAccTggGgcAgtGg | 92 | 41 |
| mmu-miR-324-5p | cAccAatGccmCtaGggGatGcg | 83 | 54 |
| mmu-miR-325 | acamCttActGagmCacmCtamCtaGg | 78 | 42 |
| mmu-miR-326 | actGgaGgaAggGccmCagAgg | 86 | 46 |
| mmu-miR-328 | acGgaAggGcaGagAggGccAg | 87 | 31 |
| mmu-miR-329 | aAaaAggTtaGctGggTgtGtt | 75 | 32 |
| mmu-miR-33 | cAatGcaActAcaAtgmCac | 68 | 30 |
| mmu-miR-330 | tmCtcTgcAggmCccTgtGctTtgc | 83 | 52 |
| mmu-miR-331 | tTctAggAtaGgcmCcaGggGc | 84 | 51 |
| mmu-miR-335 | amCatTttTcgTtaTtgmCtcTtga | 67 | 26 |
| mmu-miR-337 | aaaGgcAtcAtaTagGagmCtgAa | 74 | 35 |
| mmu-miR-338 | tcaAcaAaaTcamCtgAtgmCtgGa | 73 | 33 |
| mmu-miR-339 | tgAgcTccTggAggAcaGgga | 83 | 47 |
| mmu-miR-340 | ggcTatAaaGtaActGagAcgGa | 72 | 34 |
| mmu-miR-341 | amCtgAccGacmCgamCcgAtcGa | 84 | 53 |
| mmu-miR-342 | gacGggTgcGatTtcTgtGtgAga | 82 | 34 |
| mmu-miR-344 | amCagTcaGgcTttGgcTagAtca | 79 | 53 |
| mmu-miR-345 | gcActGgamCtaGggGtcAgca | 83 | 43 |
| mmu-miR-346 | agaGgcAggmCacTcgGgcAgamCa | 91 | 38 |
| mmu-miR-34a | aamCaamCcaGctAagAcamCtgmCca | 80 | 27 |
| mmu-miR-34b | caaTcaGctAatTacActGccTa | 71 | 40 |
| mmu-miR-34c | gcAatmCagmCtaActAcamCtgmCct | 76 | 31 |
| mmu-miR-350 | tgaAagTgtAtgGgcTttGtgAa | 73 | 42 |
| mmu-miR-351 | cagGctmCaaAggGctmCctmCagGga | 84 | 59 |
| mmu-miR-361 | gTacmCccTggAgaTtcTgaTaa | 73 | 29 |
| mmu-miR-370 | aAccAggTtcmCacmCccAgcAggc | 86 | 34 |
| mmu-miR-375 | tcAcgmCgaGccGaamCgaAcaAa | 81 | 39 |
| mmu-miR-376a | acgTggAttTtcmCtcTacGat | 71 | 47 |
| mmu-miR-376b | aAagTggAtgTtcmCtcTatGat | 70 | 39 |
| mmu-miR-377 | acAaaAgtTgcmCttTgtGtgAt | 73 | 48 |
| mmu-miR-378 | amCacAggAccTggAgtmCagGag | 84 | 51 |
| mmu-miR-379 | cctAcgTtcmCatAgtmCtamCca | 72 | 33 |
| mmu-miR-380-3p | aagAtgTggAccAtamCtamCata | 69 | 49 |
| mmu-miR-380-5p | gmCgcAtgTtcTatGgtmCaamCca | 80 | 41 |
| mmu-miR-381 | amCagAgaGctTgcmCctTgtAta | 76 | 37 |
| mmu-miR-382 | cgaAtcmCacmCacGaamCaamCttc | 75 | 23 |
| mmu-miR-383 | agcmCacAgtmCacmCttmCtgAtct | 76 | 25 |
| mmu-miR-384 | tGtgAacAatTtcTagGaat | 64 | 46 |
| mmu-miR-409 | aAggGgtTcamCcgAgcAacAttc | 80 | 35 |
| mmu-miR-410 | aacAggmCcaTctGtgTtaTatt | 70 | 39 |
| mmu-miR-411 | actGagGgtTagTggAccGtgTt | 80 | 40 |
| mmu-miR-412 | acgGctAgtGgamCcaGgtGaaGt | 86 | 53 |
| mmu-miR-425 | ggmCggAcamCgamCatTccmCgat | 83 | 43 |
| mmu-miR-7 | caamCaaAatmCacTagTctTcca | 69 | 30 |
| mmu-miR-7b | aamCaaAatmCacAagTctTcca | 68 | 24 |
| mmu-miR-9 | cAtamCagmCtaGatAacmCaaAga | 70 | 34 |
| mmu-miR-9* | acTttmCggTtaTctAgcTtta | 65 | 27 |
| mmu-miR-92 | cagGccGggAcaAgtGcaAta | 79 | 28 |
| mmu-miR-93 | ctAccTgcAcgAacAgcActTtg | 77 | 31 |
| mmu-miR-96 | aGcaAaaAtgTgcTagTgcmCaaa | 75 | 38 |
| mmu-miR-98 | aAcaAtamCaamCttActAccTca | 67 | 17 |
| mmu-miR-99a | acAagAtcGgaTctAcgGgt | 77 | 40 |
| mmu-miR-99b | cgcAagGtcGgtTctAcgGgtg | 82 | 42 |
| osa-miR1456a | gtgmCtcActmCtcTtcTgtmCa | 71 | 25 |
| osa-miR156b | gtgmCtcActmCtcTtcTgtmCa | 71 | 25 |
| osa-miR156c | gtgmCtcActmCtcTtcTgtmCa | 71 | 25 |
| osa-miR156d | gtgmCtcActmCtcTtcTgtmCa | 71 | 25 |
| osa-miR156e | gtgmCtcActmCtcTtcTgtmCa | 71 | 25 |
| osa-miR156f | gtgmCtcActmCtcTtcTgtmCa | 71 | 25 |
| osa-miR156g | gtgmCtcActmCtcTtcTgtmCa | 71 | 25 |
| osa-miR156h | gtgmCtcActmCtcTtcTgtmCa | 71 | 25 |
| osa-miR156i | gtgmCtcActmCtcTtcTgtmCa | 71 | 25 |
| osa-miR156j | gtgmCtcActmCtcTtcTgtmCa | 71 | 25 |
| osa-miR156k | tgTgcTctmCtcTctTctGtca | 72 | 21 |
| osa-miR156l | taTgcTcamCtcTctTctGtcg | 71 | 17 |
| osa-miR159a | cagAgcTccmCttmCaaTccAaa | 73 | 36 |
| osa-miR159b | cagAgcTccmCttmCaaTccAaa | 73 | 36 |
| osa-miR159c | tggAgcTccmCttmCaaTccAat | 74 | 46 |
| osa-miR159d | cggAgcTccmCttmCaaTccAat | 75 | 46 |
| osa-miR159e | aggAgcTccmCttmCaaTccAat | 74 | 46 |
| osa-miR159f | tagAgcTccmCttmCaaTccAag | 72 | 36 |
| osa-miR160a | tggmCatAcaGggAgcmCagGca | 85 | 49 |
| osa-miR160b | tggmCatAcaGggAgcmCagGca | 85 | 49 |
| osa-miR160c | tggmCatAcaGggAgcmCagGca | 85 | 49 |
| osa-miR160d | tggmCatAcaGggAgcmCagGca | 85 | 49 |
| osa-miR160e | cggmCatAcaGggAgcmCagGca | 85 | 43 |
| osa-miR160f | tgGcaTtcAggGagmCcaGgca | 84 | 60 |
| osa-miR162a | ctgGatGcaGagGttTatmCga | 73 | 34 |
| osa-miR162b | ctgGatGcaGagGctTatmCga | 76 | 36 |
| osa-miR164a | tgcAcgTgcmCctGctTctmCca | 82 | 46 |
| osa-miR164b | tgcAcgTgcmCctGctTctmCca | 82 | 46 |
| osa-miR164c | tGcamCgtAccmCtgmCttmCtcmCa | 82 | 32 |
| osa-miR164d | agcAcgTgcmCctGctTctmCca | 82 | 47 |
| osa-miR164e | ctcAcgTgcmCctGctTctmCca | 80 | 36 |
| osa-miR166a | gGggAatGaaGccTggTccGa | 84 | 33 |
| osa-miR166b | gGggAatGaaGccTggTccGa | 84 | 33 |
| osa-miR166c | gGggAatGaaGccTggTccGa | 84 | 33 |
| osa-miR166d | gGggAatGaaGccTggTccGa | 84 | 33 |
| osa-miR166e | gGggAatGaaGccTggTccGa | 84 | 33 |
| osa-miR166f | gGggAatGaaGccTggTccGa | 84 | 33 |
| osa-miR166g | gAggAatGaaGccTggTccGa | 80 | 29 |
| osa-miR166h | gAggAatGaaGccTggTccGa | 80 | 29 |
| osa-miR166i | gAggAatGaaGccTgaTccGa | 78 | 29 |
| osa-miR166j | gAggAatGaaGccTgaTccGa | 78 | 29 |
| osa-miR166k | aGggAttGaaGccTggTccGa | 83 | 37 |
| osa-miR1661 | aGggAttGaaGccTggTccGa | 83 | 37 |
| osa-miR167a | tAgaTcaTgcTggmCagmCttmCa | 79 | 53 |
| osa-miR167b | tAgaTcaTgcTggmCagmCttmCa | 79 | 53 |
| osa-miR167c | tAgaTcaTgcTggmCagmCttmCa | 79 | 53 |
| osa-miR167d | cAgaTcaTgcTggmCagmCttmCa | 80 | 53 |
| osa-miR167e | cAgaTcaTgcTggmCagmCttmCa | 80 | 53 |
| osa-miR167f | cAgaTcaTgcTggmCagmCttmCa | 80 | 53 |
| osa-miR167g | cAgaTcaTgcTggmCagmCttmCa | 80 | 53 |
| osa-miR167h | cAgaTcaTgcTggmCagmCttmCa | 80 | 53 |
| osa-miR167i | cAgaTcaTgcTggmCagmCttmCa | 80 | 53 |
| osa-miR168a | gtmCccGatmCtgmCacmCaaGcga | 82 | 38 |
| osa-miR168b | ttcmCcgAgcTgcAccAagmCct | 83 | 30 |
| osa-miR169a | tcGgcAagTcaTccTtgGctg | 78 | 40 |
| osa-miR169b | ccGgcAagTcaTccTtgGctg | 79 | 40 |
| osa-miR169c | ccGgcAagTcaTccTtgGctg | 79 | 40 |
| osa-miR169d | ccGgcAatTcaTccTtgGcta | 76 | 33 |
| osa-miR169e | ccGgcAagTcaTccTtgGcta | 78 | 35 |
| osa-miR169f | taGgcAagTcaTccTtgGcta | 74 | 47 |
| osa-miR169g | taGgcAagTcaTccTtgGcta | 74 | 47 |
| osa-miR169h | caGgcAagTcaTccTtgGcta | 76 | 41 |
| osa-miR169i | caGgcAagTcaTccTtgGcta | 76 | 41 |
| osa-miR169j | caGgcAagTcaTccTtgGcta | 76 | 41 |
| osa-miR169k | caGgcAagTcaTccTtgGcta | 76 | 41 |
| osa-miR169l | caGgcAagTcaTccTtgGcta | 76 | 41 |
| osa-miR169m | caGgcAagTcaTccTtgGcta | 76 | 41 |
| osa-miR169n | taGgcAagTcaTtcTtgGcta | 71 | 47 |
| osa-miR169o | taGgcAagTcaTtcTtgGcta | 71 | 47 |
| osa-miR169p | ccgGcaAgtTtgTccTtgGcta | 76 | 52 |
| osa-miR169q | caTggGcaGtcTccTtgGcta | 75 | 47 |
| osa-miR171a | gAtaTtgGcgmCggmCtcAatmCa | 78 | 54 |
| osa-miR171b | gaTatTggmCacGgcTcaAtca | 75 | 46 |
| osa-miR171c | gaTatTggmCacGgcTcaAtca | 75 | 46 |
| osa-miR171d | gaTatTggmCacGgcTcaAtca | 75 | 46 |
| osa-miR171e | gaTatTggmCacGgcTcaAtca | 75 | 46 |
| osa-miR171f | gaTatTggmCacGgcTcaAtca | 75 | 46 |
| osa-miR171g | gaTatTggmCtcGgcTcamCctc | 78 | 34 |
| osa-miR171h | agTgaTatTggTtcGgcTcac | 74 | 34 |
| osa-miR172a | atgmCagmCatmCatmCaaGatTct | 73 | 45 |
| osa-miR172b | aTgcAgcAtcAtcAagAttmCc | 74 | 39 |
| osa-miR172c | gTgcAgcAtcAtcAagAttmCa | 74 | 39 |
| osa-miR319a | gggAgcAccmCttmCagTccAa | 78 | 39 |
| osa-miR319b | gggAgcAccmCttmCagTccAa | 78 | 39 |
| osa-miR393 | gAtcAatGcgAtcmCctTtgGa | 74 | 56 |
| osa-miR393b | agaTcaAtgmCgaTccmCttTgga | 73 | 56 |
| osa-miR394 | gGagGtgGacAgaAtgmCcaa | 77 | 29 |
| osa-miR395a | gagTtcmCccmCaaAtamCttmCac | 71 | 23 |
| osa-miR395b | gAgtTccmCccAaamCacTtcAc | 75 | 28 |
| osa-miR395c | gAgtTccmCccAagmCacTtcAc | 78 | 28 |
| osa-miR395d | gAgtTccmCccAaamCacTtcAc | 75 | 28 |
| osa-miR395e | gAgtTccmCccAaamCacTtcAc | 75 | 28 |
| osa-miR395f | gatTtcmCccmCaaAcgmCttmCac | 74 | 22 |
| osa-miR395g | gAgtTccmCccAaamCacTtcAc | 75 | 28 |
| osa-miR395h | gAgtTccmCccAaamCacTtcAc | 75 | 28 |
| osa-miR395i | gAgtTccmCccAaamCacTtcAc | 75 | 28 |
| osa-miR395j | gAgtTccmCccAaamCacTtcAc | 75 | 28 |
| osa-miR395k | gAgtTccmCccAaamCacTtcAc | 75 | 28 |
| osa-miR3951 | gAgtTccmCccAaamCacTtcAc | 75 | 28 |
| osa-miR395m | gAgtTccmCccAaamCacTtcAc | 75 | 28 |
| osa-miR395n | gAgtTtcmCccAaamCacTtcAc | 73 | 35 |
| osa-miR395o | gAgtTtcmCccAaamCacTtcAc | 73 | 35 |
| osa-miR395p | gatTtcmCccmCaaAcgmCttmCac | 74 | 22 |
| osa-miR395q | gAgtTccTccAaamCacTtcAc | 72 | 29 |
| osa-miR395r | gAgtTtcmCccAaamCacTtcAc | 73 | 35 |
| osa-miR395s | gatTtcmCccmCaaAcgmCttmCac | 74 | 22 |
| osa-miR396a | cagTtcAagAaaGctGtgGaa | 70 | 35 |
| osa-miR396b | cagTtcAagAaaGctGtgGaa | 70 | 35 |
| osa-miR396c | aagTtcAagAaaGctGtgGaa | 69 | 24 |
| osa-miR397a | caTcaAcgmCtgmCacTcaAtga | 73 | 39 |
| osa-miR397b | caTcaAcgmCtgmCacTcaAtaa | 71 | 35 |
| osa-miR398a | aagGggTgamCctGagAacAca | 80 | 39 |
| osa-miR398b | caGggGcgAccTgaGaamCaca | 83 | 43 |
| osa-miR399a | cAggGcaAttmCtcmCttTggmCa | 78 | 48 |
| osa-miR399b | cAggGcaAttmCtcmCttTggmCa | 78 | 48 |
| osa-miR399c | cAggGcaAttmCtcmCttTggmCa | 78 | 48 |
| osa-miR399d | caGggmCaamCtcTccTttGgca | 81 | 39 |
| osa-miR399e | cTggGcaAatmCtcmCttTggmCa | 77 | 41 |
| osa-miR399f | cTggGcaAatmCtcmCttTggmCa | 77 | 41 |
| osa-miR399g | cmCggGcaAatmCtcmCttTggmCa | 80 | 41 |
| osa-miR399h | cTggGcaAgtmCtcmCttTggmCa | 80 | 37 |
| osa-miR399i | caGggmCagmCtcTccTttGgca | 83 | 63 |
| osa-miR399j | taGggmCaamCtcTccTttGgca | 80 | 39 |
| osa-miR399k | cggGgcAaaTttmCctTtgGca | 76 | 53 |
| osa-miR408 | gmCcaGggAagAggmCagTgcAg | 88 | 35 |
| osa-miR413 | gtgmCagAacAagTgaAacTag | 70 | 24 |
| osa-miR414 | gGacGatGatGatGagGatGa | 77 | 21 |
| osa-miR415 | ctgmCtcTgcTtcTgtTctGtt | 71 | 19 |
| osa-miR416 | tgAacAgtGtamCggAcgAaca | 75 | 42 |
| osa-miR417 | tgGaamCaaAttmCacTacAttc | 66 | 26 |
| osa-miR418 | cgTcaTttmCatmCatmCacAtta | 67 | 16 |
| osa-miR419 | caamCatmCgtmCagmCatTcaTca | 74 | 18 |
| osa-miR420 | atcAttTccGtgAttAatTta | 60 | 32 |
| osa-miR426 | cgtAagGacAaamCttmCcaAaa | 69 | 31 |
| rno-let-7a | aamCtaTacAacmCtamCtamCctmCa | 70 | 16 |
| rno-let-7b | aamCcamCacAacmCtamCtamCctmCa | 77 | 6 |
| rno-let-7c | aamCcaTacAacmCtamCtamCctmCa | 74 | 11 |
| rno-let-7d | actAtgmCaamCctActAccTct | 71 | 24 |
| rno-let-7d* | agAaaGgcAgcAggTcgTatAg | 79 | 23 |
| rno-let-7e | actAtamCaamCctmCctAccTca | 71 | 16 |
| rno-let-7f | aamCtaTacAatmCtamCtamCctmCa | 67 | 16 |
| rno-let-7i | amCagmCacAaamCtamCtamCctmCa | 76 | 18 |
| rno-miR-100 | cacAagTtcGgaTctAcgGgtt | 77 | 38 |
| rno-miR-101 | cttmCagTtaTcamCagTacTgta | 68 | 54 |
| rno-miR-101b | cttmCagmCtaTcamCagTacTgta | 70 | 54 |
| rno-miR-103 | tmCatAgcmCctGtamCaaTgcTgct | 80 | 63 |
| rno-miR-106b | atcTgcActGtcAgcActTta | 72 | 35 |
| rno-miR-107 | tGatAgcmCctGtamCaaTgcTgct | 80 | 63 |
| rno-miR-10a | cAcaAatTcgGatmCtamCagGgta | 74 | 37 |
| rno-miR-10b | acamCaaAttmCggTtcTacAggg | 73 | 27 |
| rno-miR-122a | acAaamCacmCatTgtmCacActmCca | 78 | 25 |
| rno-miR-124a | tggmCatTcamCcgmCgtGccTtaa | 80 | 43 |
| rno-miR-125a | cAcaGgtTaaAggGtcTcaGgga | 79 | 35 |
| rno-miR-125b | tcamCaaGttAggGtcTcaGgga | 77 | 35 |
| rno-miR-126 | gcAttAttActmCacGgtAcga | 71 | 25 |
| rno-miR-126* | cgmCgtAccAaaAgtAatAatg | 68 | 28 |
| rno-miR-127 | agcmCaaGctmCagAcgGatmCcga | 81 | 54 |
| rno-miR-128a | aaAagAgamCcgGttmCacTgtGa | 77 | 47 |
| rno-miR-128b | gaAagAgamCcgGttmCacTgtGa | 78 | 47 |
| rno-miR-129 | agcAagmCccAgamCcgmCaaAaag | 80 | 21 |
| rno-miR-129* | aTgcTttTtgGggTaaGggmCtt | 78 | 37 |
| rno-miR-130a | aTgcmCctTttAacAttGcamCtg | 74 | 42 |
| rno-miR-130b | aTgcmCctTtcAtcAttGcamCtg | 75 | 34 |
| rno-miR-132 | cgAccAtgGctGtaGacTgtTa | 76 | 48 |
| rno-miR-133a | acAgcTggTtgAagGggAccAa | 82 | 41 |
| rno-miR-134 | ccmCtcTggTcaAccAgtmCaca | 77 | 57 |
| rno-miR-135a | tcamCatAggAatAaaAagmCcaTa | 69 | 22 |
| rno-miR-135b | cacAtaGgaAtgAaaAgcmCata | 70 | 22 |
| rno-miR-136 | tccAtcAtcAaaAcaAatGgaGt | 71 | 25 |
| rno-miR-137 | cTacGcgTatTctTaaGcaAta | 68 | 48 |
| rno-miR-138 | gatTcamCaamCacmCagmCt | 70 | 24 |
| rno-miR-139 | aGacAcgTgcActGtaGa | 75 | 42 |
| rno-miR-140 | ctAccAtaGggTaaAacmCact | 71 | 43 |
| rno-miR-140* | tgtmCcgTggTtcTacmCctGtgGta | 80 | 50 |
| rno-miR-141 | cmCatmCttTacmCagAcaGtgTta | 70 | 33 |
| rno-miR-142-3p | tmCcaTaaAgtAggAaamCacTaca | 72 | 29 |
| rno-miR-142-5p | gtaGtgmCttTctActTtaTg | 63 | 36 |
| rno-miR-143 | tGagmCtamCagTgcTtcAtcTca | 75 | 56 |
| rno-miR-144 | ctaGtamCatmCatmCtaTacTgta | 64 | 37 |
| rno-miR-145 | aAggGatTccTggGaaAacTggAc | 79 | 50 |
| rno-miR-146 | aAccmCatGgaAttmCagTtcTca | 73 | 44 |
| rno-miR-148b | acaAagTtcTgtGatGcamCtga | 72 | 39 |
| rno-miR-150 | cacTggTacAagGgtTggGaga | 78 | 30 |
| rno-miR-151 | cmCtcAagGagmCctmCagTctAgt | 78 | 42 |
| rno-miR-151* | tacTagActGtgAgcTccTcga | 74 | 42 |
| rno-miR-152 | cmCcaAgtTctGtcAtgmCacTga | 78 | 36 |
| rno-miR-153 | tcamCttTtgTgamCtaTgcAa | 68 | 35 |
| rno-miR-154 | cGaaGgcAacAcgGatAacmCta | 78 | 40 |
| rno-miR-15b | tgtAaamCcaTgaTgtGctGcta | 74 | 38 |
| rno-miR-16 | cgmCcaAtaTttAcgTgcTgcTa | 74 | 34 |
| rno-miR-17 | actAccTgcActGtaAgcActTtg | 74 | 39 |
| rno-miR-18 | tatmCtgmCacTagAtgmCacmCtta | 71 | 40 |
| rno-miR-181a | acTcamCcgAcaGcgTtgAatGtt | 77 | 49 |
| rno-miR-181b | cccAccGacAgcAatGaaTgtt | 78 | 30 |
| rno-miR-181c | amCtcAccGacAggTtgAatGtt | 76 | 33 |
| rno-miR-183 | caGtgAatTctAccAgtGccAta | 73 | 32 |
| rno-miR-184 | acmCctTatmCagTtcTccGtcmCa | 76 | 23 |
| rno-miR-185 | gAacTgcmCttTctmCtcmCa | 70 | 27 |
| rno-miR-186 | aaGccmCaaAagGagAatTctTtg | 71 | 48 |
| rno-miR-187 | cGgcTgcAacAcaAgamCacGa | 84 | 31 |
| rno-miR-190 | acmCtaAtaTatmCaaAcaTatmCa | 62 | 31 |
| rno-miR-191 | agcTgcTttTggGatTccGttg | 74 | 42 |
| rno-miR-192 | gGctGtcAatTcaTagGtcAg | 73 | 46 |
| rno-miR-193 | ctGggActTtgTagGccAgtt | 76 | 31 |
| rno-miR-194 | tccAcaTggAgtTgcTgtTaca | 75 | 41 |
| rno-miR-195 | gmCcaAtaTttmCtgTgcTgcTa | 73 | 28 |
| rno-miR-196a | ccaAcaAcaTgaAacTacmCta | 67 | 20 |
| rno-miR-196b | cmCaamCaamCagGaaActAccTa | 73 | 27 |
| rno-miR-199a | gaAcaGgtAgtmCtgAacActGgg | 78 | 40 |
| rno-miR-19a | tmCagTttTgcAtaGatTtgmCaca | 72 | 37 |
| rno-miR-19b | tmCagTttTgcAtgGatTtgmCaca | 75 | 34 |
| rno-miR-20 | ctAccTgcActAtaAgcActTta | 70 | 26 |
| rno-miR-20* | tgtAagTgcTcgTaaTgcAgt | 74 | 26 |
| rno-miR-200a | acaTcgTtamCcaGacAgtGtta | 72 | 39 |
| rno-miR-200b | gtcAtcAttAccAggmCagTatTa | 71 | 31 |
| rno-miR-200c | ccAtcAttAccmCggmCagTatTa | 74 | 38 |
| rno-miR-203 | cTagTggTccTaaAcaTttmCac | 69 | 23 |
| rno-miR-204 | aggmCatAggAtgAcaAagGgaa | 75 | 25 |
| rno-miR-205 | caGacTccGgtGgaAtgAagGa | 81 | 39 |
| rno-miR-206 | ccamCacActTccTtamCatTcca | 73 | 11 |
| rno-miR-208 | acAagmCttTttGctmCgtmCttAt | 71 | 34 |
| rno-miR-21 | tmCaamCatmCagTctGatAagmCta | 72 | 48 |
| rno-miR-210 | tcAgcmCgcTgtmCacAcgmCacAg | 87 | 38 |
| rno-miR-211 | aggmCaaAggAtgAcaAagGgaa | 75 | 18 |
| rno-miR-212 | gGccGtgActGgaGacTgtTa | 81 | 37 |
| rno-miR-213 | gGtamCaaTcaAcgGtcGatGgt | 79 | 67 |
| rno-miR-214 | ctGccTgtmCtgTgcmCtgmCtgt | 81 | 30 |
| rno-miR-216 | camCagTtgmCcaGctGagAtta | 74 | 64 |
| rno-miR-217 | atmCcaGtcAgtTccTgaTgcAgta | 77 | 43 |
| rno-miR-218 | acAtgGttAgaTcaAgcAcaa | 70 | 40 |
| rno-miR-219 | agAatTgcGttTggAcaAtca | 70 | 35 |
| rno-miR-22 | acaGttmCttmCaamCtgGcaGctt | 74 | 48 |
| rno-miR-221 | gAaamCccAgcAgamCaaTgtAgct | 80 | 31 |
| rno-miR-222 | gaGacmCcaGtaGccAgaTgtAgct | 80 | 38 |
| rno-miR-223 | gGggTatTtgAcaAacTgamCa | 73 | 40 |
| rno-miR-23a | gGaaAtcmCctGgcAatGtgAt | 76 | 37 |
| rno-miR-23b | ggtAatmCccTggmCaaTgtGat | 76 | 38 |
| rno-miR-24 | cTgtTccTgcTgaActGagmCca | 80 | 35 |
| rno-miR-25 | tcaGacmCgaGacAagTgcAatg | 77 | 27 |
| rno-miR-26a | gcmCtaTccTggAttActTgaa | 70 | 34 |
| rno-miR-26b | aacmCtaTccTgaAttActTgaa | 65 | 28 |
| rno-miR-27a | gcGgaActTagmCcamCtgTgaa | 77 | 35 |
| rno-miR-27b | gcAgaActTagmCcamCtgTgaa | 74 | 38 |
| rno-miR-28 | ctmCaaTagActGtgAgcTccTt | 73 | 43 |
| rno-miR-290 | aaaAagTgcmCccmCatAgtTtgAg | 75 | 29 |
| rno-miR-291-3p | gGcamCacAaaGtgGaaGcamCttt | 78 | 52 |
| rno-miR-291-5p | aGagAggGccTccActTtgAtg | 77 | 46 |
| rno-miR-292-3p | acActmCaaAacmCtgGcgGcamCtt | 80 | 33 |
| rno-miR-292-5p | caaAagAgcmCccmCagTttGagt | 76 | 32 |
| rno-miR-296 | acAggAttGagGggGggmCcct | 88 | 48 |
| rno-miR-297 | cAtgmCatAcaTgcAcamCatAcat | 74 | 47 |
| rno-miR-298 | gGaaGaamCagmCccTccTctGcc | 82 | 53 |
| rno-miR-299 | aTgtAtgTggGacGgtAaamCca | 80 | 35 |
| rno-miR-29a | aamCcgAttTcaGatGgtGcta | 75 | 43 |
| rno-miR-29b | aamCacTgaTttmCaaAtgGtgmCta | 71 | 47 |
| rno-miR-29c | amCcgAttTcaAatGgtGcta | 71 | 47 |
| rno-miR-300 | gAagAgaGctTgcmCctTgcAta | 77 | 35 |
| rno-miR-301 | atGctTtgAcaAtamCtaTtgmCacTg | 72 | 42 |
| rno-miR-30a-3p | gctGcaAacAtcmCgamCtgAaag | 74 | 28 |
| rno-miR-30a-5p | cTtcmCagTcgAggAtgTttAca | 73 | 31 |
| rno-miR-30b | agcTgaGtgTagGatGttTaca | 71 | 33 |
| rno-miR-30c | gmCtgAgaGtgTagGatGttTaca | 73 | 33 |
| rno-miR-30d | cttmCcaGtcGggGatGttTaca | 76 | 44 |
| rno-miR-30e | tcmCagTcaAggAtgTttAca | 69 | 30 |
| rno-miR-31 | cagmCtaTgcmCagmCatmCttGcct | 79 | 38 |
| rno-miR-32 | gcaActTagTaaTgtGcaAta | 65 | 43 |
| rno-miR-320 | tTcgmCccTctmCaamCccAgcTttt | 80 | 26 |
| rno-miR-322 | tgtTgcAgcGctTcaTgtTt | 74 | 48 |
| rno-miR-323 | agAggTcgAccGtgTaaTgtGc | 80 | 46 |
| rno-miR-324-3p | ccAgcAgcAccTggGgcAgtGg | 92 | 41 |
| rno-miR-324-5p | acAccAatGccmCtaGggGatGcg | 84 | 54 |
| rno-miR-325 | acamCttActGagmCacmCtamCtaGg | 78 | 42 |
| rno-miR-326 | actGgaGgaAggGccmCagAgg | 86 | 46 |
| rno-miR-327 | accmCtcAtgmCccmCtcAagg | 76 | 27 |
| rno-miR-328 | acGgaAggGcaGagAggGccAg | 87 | 31 |
| rno-miR-329 | aAaaAggTtaGctGggTgtGtt | 75 | 32 |
| rno-miR-33 | cAatGcaActAcaAtgmCac | 68 | 30 |
| rno-miR-330 | tmCtcTgcAggmCccTgtGctTtgc | 83 | 52 |
| rno-miR-331 | tTctAggAtaGgcmCcaGggGc | 84 | 51 |
| rno-miR-333 | aaaAgtAacTagmCacAccAc | 69 | 24 |
| rno-miR-335 | amCatTttTcgTtaTtgmCtcTtga | 67 | 26 |
| rno-miR-336 | aGacTagAtaTggAagGgtGa | 75 | 28 |
| rno-miR-337 | aaaGgcAtcAtaTagGagmCtgAa | 74 | 35 |
| rno-miR-338 | tcaAcaAaaTcamCtgAtgmCtgGa | 73 | 33 |
| rno-miR-339 | tgAgcTccTggAggAcaGgga | 83 | 47 |
| rno-miR-340 | ggcTatAaaGtaActGagAcgGa | 72 | 34 |
| rno-miR-341 | amCtgAccGacmCgamCcgAtcGa | 84 | 53 |
| rno-miR-342 | gacGggTgcGatTtcTgtGtgAga | 82 | 34 |
| rno-miR-343 | tctGggmCacAcgGagGgaGa | 87 | 40 |
| rno-miR-344 | amCggTcaGgcTttGgcTagAtca | 81 | 63 |
| rno-miR-345 | gcActGgamCtaGggGtcAgca | 83 | 43 |
| rno-miR-346 | aGagGcaGgcActmCagGcaGaca | 86 | 37 |
| rno-miR-347 | tggGcgAccmCagAggGaca | 82 | 43 |
| rno-miR-349 | agaGgtTaaGacAgcAggGctg | 79 | 39 |
| rno-miR-34a | aamCaamCcaGctAagAcamCtgmCca | 80 | 27 |
| rno-miR-34b | caaTcaGctAatTacActGccTa | 71 | 40 |
| rno-miR-34c | gcAatmCagmCtaActAcamCtgmCct | 76 | 31 |
| rno-miR-350 | gTgaAagTgtAtgGgcTttGtgAa | 76 | 42 |
| rno-miR-351 | cagGctmCaaAggGctmCctmCagGga | 84 | 59 |
| rno-miR-352 | tamCtaTgcAacmCtamCtamCtct | 68 | 26 |
| rno-miR-421 | caAcaAacAttTaaTgaGgcc | 68 | 30 |
| rno-miR-7 | aacAaaAtcActAgtmCttmCca | 66 | 30 |
| rno-miR-7* | tatGgcAgamCtgTgaTttGttg | 73 | 45 |
| rno-miR-7b | aamCaaAatmCacAagTctTcca | 68 | 24 |
| rno-miR-9 | tcAtamCagmCtaGatAacmCaaAga | 71 | 34 |
| rno-miR-92 | cagGccGggAcaAgtGcaAta | 79 | 28 |
| rno-miR-93 | ctAccTgcAcgAacAgcActTtg | 77 | 31 |
| rno-miR-96 | aGcaAaaAtgTgcTagTgcmCaaa | 75 | 38 |
| rno-miR-98 | aAcaAtamCaamCttActAccTca | 67 | 17 |
| rno-miR-99a | cacAagAtcGgaTctAcgGgtt | 77 | 42 |
| rno-miR-99b | cgcAagGtcGgtTctAcgGgtg | 82 | 42 |
| zma-miR156a | gtgmCtcActmCtcTtcTgtmCa | 71 | 25 |
| zma-miR156b | gtgmCtcActmCtcTtcTgtmCa | 71 | 25 |
| zma-miR156c | gtgmCtcActmCtcTtcTgtmCa | 71 | 25 |
| zma-miR156d | gtgmCtcActmCtcTtcTgtmCa | 71 | 25 |
| zma-miR156e | gtgmCtcActmCtcTtcTgtmCa | 71 | 25 |
| zma-miR156f | gtgmCtcActmCtcTtcTgtmCa | 71 | 25 |
| zma-miR156g | gtgmCtcActmCtcTtcTgtmCa | 71 | 25 |
| zma-miR156h | gtgmCtcActmCtcTtcTgtmCa | 71 | 25 |
| zma-miR156i | gtgmCtcActmCtcTtcTgtmCa | 71 | 25 |
| zma-miR160a | tggmCatAcaGggAgcmCagGca | 85 | 49 |
| zma-miR160b | tggmCatAcaGggAgcmCagGca | 85 | 49 |
| zma-miR160c | tggmCatAcaGggAgcmCagGca | 85 | 49 |
| zma-miR160d | tggmCatAcaGggAgcmCagGca | 85 | 49 |
| zma-miR160e | tggmCatAcaGggAgcmCagGca | 85 | 49 |
| zma-miR162 | tggAtgmCagAggTttAtcGa | 73 | 28 |
| zma-miR164a | tgcAcgTgcmCctGctTctmCca | 82 | 46 |
| zma-miR164b | tgcAcgTgcmCctGctTctmCca | 82 | 46 |
| zma-miR164c | tgcAcgTgcmCctGctTctmCca | 82 | 46 |
| zma-miR164d | tgcAcgTgcmCctGctTctmCca | 82 | 46 |
| zma-miR166a | gGggAatGaaGccTggTccGa | 84 | 33 |
| zma-miR166b | gggAatGaaGccTggTccGa | 79 | 29 |
| zma-miR166c | gggAatGaaGccTggTccGa | 79 | 29 |
| zma-miR166d | gggAatGaaGccTggTccGa | 79 | 29 |
| zma-miR166e | gggAatGaaGccTggTccGa | 79 | 29 |
| zma-miR166f | gggAatGaaGccTggTccGa | 79 | 29 |
| zma-miR166g | gggAatGaaGccTggTccGa | 79 | 29 |
| zma-miR166h | gggAatGaaGccTggTccGa | 79 | 29 |
| zma-miR166i | gggAatGaaGccTggTccGa | 79 | 29 |
| zma-miR167a | tAgaTcaTgcTggmCagmCttmCa | 79 | 53 |
| zma-miR167b | tAgaTcaTgcTggmCagmCttmCa | 79 | 53 |
| zma-miR167c | tAgaTcaTgcTggmCagmCttmCa | 79 | 53 |
| zma-miR167d | tAgaTcaTgcTggmCagmCttmCa | 79 | 53 |
| zma-miR169a | tcGgcAagTcaTccTtgGctg | 78 | 40 |
| zma-miR169b | tcGgcAagTcaTccTtgGctg | 78 | 40 |
| zma-miR171a | ataTtgGcgmCggmCtcAatmCa | 76 | 46 |
| zma-miR171b | gtgAtaTtgGcamCggmCtcAa | 74 | 43 |
| zma-miR172a | tgmCagmCatmCatmCaaGatTct | 73 | 39 |
| zma-miR172b | tgmCagmCatmCatmCaaGatTct | 73 | 39 |
| zma-miR172c | tgmCagmCatmCatmCaaGatTct | 73 | 39 |
| zma-miRl72d | tgmCagmCatmCatmCaaGatTct | 73 | 39 |

### EXAMPLE 13

### Determination of microRNA expression in zebrafish embryonic development by whole mount in situ hybridization of embryos using LNA-substituted miRNA detection probes

### Zebrafish

Zebrafish were kept under standard conditions (M. Westerfield, The zebrafish book (University of Oregon Press, 1993). Embryos were staged according to (C. B. Kimmel, W. W. Ballard, S. R. Kimmel, B. Ullmann, T. F. Schilling, Dev Dyn 203, 253-310 (1995). Homozygous albino embryos and larvae were used for the in situ hybridizations.

### LNA-substituted microRNA probes

The sequences of the LNA-substituted microRNA probes are listed below. The LNA probes were labeled with digoxigenin (DIG) using a DIG 3'-end labeling kit (Roche) and purified using Sephadex G25 MicroSpin columns (Amersham). For in situ hybridizations approximately 1-2 pmol of labeled probe was used.

**Table 1. List of LNA-substituted detection probes for determination of microRNA expression in zebrafish embryonic development by whole mount in situ hybridization of embryos**

| LNA nucleotides are depicted by capital letters, DNA nucleotides by lowercase letters, mC denotes LNA methyl-cytosine. | | |
|---|---|---|
| **Probe name** | **Probe sequence 5'-3'** | **Calc Tm °C** |
| hsa-let7f/LNA | aamCtaTacAatmCtamCtamCctmCa | 67 |
| hsa-miR19b/LNA | tmCagTttTgcAtgGatTtgmCaca | 75 |
| hsa-miR17-5p/LNA | actAccTgcActGtaAgcActTtg | 74 |
| hsa-miR217/LNA | atcmCaaTcaGttmCctGatGcaGta | 75 |
| hsa-miR218/LNA | acAtgGttAgaTcaAgcAcaa | 70 |
| hsa-miR222/LNA | gaGacmCcaGtaGccAgaTgtAgct | 80 |
| hsa-let7i/LNA | agmCacAaamCtamCtamCctmCa | 71 |
| hsa-miR27b/LNA | cagAacTtaGccActGtgAa | 68 |
| hsa-miR301/LNA | gctTtgAcaAtamCtaTtgmCacTg | 70 |
| hsa-miR30b/LNA | gcTgaGtgTagGatGttTaca | 70 |
| hsa-miR100/LNA | cacAagTtcGgaTctAcgGgtt | 77 |
| hsa-miR34a/LNA | aamCaamCcaGctAagAcamCtgmCca | 80 |
| hsa-miR7/LNA | aacAaaAtcActAgtmCttmCca | 66 |
| hsa-miR125b/LNA | tcamCaaGttAggGtcTcaGgga | 77 |
| hsa-miR133a/LNA | acAgcTggTtgAagGggAccAa | 82 |
| hsa-miR101/LNA | cttmCagTtaTcamCagTacTgta | 68 |
| hsa-miR108/LNA | aatGccmCctAaaAatmCctTat | 66 |
| hsa-miR107/LNA | tGatAgcmCctGtamCaaTgcTgct | 80 |
| hsa-miR153/LNA | tcamCttTtgTgamCtaTgcAa | 68 |
| hsa-miR10b/LNA | amCaaAttmCggTtcTacAggGta | 73 |
| mmu-miR10b/LNA | acamCaaAttmCggTtcTacAggg | 73 |
| hsa-miR194/LNA | tccAcaTggAgtTgcTgtTaca | 75 |
| hsa-miR199a/LNA | gaAcaGgtAgtmCtgAacActGgg | 78 |
| hsa-miR199a*/LNA | aacmCaaTgtGcaGacTacTgta | 74 |
| hsa-miR20/LNA | ctAccTgcActAtaAgcActTta | 70 |
| hsa-miR214/LNA | ctGccTgtmCtgTgcmCtgmCtgt | 81 |
| hsa-miR219/LNA | agAatTgcGttTggAcaAtca | 70 |
| hsa-miR223/LNA | gGggTatTtgAcaAacTgamCa | 73 |
| hsa-miR23a/LNA | gGaaAtcmCctGgcAatGtgAt | 76 |
| hsa-miR24/LNA | cTgtTccTgcTgaActGagmCca | 80 |
| hsa-miR26a/LNA | agcmCtaTccTggAttActTgaa | 70 |
| hsa-miR126/LNA | gcAttAttActmCacGgtAcga | 71 |
| hsa-miR126*/LNA | cgmCgtAccAaaAgtAatAatg | 68 |
| hsa-miR128a/LNA | aaAagAgamCcgGttmCacTgtGa | 77 |
| mmu-miR7b/LNA | aamCaaAatmCacAagTctTcca | 68 |
| hsa-let7c/LNA | aamCcaTacAacmCtamCtamCctmCa | 74 |
| hsa-let7b/LNA | aamCcamCacAacmCtamCtamCctmCa | 77 |
| hsa-miR103/LNA | tmCatAgcmCctGtamCaaTgcTgct | 80 |
| hsa-miR129/LNA | agcAagmCccAgamCcgmCaaAaag | 80 |
| rno-miR129*/LNA | aTgcTttTtgGggTaaGggmCtt | 78 |
| hsa-miRl30a/LNA | gcmCctTttAacAttGcamCtg | 70 |
| hsa-miR132/LNA | cgAccAtgGctGtaGacTgtTa | 76 |
| hsa-miR135a/LNA | tcamCatAggAatAaaAagmCcaTa | 69 |
| hsa-miR137/LNA | cTacGcgTatTctTaaGcaAta | 68 |
| hsa-miR200a/LNA | acaTcgTtamCcaGacAgtGtta | 72 |
| hsa-miR142-3p/LNA | tmCcaTaaAgtAggAaamCacTaca | 72 |
| hsa-miR142-5p/LNA | gtaGtgmCttTctActTtaTg | 63 |
| hsa-miR181b/LNA | aamCccAccGacAgcAatGaaTgtt | 81 |
| hsa-miR183/LNA | caGtgAatTctAccAgtGccAta | 73 |
| hsa-miR190/LNA | acmCtaAtaTatmCaaAcaTatmCa | 62 |
| hsa-miR193/LNA | ctGggActTtgTagGccAgtt | 76 |
| hsa-miR19a/LNA | tmCagTttTgcAtaGatTtgmCaca | 72 |
| hsa-miR204/LNA | cagGcaTagGatGacAaaGggAa | 78 |
| hsa-miR205/LNA | caGacTccGgtGgaAtgAagGa | 81 |
| hsa-miR216/LNA | camCagTtgmCcaGctGagAtta | 74 |
| hsa-miR221/LNA | gAaamCccAgcAgamCaaTgtAgct | 80 |
| hsa-miR25/LNA | tcaGacmCgaGacAagTgcAatg | 77 |
| hsa-miR29c/LNA | taamCcgAttTcaAatGgtGcta | 70 |
| hsa-miR29b/LNA | amCacTgaTttmCaaAtgGtgmCta | 71 |
| hsa-miR30c/LNA | gmCtgAgaGtgTagGatGttTaca | 73 |
| hsa-miR140/LNA | ctAccAtaGggTaaAacmCact | 71 |
| hsa-miR9*/LNA | acTttmCggTtaTctAgcTtta | 65 |
| hsa-miR92/LNA | amCagGccGggAcaAgtGcaAta | 81 |
| hsa-miR96/LNA | aGcaAaaAtgTgcTagTgcmCaaa | 75 |
| hsa-miR99a/LNA | cacAagAtcGgaTctAcgGgtt | 77 |
| hsa-miR145/LNA | aAggGatTccTggGaaAacTggAc | 79 |
| hsa-miR155/LNA | ccmCctAtcAcgAttAgcAttAa | 71 |
| hsa-miR29a/LNA | aamCcgAttTcaAatGgtGctAg | 75 |
| rno-miR140*/LNA | gtcmCgtGgtTctAccmCtgTggTa | 81 |
| hsa-miR206/LNA | ccamCacActTccTtamCatTcca | 73 |
| hsa-miR124a/LNA | tggmCatTcamCcgmCgtGccTtaa | 80 |
| hsa-miR122a/LNA | acAaamCacmCatTgtmCacActmCca | 78 |
| hsa-miR1/LNA | tamCatActTctTtamCatTcca | 64 |
| hsa-miR181a/LNA | acTcamCcgAcaGcgTtgAatGtt | 77 |
| hsa-miR10a/LNA | cAcaAatTcgGatmCtamCagGgta | 74 |
| hsa-miR196a/LNA | ccaAcaAcaTgaAacTacmCta | 67 |
| hsa-let7a/LNA | aamCtaTacAacmCtamCtamCctmCa | 70 |
| hsa-miR9/LNA | tcAtamCagmCtaGatAacmCaaAga | 71 |
| hsa-miR210/LNA | agcmCgcTgtmCacAcgmCacAg | 84 |
| hsa-miR144/LNA | taGtamCatmCatmCtaTacTgta | 64 |
| hsa-miR338/LNA | caAcaAaaTcamCtgAtgmCtgGa | 72 |
| hsa-miR187/LNA | ggcTgcAacAcaAgamCacGa | 79 |
| hsa-miR200b/LNA | cAtcAttAccAggmCagTatTaga | 71 |
| hsa-miR184/LNA | cmCctTatmCagTtcTccGtcmCa | 75 |
| hsa-miR27a/LNA | gcGgaActTagmCcamCtgTgaa | 77 |
| hsa-miR215/LNA | ctgTcaAttmCatAggTcat | 65 |
| hsa-miR203/LNA | agTggTccTaaAcaTttmCac | 68 |
| hsa-miRl6/LNA | ccaAtaTttAcgTgcTgcTa | 68 |
| hsa-miR152/LNA | aAgtTctGtcAtgmCacTga | 72 |
| hsa-miR138/LNA | gatTcamCaamCacmCagmCt | 70 |
| hsa-miR143/LNA | gagmCtamCagTgcTtcAtcTca | 72 |
| hsa-miR195/LNA | gmCcaAtaTttmCtgTgcTgcTa | 73 |
| hsa-mir375/LNA | tAacGcgAgcmCgaAcgAacAaa | 79 |
| dre-miR93/LNA | ctAccTgcAcaAacAgcActTt | 73 |
| dre-miR22/LNA | acaGttmCttmCagmCtgGcaGctt | 76 |
| dre-miR213/LNA | gGtamCagTcaAcgGtcGatGgt | 80 |
| dre-miR31/LNA | cagmCtaTgcmCaamCatmCttGcc | 76 |
| dre-miR189/LNA | amCtgTtaTcaGctmCagTagGcac | 75 |
| dre-miR18/LNA | tatmCtgmCacTaaAtgmCacmCtta | 69 |
| dre-miR15a/LNA | cAcaAacmCatTctGtgmCtgmCta | 74 |
| dre-miR34b/LNA | cAatmCagmCtaAcaAcamCtgmCcta | 74 |
| dre-miR148a/LNA | acaAagTtcTgtAatGcamCtga | 69 |
| dre-miR125a/LNA | camCagGttAagGgtmCtcAggGa | 80 |
| dre-miR139/LNA | agAcamCatGcamCtgTaga | 69 |
| dre-miR150/LNA | cacTggTacAagGatTggGaga | 75 |
| dre-miR192/LNA | ggcTgtmCaaTtcAtaGgtmCa | 73 |
| dre-miR98/LNA | aacAacAcaActTacTacmCtca | 68 |
| dre-let7g/LNA | amCtgTacAaamCaamCtamCctmCa | 73 |
| dre-miR30a-5p/LNA | gctTccAgtmCggGgaTgtTtamCa | 80 |
| dre-miR26b/LNA | aacmCtaTccTggAttActTgaa | 68 |
| dre-miR21/LNA | cAacAccAgtmCtgAtaAgcTa | 72 |
| dre-miR146/LNA | accmCttGgaAttmCagTtcTca | 72 |
| dre-miR182/LNA | tgtGagTtcTacmCatTgcmCaaa | 72 |
| dre-miR182*/LNA | taGttGgcAagTctAgaAcca | 72 |
| dre-miR220/LNA | aAgtGtcmCgaTacGgtTgtGg | 81 |
| hsa-miR138/LNA | gatTcamCaamCacmCagmCt | 70 |
| dre-miR141/LNA | gcaTcgTtamCcaGacAgtGtt | 74 |
| hsa-miR143/LNA | gagmCtamCagTgcTtcAtcTca | 72 |
| hsa-miR195/LNA | gmCcaAtaTttmCtgTgcTgcTa | 73 |
| dre-mir-30a-3p/LNA | acaGcaAacAtcmCaamCtgAaag | 72 |
| hsa-mir375/LNA | tAacGcgAgcmCgaAcgAacAaa | 79 |

### Whole-mount in situ hybridizations

Whole-mount in situ hybridizations were performed essentially as described (B. Thisse et al., Methods Cell Biol 77, 505-19 (2004).), with the following modifications: Hybridization, washing and incubation steps were done in 2.0 ml eppendorf tubes. All PBS and SSC solutions contained 0.1% Tween (PBST and SSCT). Embryos of 12, 16, 24, 48, 72 and 120 hpf were treated with proteinase K for 2, 5, 10, 30, 45 and 90 min, respectively. After proteinase K treatment and refixation with 4% paraformaldehyde, endogenous alkaline phosphatase activity was blocked by incubation of the embryos in 0.1 M ethanolamine and 2.5% acetic anhydride for 10 min, followed by extensive washing with PBST. Hybridizations were performed in 200 µl of hybridization mix. The temperature of hybridization and subsequent washing steps was adjusted to approximately 22°C below the predicted melting temperatures of the LNA-modified probes. Staining with NBT/BCIP was done overnight at 4°C. After staining, the embryos were fixed overnight in 4% paraformaldehyde. Next, embryos were dehydrated in an increasing methanol series and subsequently placed in a 2:1 mixture of benzyl benzoate and benzyl alcohol. Embryos were mounted on a hollow glass slide and covered with a coverslip.

### Plastic sectioning

Embryos and larvae stained by whole-mount in situ hybridization were transferred from benzyl benzoate/benzyl alcohol to 100% methanol and incubated for 10 min. Specimens were washed twice with 100% ethanol for 10 min and incubated overnight in 100% Technovit 8100 infiltration solution (Kulzer) at 4°C. Next, specimens were transferred to a mold and embedded overnight in Technovit 8100 embedding medium (Kulzer) deprived of air at 4°C. Sections of 7 µm thickness were cut with a microtome (Reichert-Jung 2050), stretched on water and mounted on glass slides. Sections were dried overnight. Counterstaining was done by 0.05% neutral red for 12 sec, followed by extensive washing with water. Sections were preserved with Pertex and mounted under a coverslip.

### Image acquisition

Embryos and larvae stained by whole-mount in situ hybridization were analyzed with Zeiss Axioplan and Leica MZFLIII microscopes and subsequently photographed with digital cameras. Sections were analyzed with a Nikon Eclipse E600 microscope and photographed with a digital camera (Nikon, DXM1200). Images were adjusted with Adobe Photoshop 7.0 software.

**Table 2. MicroRNA expression patterns in zebrafish embryonic development determined by whole mount in situ hybridization of embryos using LNA-substituted miRNA detection probes.**

| **MicroRNA** | **Class*** | **In situ expression pattern in zebrafish** |
|---|---|---|
| miR-1 | A | Body, head and fin muscles |
| miR-122a | A | Liver; pancreas |
| miR-124a | A | Differentiated cells of brain; spinal cord and eyes; cranial ganglia |
| miR-128a | A | Brain (specific neurons in fore- mid- and hindbrain); spinal cord; cranial nerves/ganglia |
| miR-133a | A | Body, head and fin muscles |
| miR-138 | A | Outflow tract of the heart; brain; cranial nerves/ganglia; undefin. bilateral structure in head;neurons in spinal cord |
| miR-144 | A | Blood |
| miR-194 | A | Gut and gall bladder; liver; pronephros |
| miR-206 | A | Body, head and fin muscles |
| miR-219 | A | Brain (mid- and hindbrain); spinal cord |
| miR-338 | A | Lateral line; cranial ganglia |
| miR-9 | A | Proliferating cells of brain, spinal cord and eyes |
| miR-9* | A | Proliferating cells of brain, spinal cord and eyes |
| miR-200a | A | Nose epithelium; lateral line organs; epidermis; gut (proctodeum); taste buds |
| miR-132 | A | Brain (specific neurons in fore- and midbrain) |
| miR-142-5p | A | Thymic primordium |
| miR-7 | A | Neurons in forebrain; diencephalon/hypothalamus; pancreatic islet |
| miR-143 | A | Gut and gall bladder; swimbladder; heart; nose |
| miR-145 | A | Gut and gall bladder; gills; swimbladder; branchial arches; fins; outflow tract of the heart; ear |
| miR-181a | A | Brain (tectum, telencephalon); eyes; thymic primordium; gills |
| miR-181b | A | Brain (tectum, telencephalon); eyes; thymic primordium; gills |
| miR-215 | A | Gut and gall bladder |
| let-7a | A | Brain; spinal cord |
| let-7b | A | Brain; spinal cord |
| miR-125a | A | Brain; spinal cord; cranial ganglia |
| miR-125b | A | Brain; spinal cord; cranial ganglia |
| miR-142-3p | A | Thymic primordium; blood cells |
| miR-200b | A | Nose epithelium; lateral line organs; epidermis; gut (proctodeum); taste buds |
| miR-218 | A | Brain (neurons and/or cranial nerves/ganglia in hindbrain); spinal cord |
| miR-222 | A | Neurons and/or cranial ganglia in forebrain and midbrain; rhombomere in early stages |
| miR-23a | A | Pharyngeal arches; oral cavity; posterior tail; cardiac valves |
| miR-27a | A | Undefined structures in branchial arches; tip of tail in early stages |
| miR-34a | A | Brain (cerebellum); neurons in spinal cord |
| miR-375 | A | Pituitary gland; pancreatic islet |
| miR-99a | A | Brain (hindbrain, diencephalon); spinal cord |
| let-7i | A | Brain (tectum, diencephalon) |
| miR-100 | A | Brain (hindbrain, diencephalon); spinal cord |
| miR-103 | A | Brain; spinal cord |
| miR-107 | A | Brain; spinal cord |
| miR-126 | A | Bloodvessels and heart |
| miR-137 | A | Brain (neurons and/or cranial nerves/ganglia in fore-, mid- and hindbrain); spinal cord |
| miR-140 | A | Cartilage of pharyngeal arches,head skeleton and fins |
| miR-140* | A | Cartilage of pharyngeal arches,head skeleton and fins |
| miR-141 | A | Nose epithelium; lateral line organs; epidermis; gut (proctodeum); taste buds |
| miR-150 | A | Cardiac valves; undefined structures in epithelium of branchial arches |
| miR-182 | A | Nose epithelium; haircells of lateral line organs and ear; cranial ganglia; rods, cones and bipolar cells of eye; epiphysis |
| miR-183 | A | Nose epithelium; haircells of lateral line organs and ear; cranial ganglia; rods, cones and bipolar cells of eye; epiphysis |
| miR-184 | A | Lens; hatching gland in early stages |
| miR-199a | A | Epithelia surrounding cartilage of pharyngeal arches, oral cavity and pectoral fins; epidermis of head; tailbud |
| miR-199a* | A | Epithelia surrounding cartilage of pharyngeal arches, oral cavity and pectoral fins; epidermis of head; tailbud |
| miR-203 | A | Most outer layer of epidermis |
| miR-204 | A | Neural crest; pigment cells of skin and eye; swimbladder |
| miR-205 | A | Epidermis; epithelia of branchial arches; intersegmental cells; not in sensory epithelia |
| miR-221 | A | Brain (Neurons and/or cranial ganglia in forebrain and midbrain; rhombomere in early stages) |
| miR-7b | A | Brain (fore-, mid- and hindbrain); spinal cord |
| miR-96 | A | Nose epithelium; haircells of lateral line organs and ear; cranial ganglia; rods, cones and bipolar cells of eye; epiphysis |
| miR-217 | B | Brain (tectum, hindbrain); spinal cord; proliferative cells of eyes; pancreas |
| miR-126* | B | ND |
| miR-31 | B | Ubiquitous |
| miR-216 | B | Brain (tectum); spinal cord; proliferative cells of eyes; pancreas; body muscles |
| miR-30a-5p | B | Pronephros; cells in epidermis; lens in early stages |
| miR-153 | B | Brain (fore- mid- and hindbrain, diencephalon/hypothalamus) |
| miR-15a | C | Ubiquitous (head, spinal cord, gut, outline somites, neuromasts) |
| miR-17-5p | C | Ubiquitous (head, spinal cord, gut, outline somites, neuromasts) |
| miR-18 | C | Ubiquitous (head, spinal cord, gut, outline somites, neuromasts) |
| miR-195 | C | Ubiquitous |
| miR-19b | C | Ubiquitous (head, spinal cord, gut, outline somites, neuromasts) |
| miR-20 | C | Ubiquitous (head, spinal cord, gut, outline somites, neuromasts) |
| miR-26a | C | Ubiquitous (head, spinal cord, gut, outline somites, neuromasts) |
| miR-92 | C | Ubiquitous (head, spinal cord, gut, outline somites, neuromasts) |
| let-7c | C | Brain; spinal cord |
| miR-101 | C | ND |
| miR-16 | C | Brain |
| miR-21 | C | Cardiac valves; otoliths in ear; rhombomere in early stages |
| miR-30b | C | Pronephros; cells in epidermis |
| miR-30c | C | Pronephros; cells in epidermis and epithelia of branchial arches; neurons in hindbrain |
| miR-26b | C | Ubiquitous (head, spinal cord, gut, outline somites, neuromasts) |
| let-7g | C | Ubiquitous (head, spinal cord, gut, outline somites, neuromasts) |
| miR-19a | C | Ubiquitous (head, spinal cord, gut, outline somites, neuromasts) |
| miR-210 | C | Ubiquitous (head, spinal cord, gut, outline somites, neuromasts) |
| miR-22 | C | Ubiquitous |
| miR-25 | C | Ubiquitous (head, spinal cord, gut, outline somites, neuromasts) |
| miR-93 | C | Ubiquitous (head, spinal cord, gut, outline somites, neuromasts) |
| miR-189 | D | ND |
| miR-30a-3p | D | ND |
| miR-34b | D | Cells in pronephric duct; nose |
| miR-129* | D | ND |
| miR-135a | D | ND |
| miR-182* | D | ND |
| miR-187 | D | ND |
| miR-220 | D | ND |
| miR-301 | D | ND |
| miR-223 | D | ND |
| let-7f | - | Brain; spinal cord |
| miR-108 | - | Ubiquitous |
| miR-10a | - | Posterior trunk; later restricted to spinal cord |
| miR-10b | - | Posterior trunk; later restricted to spinal cord |
| miR-129 | - | Brain |
| miR-130a | - | ND |
| miR-139 | - | Nose; neuromasts |
| miR-146 | - | Neurons in forebrain; branchial arches and head skeletion |
| miR-148a | - | ND |
| miR-152 | - | Ubiquitous |
| miR-155 | - | ND |
| miR-190 | - | ND |
| miR-193 | - | ND |
| miR-196a | - | Posterior trunk; later restricted to spinal cord |
| miR-213 | - | Nose (epithelium or olfactory neurons), eyes (ganglion cell layer) |
| miR-214 | - | Epithelia surrounding cartilage of pharyngeal arches, oral cavity |
| | | and pectoral fins; epidermis of head; tailbud |
| miR-24 | - | Pharyngeal arches; oral cavity; posterior tail; cardiac valves |
| miR-27b | - | Cells in branchial arches |
| miR-29a | - | ND |
| miR-29b | - | ND |
| miR-29c | - | ND |
| miR-98 | - | Brain |

| | | |
|---|---|---|
| * Main class in which expression patterns were compared: A, specific expression; B, marginal specific expression or very low absolute expression; C, ubiquitous expression. D, no detectable expression. | | |

Wienholds et al., Science, 2005, 309, 310-311 (published after the effective date of the data above) relates to the findings referred to in Table 2 - that reference also includes a number of figures which visually demonstrates the tissue distribution of a number of miRNAs. Wienholds *et al.* is consequently incorporated by reference herein.

**Table 3. List of LNA-substituted detection probes useful as specificity controls in detection of vertebrate microRNAs.**

| LNA nucleotides are depicted by capital letters, DNA nucleotides by lowercase letters, mC denotes LNA methyl-cytosine. | | |
|---|---|---|
| **Probe name** | **Sequence 5'-3'** | **Self-comp score** |
| hsa-miR206/LNA/2MM | ccamCacActmCtcTtamCatTcca | 8 |
| hsa-miR206/LNA/1MM | ccamCacActmCccTtamCatTcca | 8 |
| hsa-miRl24a/LNA/2MM | tggmCatTcaAagmCgtGccTtaa | 60 |
| hsa-miR124a/LNA/1MM | tggmCatTcaAcgmCgtGccTtaa | 60 |
| hsa-miR122a/LNA/2MM | acAaamCacmCacmCgtmCacActmCca | 18 |
| hsa-miR122a/LNA/1MM | acAaamCacmCatmCgtmCacActmCca | 18 |

The above demonstrates that it is possible to map an animal's miRNA against various tissues, and it is thus possible to determine the origin of a cell based on a determination of miRNA from said cell.

This has interesting implications. As mentioned above, it is a known clinical problem to determine the exact origin of a number of metastatic cancers and this has several consequences. First of all, it is not possible to locate the primary tumour (which may be much smaller than the metastatic tumour which has been detected), but it is in such cases also difficult if not impossible to determine the optimum treatment because of lack of knowledge of the tissue origin of the primary tumour.

Cancer of unknown primary site is a common clinical entity, accounting for 2% of all cancer diagnoses in the Surviellance, Epidemiology, and End Results (SEER) registries between 1973 and 1987 (C. Muir. Cancer of unknown primary site Cancer 1995. 75: 353-356). In spite of the frequency of this syndrome, relatively little attention has been given to this group of patients, and systematic study of the entity has lagged behind that of other areas in oncology. Widespread pessimism concerning the therapy and prognosis of these patients has been the major reason for the lack of effort in this area. The patient with carcinoma of unknown primary site is commonly stereotyped as an elderly, debilitated individual with metastases at multiple visceral sites. Early attempts at systemic therapy yielded low response rates and had a negligible effect on survival, thereby strengthening arguments for a nihilistic approach to these patients. The heterogeneity of this group has also made the design of therapeutic studies difficult; it is well recognized that cancers with different biologies from many primary sites are represented. In the past 10 years, substantial improvements have been made in the management and treatment of some patients with carcinoma of unknown primary site. The identification of treatable patients within this heterogeneous group has been made possible by the recognition of several clinical syndromes that predict chemotherapy responsiveness, and also by the development of specialized pathologic techniques that can aid in tumor characterization. Therefore, the optimal management of patients with cancer of unknown primary site now requires appropriate clinical and pathologic evaluation to identify treatable subgroups, followed by the administration of specific therapy. Many patients with adenocarcinoma of unknown primary site have widespread metastases and poor performance status at the time of diagnosis. The outlook for most of these patients is poor, with median survival of 4 to 6 months. However, subsets of patients with a much more favorable outlook are contained within this large group, and optimal initial evaluation enables the identification of these treatable subsets. In addition, empiric chemotherapy incorporating newer agents has produced higher response rates and probably improves the survival of patients with good performance status.

Fine-needle aspiration biopsy (FNA) provides adequate amounts of tissue for definitive diagnosis of poorly differentiated tumors, and identification of the primary source in about one fourth of cases (C.V. Reyes, K.S. Thompson, J.D. Jensen, and A.M. Chouelhury.

Metastasis of unknown origin: the role of fine needle aspiration cytology Diagn Cytopathol 1998. 18: 319-322).

As one example, most patients with squamous cell carcinoma involving inguinal lymph nodes have a detectable primary site in the genital or anorectal area. In women, careful examination of the vulva, vagina, and cervix is important, with biopsy of any suspicious areas. Men should undergo a careful inspection of the penis. Digital examination and anoscopy should be performed in both sexes to exclude lesions in the anorectal area. Identification of a primary site in these patients is important, since curative therapy is available for carcinomas of the vulva, vagina, cervix, and anus even after they spread to regional lymph nodes. For the occasional patient in whom no primary site is identified, surgical resection with or without radiation therapy to the inguinal area sometimes results in long-term survival (A. Guarischi, T.J. Keane, and T. Elhakim. Metastatic inguinal nodes from an unknown primary neoplasm. A review of 56 cases Cancer 1987. 59: 572-577). Hence, clearly it is advantageous to be able to determine the origin of tumors and improved recognition of treatable subsets within the large heterogeneous population of patients with carcinoma of unknown primary site would represents a definite advance in the management and treatment of these patients. This will also allow treatable subsets to be defined with appropriate clinical and pathologic evaluation; Table X provides a summary of currently known subsets of carcinomas of unknown origin and outlines the recommended evaluation and treatment of. Clearly, identifying the primary site in cases of metastatic carcinoma of unknown origin has profound clinical importance in managing cancer patients. Currently, identification of the site of origin of a metastatic carcinoma is time consuming and often requires expensive whole-body imaging or invasive exploratory surgery.

**Table X**

| Histopathology | Clinical Evaluation (in addition to history. Physical exam, routine laboratory, chest radiography) | Special Pathologic Studies | Specific Subsets for Therapy | Therapy |
|---|---|---|---|---|
| Adenocarcinoma (well-differentiated or moderately differentiated) | CT scan of abdomen | Men: PSA stain | 1) Women, axillary node involvement | Treat as primary breast cancer |
| | Men: serum PSA | Women: ER, PR stain | | |
| | Women: Mammograms | | 2) Women, peritoneal carcinomatosis | Treat as stage III prostate cancer |
| | Additional studies to evaluate signs, symptoms | | 3) Men, blastic bone metastases, or high serum | Treat as stage IV prostate cancer |
| | | | PSA or tumor PSA staining | |
| | | | 4) Solitary metastatic lesion | Definitive local therapy |
| Squarrous carcinoma | Cervical presentation: Direct laryngoscopy, nasopharyngoscopy,bronchoscopy | - | Cervical adenopathy | Treat as locally advanced head/neck cancer |
| | | | Inguinal adenopathy | Inguinal LND ± radiation therapy |
| Poorly differentiated carcinoma | CT abdomen, chest Serum, HCG, AFP Additional studies to evaluate signs, symptoms | Immunoperoxidase staining, electron microscopy, cytogenetic studies | 1) Features of EGCT | Treat as nonseminomatous ECGT |
| | | | 2) Other patients | Empiric platinum or paclitaxel/platmum regimen |
| Neuroendocrine carcinoma | CT abdomen, chest Additional studies to evaluate signs, symptoms | Immunoperoxidase staining | 1) Low grade | Treat as advanced carcinoid tumor |
| | | | 2) Small cell carcinoma | Empiric platinum/etoposide or platinum/etoposide/paclitaxel |
| | | | 3) Poorly differentiated | |
| CT = computed tomography, PSA=prostate-specific antigen; HCG=human chorionic gonadotropin; AFP alpha-fetoprotein; ER estrogen receptor; PR=progesterone receptor; EGCT = extragonadal germcell tumor; LND = lymph node dissection. | | | | |

As previously described, microRNAs have emerged as important non-coding RNAs, involved in a wide variety of regulatory functions during cell growth, development and differentiation. Some reports clearly indicate that microRNA expression may be indicative of cell differentiation state, which again is an indication of organ o tissue specification. This finding has been confirmed in the experiments using LNA FISH probes on whole mount preparations in different developmental stages in zebra fish, where a large number of microRNAs display a very distinct tissue or organ-specific distribution. As outlined in the figures herein and in summary in table 2 many microRNAs are expressed only in single organs or tissues. For example, mir-122a is expressed primarily in liver and pancreas, mir-215 is expressed primarily in gut and gall bladder, mir-204 is primarily expressed in the neural crest, in pigment cells of skin and eye and in the swimbladder, mir-142-5p in the thymic primordium etc. This catalogue of mir tissue expression profiles may serve as the basis for a diagnostic tool determining the tissue origin of tumors of unknown origin. If, for example a tumour sample from a given sample expresses a microRNA typical of another tissue type, this may be predictive of the tumour origin. For example, if a lymph cancer type expresses microRNA markers characteristic of liver cells (eg. Mir-122a), this may be indicative that the primary tumour resides within the liver. Hence, the detailed microRNA expression pattern in zebrafish provided may serve as the basis for a diagnostic measurement of clinical tumour samples providing valuable information about tumour origin.

So, since it is possible to map miRNA in cells vs. the tissue origin of these cells, the present invention presents a convenient means for detection of tissue origin of such tumours.

Hence, the present invention in general relates to a method for determining tissue origin of tumours comprising probing cells of the tumour with a collection of probes which is capable of mapping miRNA to a tissue origin.

### EXAMPLE 14

### Detection of microRNAs by in situ hybridization in paraffin-embedded mouse brain sections using 3' digoxigenin-labeled LNA probe

### A. Deparaffinization of the sections

(i) xylene 3x 5min, (ii) ethanol 100% for 2x 5min, ethanol 70% for 5min, ethanol 50% for 5min, ethanol 25% for 5min and in DEPC-treated water for 1min.

### B. Deproteinization of sections

(i) 2x 5min in PBS; 5min in Proteinase K at 10ug/ml at 37oC (add Prot.K 20mg/ml to warm Prot.K buffer 20 min before incubation); 30sec in 0.2% Glycine in PBS and 2x30sec in PBS.

### C. Fixation

### Sections were fixed for 10 min in 4% PFA, and the slides rinsed 2x in PBS

### D. Prehybridization

Prehybridization was carried out for 2 hours at the final hybridization temperature (ca 22 degrees below the predicted Tm of the LNA probe) in hybridization buffer (50%Formamide, 5xSSC, 0.1%Tween, 9.2mM citric acid for adjustment to pH6, 50ug/ml heparin, 500ug/ml yeast RNA) in a humidified chamber (50% formamide, 5xSSC). Use DAKO Pen.

### E. Hybridization

The 3' DIG-labeled LNA probe was diluted to 20 nM in hybridization buffer and 200µl of hybridization mixture was added per slide. The slides were hybridized overnight covered with Nescofilm in a humidified chamber. The slides were rinsed in 2x SCC and then washed at hybridization temperature 3 times 30 min in 50% formamide, 2xSSC, and finally 5x 5 min in PBST at room temperature.

### F. Immunological Detection

The slides were blocked for 1 hour in blocking buffer (2% sheep serum, 2mg/ml BSA in PBST) at room temperature, incubated overnight with anti-DIG antibody (1:2000 anti-DIG-AP Fab fragments in blockingbuffer) in a humidified chamber at 4°C, washed 5-7 times 5 min in PBST and 3 times 5 min in AP buffer (see below).

### G. Colour reaction (room temperature, in dark)

The light-sensitive colour reaction (NBT/BCIP) was carried out for 1h-48h (400ul/slide) in a humidified chamber; the slides were washed for 3x 5 min in PBST, and mounted in aqeous mounting medium (glycerol) or dehydrate and mount in Entellan.

The results are shown in Figs. 5 and 6. It surprisingly appears that it is possible to detect target nucleotide sequences in these paraffin embedded sections. Previously it has been noted that it is very difficult to utilise fixated and embedded sections for hybridization assays. This is due to a variety of factor: First of all, RNA is degraded over time, so the use of long hybridization probes to detect RNA becomes increaingly difficult over time. Secondly, the very structure of a fixated and embedded section is such that it appears to be diffucult for hybridization probes to contact their target sequences.

Without being limited to any theory, it is believed that the short hybridization probes of the present invention overcome these disadvantages by being able to diffuse readily in a fixated and embedded section and by being able to hybridize with short fragments of degraded RNA still present in the section.

It should be noted that the present finding also opens for the possibility of detecting DNA in archived fixated and embedded samples. It is then e.g. possible, when using the short but highly specific probes of the present invention, to detect e.g. viral DNA in such aged samples, a possibility which to the best of the inventors' knowledge has not been available prior to the findings in the present invention.

### H. Buffers used in example 14.

H1. AP buffer
100ml Tris (100mM) 12.1g/l
20ml 5M NaCl (100mM) 5.84g/l
5ml 1M MgCl2 (5mM)
700ml sterile H2O, pH 9.5 and fill up to 1liter
H2. Colour solution (Light sensitive)
45ul 75mg/ml NBT (in 70% dimethylformamide)
35ul 50mg/ml BCIP-phosphate (in 100% dimethylformamide)
2.4mg Levamisole
in 10 ml AP buffer.

### EXAMPLE 15

### Specificity and sensitivity assessment of microRNA detection in zebrafish, Xenopus laevis and mouse by whole mount in situ hybridization of embryos using LNA-substituted miRNA detection probes

### Experimental material

Zebrafish, mouse and Xenopus tropicalis were kept under standard conditions. For all in situ hybridizations on zebrafish we used 72 hour old homozygous albino embryos. For Xenopus tropicalis 3 day old embryos were used and for mouse we used 9.5 or 10.5 dpc embryos.

### Design and synthesis of LNA-modified oligonucleotide probes

The LNA-modified DNA oligonucleotide probes are listed in Table 15-I. LNA probes were labeled with digoxigenin-ddUTP using the 3'-end labeling kit (Roche) according to the manufacturers recommendations and purified using sephadex G25 MicroSpin columns (Amersham).

**Table 15-I. List of short LNA-substituted detection probes for detection of microRNA expression in zebrafish by whole mount in situ hybridization of embryos**

| LNA nucleotides are depicted by capital letters, DNA nucleotides by lowercase letters, mC denotes LNA methyl-cytosine. | | |
|---|---|---|
| Probe name | Sequence 5'-3' | Calc Tm |
| hsa-miR124a/LNA | tggmCatTcamCcgmCgtGccTtaa | 80 |
| hsa-miR124a/LNA-2 | gmCatTcamCcgmCgtGccTtaa | 78 |
| hsa-miR124a/LNA-4 | atTcamCcgmCgtGccTtaa | 72 |
| hsa-miR124a/LNA-6 | TcamCcgmCgtGccTtaa | 71 |
| hsa-miR124a/LNA-8 | amCcgmCgtGccTtaa | 70 |
| hsa-miR124a/LNA-10 | cgmCgtGccTtaa | 60 |
| hsa-miR124a/LNA-12 | mCgtGccTtaa | 46 |
| hsa-miR124a/LNA-14 | tGccTtaa | 27 |
| hsa-miR206/LNA | ccamCacActTccTtamCatTcca | 73 |
| hsa-miR206/LNA-2 | amCacActTccTtamCatTcca | 70 |
| hsa-miR206/LNA-4 | acActTccTtamCatTcca | 64 |
| hsa-miR206/LNA-6 | ActTccTtamCatTcca | 58 |
| hsa-miR206/LNA-8 | tTccTtamCatTcca | 55 |
| hsa-miR206/LNA-10 | ccTtamCatTcca | 49 |
| hsa-miR206/LNA-12 | TtamCatTcca | 35 |
| hsa-miR206/LNA-14 | amCatTcca | 32 |
| hsa-miR124a/LNA-8/MM | amCcgmCgtAccTtaa | 70 |
| hsa-miR206/LNA-8/MM | tTccTtaAatTcca | 55 |

### Whole mount in situ hybridizations

All washing and incubation steps were performed in 2 ml eppendorf tubes. Embryos were fixed overnight at 4 oC in 4% paraformaldehyde in PBS and subsequently transferred through a graded series (25% MeOH in PBST (PBS containing 0.1% Tween-20), 50% MeOH in PBST, 75% MeOH in PBST) to 100% methanol and stored at -20 oC up to several months. At the first day of the in situ hybridization embryos were rehydrated by successive incubations for 5 min in 75% MeOH in PBST, 50% MeOH in PBST, 25% MeOH in PBST and 100% PBST (4 x 5 min). Fish, mouse and Xenopus embryos were treated with proteinaseK (10 µg/ml in PBST) for 45 min at 37 oC, refixed for 20 min in 4% paraformaldehyde in PBS and washed 3 x 5 min with PBST. After a short wash in water, endogenous alkaline phosphatase activity was blocked by incubation of the embryos in 0.1 M tri-ethanolamine and 2.5% acetic anhydride for 10 min, followed by a short wash in water and 5 x 5 min washing in PBST. The embryos were then transferred to hybridization buffer (50% Formamide, 5x SSC, 0.1% Tween, 9.2 mM citric acid, 50 ug/ml heparin, 500 ug/ml yeast RNA) for 2-3 hour at the hybridization temperature. Hybridization was performed in fresh pre-heated hybridization buffer containing 10 nM of labeled LNA probe. Post-hybridization washes were done at the hybridization temperature by successive incubations for 15 min in HM- (hybridization buffer without heparin and yeast RNA), 75% HM-/25% 2x SSCT (SSC containing 0.1% Tween-20), 50% HM-/50% 2x SSCT, 25% HM-/75% 2x SSCT, 100% 2x SSCT and 2 x 30 min in 0.2x SSCT. Subsequently, embryos were transferred to PBST through successive incubations for 10 min in 75% 0.2x SSCT/25% PBST, 50% 0.2x SSCT/50% PBST, 25% 0.2x SSCT/75% PBST and 100% PBST. After blocking for 1 hour in blocking buffer (2% sheep serum/2mg:ml BSA in PBST), the embryos were incubated overnight at 4 oC in blocking buffer containing anti-DIG-AP FAB fragments (Roche, 1/2000). The next day, zebrafish embryos were washed 6 x 15 min in PBST, mouse and X. tropicalis embryos were washed 6 x 1 hour in TBST containing 2 mM levamisole and then for 2 days at 4oC with regular refreshment of the wash buffer. After the post-antibody washes, the embryos were washed 3 x 5 min in staining buffer (100 mM tris HCl pH9.5, 50 mM MgCl2, 100 mM NaCl, 0.1% tween 20). Staining was done in buffer supplied with 4.5 µl/ml NBT (Roche, 50 mg/ml stock) and 3.5 µl/ml BCIP (Roche, 50 mg/ml stock). The reaction was stopped with 1 mM EDTA in PBST and the embryos were stored at 4oC. The embryos were mounted in Murray's solution (2:1 benzylbenzoate:benzylalcohol) via an increasing methanol series (25% MeOH in PBST, 50% MeOH in PBST, 75% MeOH in PBST, 100% MeOH) prior to imaging.

### Image acquisition

Embryos and larvae stained by whole-mount in situ hybridization were analyzed with Zeiss Axioplan and Leica MZFLIII microscopes and subsequently photographed with digital cameras. Sections were analyzed with a Nikon Eclipse E600 microscope and photographed with a digital camera (Nikon, DXM1200). Images were adjusted with Adobe Photoshop 7.0 software.

### Results

We first compared the ability of LNA-modified DNA probes to detect miR-206, miR-124a and miR-122a in 72h zebrafish embryos with unmodified DNA probes of identical length and sequence. These three miRNAs are strongly expressed in the muscles, central nervous system and liver respectively. Both probe types could be easily labeled with digoxigenin (DIG) using standard 3' end labeling procedures. Labeling efficiency was checked by dot-blot analysis. Equal labeling was obtained for both LNA-modified and unmodified DNA probes (Fig. 7a). As depicted in Figure 7b, expected signals were obtained for all three miRNAs when LNA-modified probes were used for hybridization. In contrast, no such expression patterns could be seen with corresponding DNA probes under the same hybridization conditions. Lowering of the hybridization temperature resulted in high background signals for all three DNA probes Similar experiments to detect miRNAs in fish embryos using in vitro synthesized RNA probes, that carried a concatamer against the mature miRNA, were also unsuccessful. These results indicate that LNA-modified probes are well suited for sensitive in situ detection of miRNAs

### Determination of the optimal hybridization temperature for LNA-modified probes

The introduction of LNA modifications in a DNA oligonucleotide probe increases the Tm value against complementary RNA with 2-10 °C per LNA monomer. Since the Tm values of LNA-modified probes can be calculated using a thermodynamic nearest neighbor model35 we decided to determine the optimal hybridization temperature for detecting miRNAs in zebrafish using LNA-modified probes, in relation to their Tm values (Table 15-I). The probes for miR-122a (liver specific) and miR-206 (muscle specific) have a calculated Tm value of 78 °C and 73 °C respectively. For miR-122a an optimal signal was obtained at a hybridization temperature of 58 °C and the probe for miR-206 gave the best signal at a temperature of 54 °C (Fig. 8a). A decrease or an increase in the hybridization temperature results in either higher background staining or complete loss of the hybridization signal. Thus, optimal results are obtained with hybridization temperatures of ~21-22 °C below the predicted Tm value of the LNA probe.

Apart from adjusting the hybridization temperature, standard in situ procedures also make use of higher formamide concentrations to increase the hybridization stringency. We used a formamide concentration of 50% and did not investigate the effects of formamide concentration on LNA-based miRNA in situ detection further, as the hybridization temperatures were in a convenient range.

### Determination of the optimal hybridization time for LNA-modified probes

The standard zebrafish in situ protocol requires overnight hybridization. This may be necessary for long riboprobes used for mRNA in situ hybridization. We investigated the optimal hybridization time for LNA-based miRNA in situ hybridization. Significant in situ staining was obtained even after ten minutes of hybridization for miR-122a and miR-206 in 72 hour fish embryos (Fig. 8b). After one hour of hybridization the signal strength was comparable to the staining obtained after an overnight hybridization. This indicates that the hybridization times can be easily shortened for in situs using LNA probes, which would reduce the overall miRNA in situ protocol for zebrafish from three to two days.

### Determination of the specificity of LNA-modified probes

Many miRNAs belong to miRNA families. Some of the family members differ by one or two bases only, e.g. let-7c and let-7e (two mismatches) or miR-10a and miR-10b (one mismatch) and it might be that these do not have identical expression patterns. Indeed, from recent work it is clear that let-7c and let-7e have different expression patterns in the limb buds of the early mouse embryo. To examine the specificity of LNA-modified probes we set out to perform in situ hybridizations with single and double mismatched probes for miR-124a, miR-206 and miR-122a (Table 15-I) under the same hybridization conditions as the fully complementary probe (Fig. 9). For miR-122a and miR-206 specific staining was lost upon introduction of a single central mismatch in the LNA probe. For the miR-124a probe two central mismatches were needed for adequate discrimination. These data demonstrate the high specificity of LNA-based miRNA in situ hybridization.

To investigate if the in situ signal is fully coming from mature miRNAs or also from precursors, we designed probes against star and loop sequences of miR-183 and miR-217. miR-183 is specific for the haircells of the lateral line organ and the ear, rods and cones and bipolar cells in the eye and sensory epithelia in the nose, while miR-217 is specific for the exocrine pancreas. We could not detect any pattern with probes against star and loop sequences for these miRNAs, suggesting that LNA-modified probes mainly detect mature miRNAs.

### Reduction of the LNA probe length

In our initial in situ miRNA detection experiments, we used LNA-modified probes complementary to the complete mature miRNA sequence. Next, we decided to determine the minimal probe length, by which it would still be possible to get specific staining. Therefore, we systematically shortened the probes against miR-124a and miR-206 and performed in situ hybridization on 72h zebrafish embryos with hybridization temperatures adjusted to 21 °C below the Tm value of the shortened probes. We could specifically detect miR-206 and miR-124a with shortened versions of the LNA probes complementary to a 12-nt region at the 5'-end of the miRNA (Fig. 10). In situ staining was virtually lost when 10-nt or 8-nt probes were used, although the 10-nt miR-124a probe gave a weak hybridization signal in the brain.

We expect that shorter LNA probes would exhibit significantly enhanced mismatch discrimination. As described above, in the case of miR-124a a single mismatch in a 22-mer LNA-modified probe was not sufficient for adequate discrimination. We thus tested single mismatch versions of the 14-mer LNA probes for miR-206 and miR-124a and found that in both cases the hybridization signal was completely lost (Fig. 10).

### Detection of miRNAs in Xenopus laevis and mouse embryos

Thus far, we have reported the use of LNA probes for the detection of miRNAs only in the zebrafish embryo. To explore the usefulness of the LNA probe technology for detection of miRNAs in other organisms, we performed whole mount in situ hybridization on mouse and Xenopus tropicalis embryos with probes for miR-124a and miR-1, both of which are known to be abundant and tissue specific miRNAs (Fig. 11a and b). miR-124a was specific for tissues of the central nervous system in both organisms. miR-1 was expressed in the body wall muscles and the muscles of the head in Xenopus. In mouse, miR-1 was mainly expressed in the somitic muscles and the heart. These data are in agreement with the expression patterns in zebrafish and with expression studies based on dissected tissues from mouse, which show that miR-124a is brain specific and miR-1 is a muscle specific miRNA. Recently, a LacZ fusion construct of miR-1 also demonstrated that miR-1 is expressed in the heart and the somites of the early mouse embryo.

Next, we decided to determine the whole mount expression patterns in mouse embryos for miR-1, miR-206, miR-17, miR-20, miR-124a, miR-9, miR-126, miR-219, miR-196a, miR-10b and miR-10a, where the patterns were similar to what we previously observed in the zebrafish. In addition, miR-10a and miR-196a were found to be active in the posterior trunk in mouse embryos as visualized by miRNA-responsive sensors and we also found these miRNAs to be expressed in the same regions. For miR-182, miR-96, miR-183 and miR-125b the expression patterns were different compared to zebrafish. miR-182, miR-96 and miR-183 are expressed in the cranial and dorsal root ganglia. In zebrafish the same miRNAs show expression in the haircells of the lateral line neuromasts and the inner ear but also in the cranial ganglia. miR-125b is expressed at the midbrain hindbrain boundary in the early mouse embryo, whereas in zebrafish this miRNA is expressed in the brain and spinal cord.

Hence, based on the above it can be concluded that the present invention relates to aspects including:
a) Use of an oligonucleotide in the isolation, purification, amplification, detection, identification, quantification, inhibition or capture of non-coding RNAs characterized in that the oligonucleotide contains a number of nucleoside analogues;
b) the use of such an oligonucleotide wherein the non-coding RNAs are selected from microRNAs, in particular mature microRNAs;
c) such uses as in a or b wherein the number of nucleoside analogue corresponds to from 20 to 40% of the oligonucleotide;
d) such uses as in a, b or c, wherein the nucleoside analogue is LNA;
e) such uses as in a, b, c or d, wherein the oligonucleotide comprises nucleoside analogues inserted with regular spacing between said nucleoside analogues, e.g. at every second nucleotide position, every third nucleotide position, or every fourth nucleotide position;
f) such uses as in a, b, c, d or e in miRNA *in situ* hybridisation, dot blot hybridisation, reverse dot blot hybridisation, in expression profiling by oligonucleotide arrays or in Northern blot analysis;
g) such uses as in a, b, c, d or e in miRNA inhibition for functional analysis and antisense-based intervention against tumorigenic miRNAs and other non-coding RNAs;
h) such uses as in a, b, c, d or e in miRNA detection for the identification of the primary site of metastatic tumors of unknown origin;
i) such uses as in a, b, c, d, e, f, g, and h wherein the length of the oligonucleotide is less than about 21 nucleotides in length and more preferably less than 18 nucleotides, and most preferably between 12 and 14 nucleotides in length, and
j) a kit for the isolation, purification, amplification, detection, identification, quantification, or capture of a non-coding RNA, in particular mature microRNAs, the kit comprising a reaction body and one or more modified nucleotides.

### EMBODIMENTS

1. A collection of detection probes, wherein each member of said collection comprises a recognition sequence consisting of nucleobases and affinity enhancing nucleobase analogues, and wherein the recognition sequences exhibit a combination of high melting temperatures and low self-complementarity scores, said melting temperatures being the melting temperature of the duplex between the recognition sequence and its complementary DNA or RNA sequence.
2. The collection according to embodiment 1, wherein at least 80% of the detection probes include recognition sequences which exhibit a melting temperature or a measure of melting temperature corresponding to at least 5°C higher than a melting temperature or a measure of melting temperature of the self-complementarity score under condtions where the probe hybridizes specifically to its complementary target sequence.
3. The collection according to embodiment 2, wherein at least 90% of the detection probes include recognition sequences which exhibit a melting temperature or a measure of melting temperature corresponding to at least 5°C higher than a melting temperature or a measure of melting temperature of the self-complementarity score under condtions where the probe hybridizes specifically to its complementary target sequence.
4. The collection according to embodiment 2, wherein at least 95% of the detection probes include recognition sequences which exhibit a melting temperature or a measure of melting temperature corresponding to at least 5°C higher than a melting temperature or a measure of melting temperature of the self-complementarity score under condtions where the probe hybridizes specifically to its complementary target sequence.
5. The collection according to embodiment 2, wherein all of the detection probes include recognition sequences which exhibit a melting temperature or a measure of melting temperature corresponding to at least 5°C higher than a melting temperature or a measure of melting temperature of the self-complementarity score under condtions where the probe hybridizes specifically to its complementary target sequence.
6. The collection according to any one of the preceding embodiments, wherein the melting temperature or the measure of melting temperature is at least 10°C, such as at least 15, at least 20, at least 25, at least 30, at least 35, at least 40, at least 45, and at least 50°C higher than a melting temperature or measure of melting temperature fo the self-complementarity score.
7. The collection according to any one of the preceding embodiments, comprising at least 10 detection probes, 15 detection probes, such as at least 20, at least 25, at least 50, at least 75, at least 100, at least 200, at least 500, at least 1000, and at least 2000 members.
8. The collection according to any one of the preceding embodiments, which is capable of specifically detecting all members of the transcriptome of an organism.
9. The collection according to any one of embodiments 1-8, which is capable of specifically detecting all small RNAs of an organism.
10. The collection according to embodiment 9, wherein the small RNAs are miRNA or siRNA.
11. The collection according to embodiment 9 or 10, wherein the organism is selected from the group consisting of a bacterium, a yeast, a fungus, a protozoan, a plant, and an animal.
12. The collection according to any one of the preceding embodiments, wherein the affinity-enhancing nucleobase analogues are regularly spaced between the nucleobases in at least 80% of the members of said collection, such as in at least 90% or at least 95% of said collection.
13. The collection according to any one of the preceding embodiments, wherein the 3' and 5' nucleobases are not substituted by affinity enhancing nucleobase analogues.
14. The collection according to any one of embodiments 1-13, wherein the presence of the affinity enhancing nucleobases in the recognition sequence confers an increase in the binding affinity between a probe and its complementary target nucleotide sequence relative to the binding affinity exhibited by a corresponding probe, which only include nucleobases.
15. The collection according to any one of embodiments 1-14, wherein the affinity enhancing nucleobase analogues are LNA nucleobases.
16. The collection according to any one of the preceding embodiments, wherein the affinity enhancing nucleobase analogues are regularly spaced as every 2^{nd}, every 3^{rd}, every 4^{th} or every 5^{th} nucleobase in the recognition sequence, preferably as every 3^{rd} nucleobase.
17. The collection according to any one of the preceding embodiments, wherein the recognition sequence is at least a 6-mer, such as at least a 7-mer, at least an 8-mer, at least a 9-mer, at least a 10-mer, at least an 11-mer, at least a 12-mer, at least a 13-mer, at least a 14-mer, at least a 15-mer, at least a 16-mer, at least a 17-mer, at least an 18-mer, at least a 19-mer, at least a 20-mer, at least a 21-mer, at least a 22-mer, at least a 23-mer, and at least a 24-mer.
18. The collection according to any one of embodiments 1-16, wherein the recognition sequence is at most a 25-mer, such as at most a 24-mer, at most a 23-mer, at most a 22-mer, at most a 21-mer, at most a 20-mer, at most a 19-mer, at most an 18-mer, at most a 17-mer, at most a 16-mer, at most a 15-mer, at most a 14-mer, at most a 13-mer, at most a 12-mer, at most an 11-mer, at most a 10-mer, at most a 9-mer, at most an 8-mer, at most a 7-mer, and at most a 6-mer.
19. The collection according to any one of the preceding embodiments, wherein at least 80% of the members comprise recognition sequences of the same length, such as at least 90% or at least 95%.
20. The collection according to embodiment 19, wherein all members contain affinity enhancing nucleobase analogues with the same regular spacing in the recognition sequences.
21. The collection according to any one of the preceding embodiments, wherein at least one of the nucleobases in the recognition sequence is substituted with its corresponding selectively binding complementary (SBC) nucleobase.
22. The collection according to any one of the preceding embodiments, wherein the nucleobases in the sequence are selected from ribonucleotides and deoxyribonucleotides.
23. The collection according to embodiment 22, wherein the recognition sequence consists of affinity enhancing nucleobase analogues together with either ribonucleotides or deoxyribonucleotides.
24. The collection according to any one of the preceding embodiments, wherein each member is covalently bonded to a solid support.
25. The collection according to embodiment 24, wherein the solid support is selected from a bead, a microarray, a chip, a strip, a chromatographic matrix, a microtiter plate, and a fiber.
26. The collection according to any one of the preceding embodiments, wherein each detection probe includes a detection moiety and/or a ligand, optionally in the recognition sequence.
27. The collection according to any one of the preceding embodiments, wherein each detection probe includes a photochemically active group, a thermochemically active group, a chelating group, a reporter group, or a ligand that facilitates the direct of indirect detection of the probe or the immobilisation of the oligonucleotide probe onto a solid support.
28. A detection probe which is a member of a collection according to any one of the preceding embodiments.
29. A detection probe including a recognition sequence selected from the LNA containing recognition sequences set forth in the tables A-K, 1, 3 and 15-I herein.
30. A method for expanding or building a collection according to any one of embodiments 1-27, comprising
   A) defining a reference nucleotide sequence consisting of nucleobases, said reference nucleotide sequence being complementary to a target sequence for which the collection does not contain a detection probe,
   B) substituting the reference nucleotide sequence's nucleobases with affinity enhancing nucleobase analogues to provide a set of chimeric sequences wherein,
   C) determining usefulness of each of the chimeric sequences based on assessment of their ability to self-anneal and their melting temperature, and
   D) synthesizing and adding, to the collection, a probe comprising as its recognition sequence the chimeric sequence with the optimum combination of high melting temperature and low self-annealing.
31. The method according to embodiment 30, wherein step B includes provision of all possible chimeric sequences which include a particular set of affinity enhancing nucleobase analogues.
32. The method according to embodiment 30 or 31, wherein only chimeric sequences, wherein the affinity enhancing nucleobase analogues are regularly spaced between the nucleobases, are added to the collection in step D.
33. A method for designing an optimized detection probe for a target nucleotide sequence, comprising
   1) defining a reference nucleotide sequence consisting of nucleobases, said reference nucleotide sequence being complementary to said target nucleotide sequence,
   2) substituting the reference nucleotide sequence's nucleobases with affinity enhancing nucleobase analogues to provide a set of chimeric sequences
   3) determining usefulness of each of the chimeric sequences based on assessment of their ability to self-anneal and their melting temperatures, and
   4) defining the optimized detection probe as the one in the set having as its recognition sequence the chimeric sequence with the optimum combination of high melting temperature and low self-annealing.
34. The method according to embodiment 33, wherein step 2 includes provision of all possible chimeric sequences which include a particular set of affinity enhancing nucleobase analogues.
35. The method according to embodiment 33 or 34, further comprising synthesizing the optimized detection probe.
36. The method according to any one of embodiments 33-35, wherein only chimeric sequences, wherein the affinity enhancing nucleobase analogues are regularly spaced between the nucleobases, are defined in step 4 or, if applicable, are synthesized.
37. The method according to any one of embodiments 33-36, wherein the detection probe is further modified by containing at least one SBC nucleobase as one of the nucleobases.
38. The method according to any one of embodiments 32-37, wherein the detection probe is a detection probe according to embodiment 28 or 29.
39. The method according to any one of embodiments 30-37, wherein, where applicable, steps A-C or 1-4, are performed in silico.
40. A computer system for designing an optimized detection probe for a target nucleic acid sequence, said system comprising
   a) input means for inputting the target nucleotide,
   b) storage means for storing the target nucleotide sequence,
   c) optionally executable code which can calculate a reference nucleotide sequence being complementary to said target nucleotide sequence and/or input means for inputting the reference nucleotide sequence,
   d) optionally storage means for storing the reference nucleotide sequence,
   e) executable code which can generate chimeric sequences from the reference nucleotide sequence or the target nucleic acid sequence, wherein said chimeric sequences comprise the reference nucleotide sequence, wherein has been in-substituted affinity enhancing nucleobase analogues,
   f) executable code which can determine the usefulness of such chimeric sequences based on assessment of their ability to self-anneal and their melting temperatures and either rank such chimeric sequences according to their usefulness,
   g) storage means for storing at least one chimeric sequence, and
   h) output means for presenting the sequence of at least one optimized detection probe.
41. The computer system according to embodiment 40, wherein the target nucleic acid sequences are the sequences of non-coding smalle RNAs, such as miRNAs.
42. A computer system comprising executable code capable of executing the method according to embodiment 39 .
43. Storage means comprising executable code which can execute the method steps according to embodiment 39.
44. A method for specific isolation, purification, amplification, detection, identification, quantification, inhibition or capture of a target nucleotide sequence in a sample, said method comprising contacting said sample with a member of a collection according to any one of embodiments 1-27 or with a probe according ot embodiment 28 or 29 under conditions that facilitate hybridization between said member/probe and said target nucleotide sequence.
45. The method according to embodiment 44, used in isolation, purification, amplification, detection, identification, quantification, inhibition or capture of a molecule comprising the target nucleotide sequence.
46. The method according to embodiment 45, wherein the molecule is a small, non-coding RNA.
47. The method according to embodiment 46, wherein the molecule is miRNA such as a mature miRNA.
48. The method according to embodiment 47, used for the identification of the primary site of metastatic tumors of unknown origin.
49. The method according to any one of embodiments 45-48, wherein the small, non-coding RNA has a length of at most 30 residues, such as at most 29, 28, 27, 26, 25, 24, 23, 22, 21, 20, 19, or 18 residues.
50. The method according to any one of embodiments 45-48, wherein the small, non-coding RNA has a length of at least 15 residues, such as at least 16, 17, 18, 19, 20, 21, 22, 23, 24, 25, 26, 27, 28, 29 or 30 residues.
51. The method according to embodiment 45, wherein the molecule is DNA or RNA present in a fixated, embedded sample such as a formalin fixated paraffine embedded sample.
52. The method according to any one of embodiments 44-51, which is used in diagnosis, prognosis, therapy outcome prediction, and therapy.

## Claims

1. An oligonucleotide comprising a recognition sequence complementary to an RNA target sequence being an miRNA binding site in its cognate mRNA target, wherein said recognition sequence is substituted with a high-affinity nucleotide analogue.

2. The oligonucleotide according to claim 1, which is an LNA substituted oligonucleotide

3. The oligonucleotide according to claim 2, wherein the LNA nucleotides are consecutive or separated by one or more non-LNA nucleotides when at least two LNA nucleotides are included.

4. The oligonucleotide according to any one of the preceding claims, which is comprised of a sequence of approximately at least 3 nucleotides.

5. The oligonucleotide according to claim 3, which is comprised of a sequence of at least about 6 nucleotides.

6. The oligonucleotide according to claim 3, which is comprised of a sequence of at least about 8-30 nucleotides corresponding to a region of the designated target nucleotide sequence.

7. The oligonucleotide according to any one of the preceding claims, which comprise from 1 to 8 stabilizing nucleotides.

8. The oligonucleotide according to any one of the preceding claims, wherein the number of nucleotide analogues corresponds to from 20 to 40% of the oligonucleotide.

9. Use of an oligonucleotide according to any one of claims 1-8, for the functional analysis of miRNAs, and their target mRNA, or alternative mRNA splice variants in vitro in plants or animals, by inhibiting the binding of mature miRNAs to their cognate target mRNAs.

10. Use of an oligonucleotide according to any one of claims 1-8, for antisense-based intervention, targeted against tumorigenic single stranded miRNAs, their target mRNAs or alternative mRNA splice variants in vivo in plants by inhibiting the binding of mature miRNAs to their cognate target mRNAs.

11. The use according to any one of claims 9 or 10, wherein the plants is selected from *Caenorhabditis elegans, Drosophila melanogaster, Arabidopsis thaliana,* rice, and maize.

12. An oligonucleotide according to any one of claims 1-8, for use in antisense-based intervention, targeted against tumorigenic single stranded miRNAs, their target mRNAs or alternative mRNA splice variants in vivo in animals by inhibiting the binding of mature miRNAs to their cognate target mRNAs.

13. The oligonucleotide according to any one of claims 9 or 12, wherein the animal is selected from human, mouse, rat, and zebrafish.

14. The oligonucleotide according to claim 12, wherein the RNA target sequence is a specific nucleic acid sequence within a target nucleic acid of a human patient.

15. An oligonucleotide according to any one of claims 1-8, for use as a medicament.
